(19) Europäisches Patentamt
European Patent Office
Office européen des brevets

(11) EP 4 066 931 A1

(12) # EUROPEAN PATENT APPLICATION
published in accordance with Art. 153(4) EPC

(43) Date of publication:
**05.10.2022 Bulletin 2022/40**

(21) Application number: **20892662.6**

(22) Date of filing: **27.11.2020**

(51) International Patent Classification (IPC):
***B01J 13/02** (2006.01)* ***C07K 1/22** (2006.01)*
***C12P 21/00** (2006.01)*

(52) Cooperative Patent Classification (CPC):
**B01J 13/02; C07K 1/14; C07K 1/22; C12P 21/00**

(86) International application number:
**PCT/CN2020/132171**

(87) International publication number:
**WO 2021/104435 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.11.2019 CN 201911209156**
**28.04.2020 CN 202010346903**

(71) Applicant: **Kangma-Healthcode (Shanghai) Biotech Co., Ltd**
**Pudong New Area**
**Shanghai 201321 (CN)**

(72) Inventors:
- **GUO, Min**
**Shanghai 201321 (CN)**
- **XU, Liqiong**
**Shanghai 201321 (CN)**
- **YU, Xue**
**Shanghai 201321 (CN)**

(74) Representative: **Swan, Elizabeth Mary**
**Withers & Rogers LLP**
**2 London Bridge**
**London SE1 9RA (GB)**

(54) **BIOMAGNETIC MICROSPHERE AND PREPARATION METHOD THEREFOR AND USE THEREOF**

(57) Provided is biological magnetic microsphere, comprising magnetic microsphere body. Magnetic microsphere body comprises, on outer surface thereof, at least one polymer having linear backbone and side chain. End of linear backbone fixes to outer surface of magnetic microsphere body, other ends of polymer unattach to outer surface of magnetic microsphere body. Biotin links to terminal end of side chain of polymer of biological magnetic microsphere. Further provided are modification to, preparation method for, use of biological magnetic microsphere. Biological magnetic microsphere can be handled and used conveniently, rapidly disperse and rapidly precipitate in solution without need to use large-scale experimental equipment such as high-speed centrifuge, can be connected via biotin with purification element having selectivity (such as avidin, affinity protein, polypeptide/protein tag, etc.), is versatile in application and can be widely and massively used in separation and purification of target substance, such as protein, including but not limited to antibody.



Processed by Luminess, 75001 PARIS (FR)

SEQUENCE LISTING

<110> KANGMA-HEALTHCODE (SHANGHAI) BIOTECH CO., LTD

<120> BIOMAGNETIC MICROSPHERE AND PREPARATION METHOD THEREFOR AND USE THEREOF

<130> P22413051EP

<150> 201911209156X
<151> 2019-11-30

<150> 2020103469030
<151> 2020-04-28

<160> 6

<170> PatentIn version 3.5

<210> 1
<211> 873
<212> DNA
<213> Artificial Sequence

<220>
<223> nucleotide sequence of Protein A

<400> 1
gcccagcacg acgaggccca gcaaaacgcc ttttaccagg ttttgaacat gcccaacttg 60

aacgccgacc agaggaacgg cttcatccag agtttgaaag acgaccccag tcaaagtgcc 120

aacgttttgg gcgaggcaca gaaattaaat gacagccagg cccccaaggc cgacgcccag 180

cagaacaact tcaacaagga tcagcagagt gccttttacg aaatattgaa catgcccaac 240

ctaaacgagg cacagagaaa tgggttcatc cagagtttaa aagatgatcc cagtcagagt 300

accaacgttt tgggggaggc caaaaagtta aacgaaagcc aggcgcccaa agctgataac 360

aacttcaaca aggagcagca gaacgccttc tacgagattc taaacatgcc caacttgaac 420

gaggagcaga ggaacggctt catccagagt ttgaaagacg atcccagtca gagtgcaaac 480

ttgctatctg aggccaagaa gctcaacgag tcacaggccc ccaaggccga taacaagttc 540

aacaaggagc agcagaacgc tttctatgaa atccttcatt tgccaaattt gaatgaggaa 600

cagaggaacg gcttcatcca gagtttgaaa gatgacccga gtcagagtgc caacttgttg 660

gccgaagcca agaagttgaa tgatgcccag gcccccaagg ccgacaacaa gtttaacaag 720

gagcagcaga atgcctttta cgagatcttg cacttgccca acttgactga ggaacaaagg 780

aacggtttca tacagagttt gaaggacgac ccctctgtta gtaaggaaat ccttgcggag 840

gcaaagaagc taaacgatgc tcaagcacca aaa 873

<210> 2
<211> 423
<212> DNA
<213> Artificial Sequence

<220>
<223> nucleotide sequence of tamavidin2

<400> 2
atgagtgatg ttcaatcttc tttgactggt acttggtata atgaattgaa ttctaaaatg 60

gaattgactg ctaataaaga tggtactttg actggtaaat atttgtctaa agttggtgat 120

gtttacgttc catatccatt gtctggtaga tataatttgc aaccaccagc tggtcaaggt 180

gttgctttgg gttgggctgt ttcttgggaa aattctaaaa ttcattctgc tactacttgg 240

tctggtcaat tcttctctga atcttctcca gttattttga ctcaatggtt attgtcttct 300

tctactgcta gaggtgatgt ttgggaatct actttggttg gtaacgattc tttcactaaa 360

actgctccaa ctgaacaaca aattgctcat gctcaattgc attgtagagc tccaagattg 420

aaa 423

<210> 3
<211> 714
<212> DNA
<213> Artificial Sequence

<220>
<223> nucleotide sequence of mEGFP

<400> 3
gtgagcaagg gcgaggagct gttcaccggg gtggtgccca tcctggtcga gctggacggc 60

gacgtaaacg gccacaagtt cagcgtgcgc ggcgagggcg agggcgatgc caccaacggc 120

aagctgaccc tgaagttcat ctgcaccacc ggcaagctgc ccgtgccctg gcccaccctc 180

gtgaccaccc tgacctacgg cgtgcagtgc ttcagccgct accccgacca catgaagcag 240

cacgacttct tcaagtccgc catgcccgaa ggctacgtcc aggagcgcac catctccttc 300

aaggacgacg gcacctacaa gacccgcgcc gaggtgaagt tcgagggcga caccctggtg 360

aaccgcatcg agctgaaggg catcgacttc aaggaggacg gcaacatcct ggggcacaag 420

ctggagtaca acttcaacag ccacaacgtc tatatcacgg ccgacaagca gaagaacggc 480

atcaaggcga acttcaagat ccgccacaac gtcgaggacg gcagcgtgca gctcgccgac 540

cactaccagc agaacacccc catcggcgac ggccccgtgc tgctgcccga caaccactac 600

ctgagcaccc agtccaagct gagcaaagac cccaacgaga agcgcgatca catggtcctg 660

ctggagttcg tgaccgccgc cgggatcact ctcggcatgg acgagctgta caag 714

<210> 4
<211> 585
<212> DNA
<213> Artificial Sequence

<220>
<223> nucleotide sequence of antibody binding region of Protein G

<400> 4
acttacaaat taatccttaa tggtaaaaca ttgaaaggcg aaacaactac tgaagctgtt 60

gatgctgcta ctgcagaaaa agtcttcaaa caatacgcta acgacaacgg tgttgacggt 120

gaatggactt acgacgatgc gactaagacc tttacagtta ctgaaaaacc agaagtgatc 180

gatgcgtctg aattaacacc agccgtgaca acttacaaac ttgttattaa tggtaaaaca 240

ttgaaaggcg aaacaactac tgaagctgtt gatgctgcta ctgcagaaaa agtcttcaaa 300

caatacgcta acgacaacgg tgttgacggt gaatggactt acgacgatgc gactaagacc 360

tttacagtta ctgaaaaacc agaagtgatc gatgcgtctg aattaacacc agccgtgaca 420

acttacaaac ttgttattaa tggtaaaaca ttgaaaggcg aaacaactac taaagcagta 480

gacgcagaaa ctgcagaaaa agccttcaaa caatacgcta acgacaacgg tgttgatggt 540

gtttggactt atgatgatgc gactaagacc tttacggtaa ctgaa 585

<210> 5
<211> 117
<212> PRT
<213> Artificial Sequence

<220>
<223> amino acid sequence of Nanobody anti-eGFP

<400> 5

Met Ala Gln Val Gln Leu Val Glu Ser Gly Gly Ala Leu Val Gln Pro
1 5 10 15

Gly Gly Ser Leu Arg Leu Ser Cys Ala Ala Ser Gly Phe Pro Val Asn
20 25 30

Arg Tyr Ser Met Arg Trp Tyr Arg Gln Ala Pro Gly Lys Glu Arg Glu
35 40 45

Trp Val Ala Gly Met Ser Ser Ala Gly Asp Arg Ser Ser Tyr Glu Asp
50 55 60

Ser Val Lys Gly Arg Phe Thr Ile Ser Arg Asp Asp Ala Arg Asn Thr
65 70 75 80

Val Tyr Leu Gln Met Asn Ser Leu Lys Pro Glu Asp Thr Ala Val Tyr
85 90 95

Tyr Cys Asn Val Asn Val Gly Phe Glu Tyr Trp Gly Gln Gly Thr Gln
100 105 110

Val Thr Val Ser Ser
115

<210> 6
<211> 693
<212> DNA
<213> Artificial Sequence

<220>
<223> nucleotide sequence of mScarlet

<400> 6
gtttcaaagg gtgaagctgt tattaaggag tttatgagat tcaaagtgca tatggaaggt 60

tctatgaatg gtcatgaatt tgaaattgag ggtgaaggtg aaggtagacc atatgaaggt 120

actcaaactg ctaaattgaa ggttactaaa ggtggtccat tgccattctc atgggatatt 180

ttgtcaccac aattcatgta tggttctaga gctttcatta agcatccagc tgatattcca 240

gattactata agcaatcatt cccagaaggt ttcaagtggg aaagagttat gaattttgaa 300

gatggtggtg ctgttactgt tactcaagat acttcattgg aagatggtac tttgatctat 360

aaggttaagt tgagaggtac taatttccca ccagatggtc cagttatgca aaagaaaact 420

atgggttggg aagctagtac tgaaagattg tatccagaag atggtgtttt gaagggtgac 480

attaagatgg ctttgagatt gaaagatggt ggtagatatt tggctgattt caagactact 540

tataaggcta agaagccagt tcaaatgcca ggtgcttaca atgttgatag aaaattggat 600

atcacctctc ataatgaaga ttatactgtt gttgagcaat acgaaagatc tgaaggtaga 660

cattctactg gtggtatgga tgaattgtat aag 693

## Description

CROSS-REFERENCES TORELATED APPLICATIONS

This application is a national stage application of PCT Patent Application NO PCT/CN2020/132171, filed on 27.11.2020, which claims priority to Chinese Patent Application NO 201911209156.X, filed on 30.11.2019, and Chinese Patent Application NO 202010346903.0, filed on 28.04.2020, the content of all of which is incorporated herein by reference.

FIELD OF THE INVENTION

The present application relates to the field of biochemical technology, and more particularly, to a biomagnetic microsphere and a preparation method therefor and use thereof.

BACKGROUND

Separation and purification of a protein, including but not limited to an antibody, an antibody fragment or a fusion protein thereof, is an important downstream link in a biological drug production process. An effect and an efficiency of the separation and purification directly affect a quality and a production cost of a protein drug. For protein purification, currently, a plurality of materials including agarose gel and more are commonly used as a purification column or a purification microsphere carrier. In the prior art, for separation and purification of an antibody substance (including an antibody molecule, an antibody fragment or a fusion protein thereof), a technical personnel mainly uses a Protein A affinity adsorption column to separate and purify an antibody molecule in a fermentation liquid or a reaction liquid. In the Protein A column, by a specific binding between the Protein A which is immobilized on a carrier and a specific site of Fc end of an antibody molecule, a specific and efficient separation of the antibody from a solution is achieved. A carrier in the Protein A column commonly used in the prior art, is mainly made of a plurality of materials including agarose gel and more.

A three-dimensional porous structure of a gel material is beneficial to increase a specific surface area of the material, thereby increasing a number of sites that are able to bind to a purification element (such as an immobilized Protein A), improving a specific binding capacity to a target protein (including an antibody). Although the three-dimensional porous structure of a carrier material is able to increase a number of protein (including the antibody) binding sites greatly, the porous structure inside the carrier will also increase a retention period of the protein during a protein elution, while a plurality of discontinuous spaces or immobilization spaces inside the carrier will further hinder the protein elution from an interior of the material, resulting in a retention ratio of the protein increase. If fixing a protein-binding site only on an outer surface of the carrier, a protein product entering the interior of the material will be avoided, both the retention period and the retention ratio of the protein during the elution will be greatly reduced; however, if only the outer surface of the carrier is adopted, then the specific surface area of the carrier will be greatly reduced, thereby the number of the protein binding sites will also be greatly reduced, that will reduce a purification efficiency.

A polymer is a macromolecular compound that may be formed by a polymerization of one or a plurality of monomer molecules. By adopting a plurality of monomer molecules having one or a plurality of active sites for a polymerization, a polymer product will be rich in a large number of the active sites, increasing the number of active sites greatly, and through these active sites, a plurality of binding sites will be formed or introduced correspondingly. There are a plurality of types and structures of the polymer, including a mesh structure formed by a plurality of molecular chains cross-linking, or a linear structure with a single linear molecular chain, or a branched structure having a plurality of branched-chains (including a branched-chain structure, a dendritic structure, a comb structure, a hyper-branched structure and more). A polymer with a different structural type has a wide application in a different field respectively.

In the prior art, in a purification column applied for the protein separation and purification, a purification element is mainly fixed by a method of covalently coupling. Taking a Protein A column as an example, wherein a Protein A is acting as the purification element. The Protein A column is applied to separating and purifying an antibody, an antibody fragment, or a fusion protein thereof, wherein the Protein A is mainly fixed by a method of covalent coupling, a C-terminal cysteine connects the Protein A to a carrier in a method of covalent coupling. Although the method of covalent coupling is able to ensure that the purification element (such as the Protein A) is fixed onto the carrier firmly, however, after a multiple usage of the purification column (such as the Protein A column), a binding performance of the purification element (such as the Protein A) will decrease, and a purification effect thereof will be reduced. Therefore, in order to ensure a higher purification efficiency and a higher purification quality, an operator shall replace all fillers in an affinity chromatography column on time. Such a process will not only consume a large amount of consumables, but also consume a lot of labors and time, causing a high purification cost.

Therefore, the current technology needs to be improved and developed.

BRIEF SUMMARY OF THE DISCLOSURE

According to the defects described above, the purpose of the present application provides a biomagnetic microsphere, which is able to be applied to separating and purifying a target object, especially a protein (including but not limited to, an antibody protein), not only being able to bind the target object in a high throughout, but also being able to replacing the purification element (such as an affinity protein) easily, having a plurality of characteristics including fast, high-throughput, reusable, and renewable, and further being able to greatly reduce a purification cost of the target object. For example, when adopting an affinity protein as a purification element, the purification cost for an antibody protein will be greatly reduced.

In order to achieve the above mentioned goals, the technical solution of the present application to solve the technical problems is as follows:

A first aspect of the present application provides a biomagnetic microsphere, the biomagnetic microsphere comprises a magnetic microsphere body, an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of biotins or biotin analogs connected, the biotins or biotin analogs may be used either as a purification element or as a connection component to further connect a plurality of other types of purification elements.

The magnetic microsphere is also called a biotin magnetic microsphere or a biotin magnetic bead.

The "fixed onto" means that a linear backbone is "fixed to" the outer surface of the magnetic microsphere body in a method of covalent connection.

Further, the linear backbone is covalently fixed onto the outer surface of the magnetic microsphere body in a direct manner or an indirectly manner via a linker (a connection component).

Further, the number of the branched-chains of the polymer is multiple; preferably, it is at least three.

Preferably, a size of the magnetic microsphere body is selected from any one of following particle size scales or a range between any two particle size scales: 0.1 μm, 0.15 μm, 0.2 μm, 0.25 μm, 0.3 μm, 0.35 μm, 0.4 μm, 0.45 μm, 0.5 μm, 0.55 μm, 0.6 μm, 0.65 μm, 0.7 μm, 0.75 μm, 0.8 μm, 0.85 μm, 0.9 μm, 0.95 μm, 1 μm, 1.5 μm, 2 μm, 2.5 μm, 3 μm, 3.5 μm, 4 μm, 4.5 μm, 5 μm, 5.5 μm, 6 μm, 6.5 μm, 7 μm, 7.5 μm, 8 μm, 8.5 μm, 9 μm, 9.5 μm, 10 μm, 15 μm, 20 μm, 25 μm, 30 μm, 35 μm, 40 μm, 45 μm, 50 μm, 55 μm, 60 μm, 65 μm, 70 μm, 75 μm, 80 μm, 85 μm, 90 μm, 95 μm, 100 μm, 150 μm, 200 μm, 250 μm, 300 μm, 350 μm, 400 μm, 450 μm, 500 μm, 550 μm, 600 μm, 650 μm, 700 μm, 750 μm, 800 μm, 850 μm, 900 μm, 950 μm, 1000 μm; the particle size is an average value.

Preferably, a diameter of the magnetic microsphere body is selected from 0.1 to 10 μm.

Preferably, a diameter of the magnetic microsphere body is selected from 0.2 to 6 μm.

Preferably, a diameter of the magnetic microsphere body is selected from 0.4 to 5 μm.

Preferably, a diameter of the magnetic microsphere body is selected from 0.5 to 3 μm.

Preferably, a diameter of the magnetic microsphere body is selected from 0.2 to 1 μm.

Preferably, a diameter of the magnetic microsphere body is selected from 0.5 to 1 μm.

Preferably, a diameter of the magnetic microsphere body is selected from 1 μm to 1mm.

Preferably, an average diameter of the magnetic microsphere body is 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, 1000 nm, with a deviation of ±20%, more preferably ±10%.

Preferably, the backbone of the polymer is a polyolefin backbone, or an acrylic polymer backbone. The acrylic polymers are defined in a "Nouns and Terms" section. Preferably, the polyolefin backbone is also an acrylic polymer backbone (that is, the linear backbone of the polymer is a polyolefin backbone and is provided by the acrylic polymer backbone).

Preferably, a monomer unit of the acrylic polymer is preferably selected from one or a combination of a plurality of acrylic monomer molecules including: acrylic acid, acrylate, acrylic ester, methacrylic acid, methacrylate, methacrylate easter and more. The acrylic polymer may be obtained by polymerizing one of the monomers stated above or by copolymerizing a corresponding combination of the monomers stated above.

Preferably, the branched-chain of the polymer covalently bonds to the biotin or the biotin analog through a functional group-based covalent bond, and bonds the biotin or the biotin analog covalently to the end of the branched-chain of the polymer. It can be obtained by a covalent reaction between a functional group contained in the branched-chains of polymer molecules on the outer surface of the biomagnetic microspheres with the biotin or the biotin analogs. Wherein, one of the preferred embodiments on the functional group is a specific binding site (a definition thereof is defined in the "Nouns and Terms" section of the detailed description of embodiments).

The functional group-based covalent bond refers to a covalent bond formed by a functional group participating in a covalent coupling. Preferably, the functional group is a carboxyl group, a hydroxyl group, an amino group, a sulfhydryl group, a salt form of a carboxyl group, a salt form of an amino group, a formate group, or a combination thereof. A preferred embodiment of the salt form of the carboxyl group is a sodium salt form such as COONa; a preferred embodiment of the salt form of the amino group may be an inorganic salt form, or an organic salt form, including but not limited to, a form of hydrochloride, hydrofluorid, and more. The "combination of functional groups" refers to all branched-chains of all polymer molecules on the outer surface of the magnetic microsphere, allowing different functional groups to participate in a formation of a covalent bond; taking the biotin as an example, that is, all biotin molecules on the outer surface of a magnetic microsphere with the biotin are able to covalently link with a plurality of different functional groups respectively, while one biotin molecule is able to link with one functional group only.

Preferably, the linear backbone of the polymer couples covalently to the outer surface of the magnetic microsphere body directly, or couples covalently to the outer surface of the magnetic microsphere body indirectly through a linking group.

Preferably, the magnetic microsphere body is a magnetic material encapsulated with $SiO_2$. Alternatively, the $SiO_2$ may be a silane coupling agent having an active site build-in.

Preferably, the magnetic material is selected from one or a combination of iron oxides, iron compounds, iron alloys, cobalt compounds, cobalt alloys, nickel compounds, nickel alloys, manganese oxides, and manganese alloys.

Further, preferably, the magnetic material is selected from one of $Fe_3O_4$, $\gamma$-$Fe_2O_3$, Iron Nitride, $Mn_3O_4$, FeCrMo, FeAlC, AlNiCo, FeCrCo, ReCo, ReFe, PtCo, MnAlC, CuNiFe, AlMnAg, MnBi, FeNiMo, FeSi, FeAl, FeSiAl, $MO•6Fe_2O_3$, GdO or a combination thereof; wherein, the Re is a rare earth element; the M is Ba, Sr, Pb, that is, the $MO•6Fe_2O_3$ is $BaO•6Fe_2O_3$, $SrO•6Fe_2O_3$ or $PbO•6Fe_2O_3$.

A second aspect of the present application provides a biomagnetic microsphere, on a basis of the biomagnetic microsphere provided by the first aspect of the present application, the biotin or the biotin analog, acting as a connection component, further connects with a purification element. That is, the end of the branched-chain of the polymer connects to the purification element through a connection component, and the connection component comprises the biotin or the biotin analog.

The purification element may comprise, but not limited to, an avidin-type tag, a polypeptide-type tag, a protein-type tag, an antibody-type tag, an antigen-type tag, or a combination thereof.

Preferably, the avidin-type tag is avidin, a biotin-binding avidin analog, a biotin analog-binding avidin analog, or a combination thereof.

Preferably, an end of the branched-chain of the polymer on the biomagnetic microspheres is connected with biotin; the purification element is avidin, which forms a binding effect of an affinity complex with the biotin.

The avidin is selected from, but not limited to, a streptavidin, a modified streptavidin, a streptavidin analog, or a combination thereof.

Preferably, the polypeptide-type tag is selected from any one of following tags or a variant thereof: a CBP tag, a histidine tag, a C-Myc tag, a FLAG tag, a Spot tag, a C tag, an Avi tag, a Streg tag, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof. The Streg tag comprises WSHPQFEK and a variant thereof.

Preferably, the protein tag is selected from any one of following tags or a variant protein thereof: an affinity protein, a SUMO tag, a GST tag, an MBP tag and a combination thereof.

Preferably, the affinity protein is selected from Protein A, Protein G, Protein L, modified Protein A, modified Protein G, modified Protein L and a combination thereof.

Preferably, the antibody-type tag is any one of an antibody, a fragment of an antibody, a single chain of an antibody, a fragment of a single chain, an antibody fusion protein, a fusion protein of an antibody fragment, a derivative thereof, or a variant thereof.

Preferably, the antibody-type tag is an anti-protein antibody.

Preferably, the antibody-type tag is an anti-fluorescent protein antibody.

Preferably, the antibody-type label is a nanobody.

Preferably, the antibody-type tag is an anti-protein nanobody.

Preferably, the antibody-type label is an anti-fluorescent protein nanobody.

Preferably, the antibody-type label is an anti-green fluorescent protein nanobody or a mutant thereof.

Preferably, the antibody-type tag is an Fc fragment.

A connection method between the purification element and the biotin or the biotin analog comprises, but not limited to, through a covalent bond, through a non-covalent bond (including a supramolecular interaction), through a connection component, or through a combination thereof.

Preferably, the covalent bond is a dynamic covalent bond; more preferably, the dynamic covalent bond comprises an imine bond, an acylhydrazone bond, a disulfide bond or a combination thereof.

Preferably, the supramolecular interaction is: a coordination binding, an affinity complex interaction, an electrostatic adsorption, a hydrogen bonding, a $\pi$-$\pi$ overlapping interaction, a hydrophobic interaction or a combination thereof.

Preferably, the purification element connects to an end of the branched-chain of the polymer through a connection component containing an affinity complex.

Preferably, the affinity complex interaction is selected from: a biotin-avidin interaction, a biotin analog-avidin interaction, a biotin-avidin analog interaction, a biotin analog-avidin analog interaction.

Preferably, the biotin or the biotin analog has the avidin or the avidin analog connected through the affinity complex interaction, the purification element connects to the avidin or the avidin analog directly or indirectly.

Preferably, the purification element connects to the biotin or the biotin analog at an end of the branched-chain of the polymer through an avidin-type tag-purification element covalent ligation complex, and through a connection component that is an affinity complex formed between the avidin-type tag and the biotin or the biotin analog; more preferably, the purification element forms a connection component of the affinity complex with the biotin or the biotin analog at the end of the branched-chain of the polymer by a avidin- purification element covalent ligation complex .

A third aspect of the present application provides a biomagnetic microsphere, on a basis of the biomagnetic microsphere provided by the first aspect of the present application, further, the biotin or the biotin analog is applied as a connection component, further connects to the avidin or the avidin analog by a binding action of the affinity complex.

The biomagnetic microsphere also becomes an avidin magnetic microsphere or an avidin magnetic bead.

Preferably, the avidin or the avidin analog can be applied either as a purification element or as a connection component to further connect a plurality of other types of purification elements. Wherein, a binding action of the affinity complex is formed between the biotin or the biotin analog and the avidin or the avidin analog

Preferably, on a basis of the biomagnetic microsphere provided in the first aspect of the present application, further comprising an avidin combined with the biotin. Wherein, the binding action of the biotin and the avidin forms an affinity complex. That is: the end of the branched-chain of the polymer on the biomagnetic microspheres connects with the biotin; the purification element is the avidin, and forms the binding action of the affinity complex with the biotin.

Preferably, the avidin is any one of streptavidin, modified streptavidin, and streptavidin analogs or a combination thereof.

A fourth aspect of the present application provides a biomagnetic microsphere, on a basis of the biomagnetic microsphere provided by the third aspect of the present application, further comprising an affinity protein connecting to the avidin or the avidin analog. In this case, the biotin or the biotin analog, the avidin or the avidin analog can all be applied as a connection component, forming a binding action of the affinity complex in between; the affinity protein can be applied as a purification element or a connection component, preferably as a purification element.

Preferably, the biomagnetic microsphere also becomes an affinity protein magnetic microsphere or an affinity protein magnetic bead.

Preferably, on a basis of the biomagnetic microspheres provided by the second aspect of the present application, the purification element is an affinity protein, the biomagnetic microsphere further comprises an avidin connected to the affinity protein, and a biotin connected to the avidin, the biotin connects to the branched-chain of the polymer; wherein, the purification element connects to the branched-chain of the polymer through a connection component, and the connection component comprises an affinity complex formed by the biotin and the avidin.

Preferably, the affinity protein is one of Protein A, Protein G, Protein L, or a modified protein thereof.

A fifth aspect of the present application provides a preparation method for the biomagnetic microsphere provided by the first aspect of the present application, comprising following steps:

(1) performing a chemical modification to the magnetic microsphere body, introducing an amino group to the outer surface of the magnetic microsphere body to form an amino-modified magnetic microsphere A; when the magnetic microsphere body is a magnetic material encapsulated by $SiO_2$, preferably a coupling agent is an aminated silane coupling agent;

preferably, performing the chemical modification to the magnetic microsphere by a coupling agent;

when the magnetic microsphere body is a magnetic material encapsulated by $SiO_2$, a silane coupling agent may be applied to perform the chemical modification to the magnetic microsphere body; the silane coupling agen is preferred to be an aminated silane coupling agent;

(2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B;

(3) under a condition of not adding a cross-linking agent, by a polymerization of a carbon-carbon double bond, a plurality of acrylic monomer molecules (such as a sodium acrylate) are polymerized, and an acrylic polymer obtained has a linear backbone and a branched-chain containing a functional group. The polymer covalently couples to the outer surface of the magnetic microsphere B through one end of the linear backbone to form an acrylic polymer modified magnetic microsphere C;

a definition of the acrylic monomer molecule and the functional group in the branched-chain of the polymer are shown in the "Nouns and Terms" section;

preferably, the functional group is a carboxyl group, a hydroxyl group, an amino group, a sulfhydryl group, a formate, an ammonium salt, a salt form of a carboxyl group, a salt form of an amino group, a formate group, or a combination of the functional groups thereof; the "combination of the functional groups" refers to the functional groups contained in all branched-chains of all the polymers on the outer surface of the magnetic microsphere, may have one or more types, which is consistent with a meaning of "combination of functional groups" as defined in the first aspect;

preferably, the functional group is a specific binding site;

(4) covalently coupling the biotin or the biotin analog to the end of the branched-chain of the polymer through the functional group contained in the branched-chain of the polymer, to obtain a biomagnetic microsphere combined with the biotin or the biotin analog (a biotin magnetic microsphere).

A sixth aspect of the present application provides a preparation method for the biomagnetic microsphere provided by the second aspect of the present application, comprising following steps:

(i) providing the biomagnetic microspheres according to claim 1; which may be prepared by adopting the steps (1) to (4) in the fifth aspect;

(ii) connecting the purification element to the biotin or the biotin analog at the end of the branched-chain of the polymer on the biomagnetic microsphere to obtain a biomagnetic microsphere having the purification element bound.

A seventh aspect of the present application provides a preparation method for the biomagnetic microsphere provided by the second aspect of the present application, comprising following steps:

(i) providing the biomagnetic microspheres according to claim 1; which may be prepared by adopting the steps (1) to (4) in the fifth aspect;

(ii) taking a covalent ligation complex of the avidin or the avidin analog and the purification element (including an avidin-purification element covalent ligation complex) as a raw material for providing the purification element, and binding to an end of a branched-chain of a polymer, forming a binding action of an affinity complex of the biotin or the biotin analog and the avidin or the avidin analog to obtain the biomagnetic microsphere with the purification element;

independently and optionally, comprising (6) sedimenting the biomagnetic microspheres by a magnet, removing a liquid phase and cleaning;

independently and optionally, comprising a replacement of the purification element, which can be achieved by replacing the covalent ligation complex of the avidin or the avidin analog with the purification element.

An eighth aspect of the present application provides a preparation method for the biomagnetic microsphere provided by the fourth aspect of the present application, comprising following steps:

(1) performing a chemically modification to the magnetic microsphere body, introducing an amino group to the outer surface of the magnetic microsphere body to form an amino-modified magnetic microsphere A; when the magnetic microsphere body is a magnetic material encapsulated by $SiO_2$, preferably a coupling agent is an aminated silane coupling agent;

preferably, performing the chemical modification to the magnetic microsphere by adopting a coupling agent;

when the magnetic microsphere body is a magnetic material encapsulated by $SiO_2$, a silane coupling agent may be applied to performing the chemical modification to the magnetic microsphere body. The silane coupling agent is preferred to be an aminated silane coupling agent;

(2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B;

(3) under a condition of not adding a cross-linking agent, by a polymerization of a carbon-carbon double bond, parforming a polymerization to a plurality of acrylic monomer molecules (such as a sodium acrylate), and an acrylic polymer obtained has a linear backbone and a branched-chain containing a functional group. The polymer covalently couples to the outer surface of the magnetic microsphere B through one end of the linear backbone to form an acrylic polymer modified magnetic microsphere C;

preferably, the functional group is a specific binding site;

a plurality of other preferred embodiments on the functional group are as same as that in the first aspect;

(4) covalently coupling the biotin through the functional group contained in the branched-chain of the polymer, to obtain a biotin-modified biomagnetic microsphere D (a biotin magnetic microsphere);

(5) by a specific binding between the biotin and the avidin, binding an avidin-affinity protein covalent ligation complex E to the end of the branched-chain of the polymer, by a binding action of the affinity complex between the biotin and the avidin, a biomagnetic microsphere F is obtained (the biomagnetic microsphere F has an affinity protein bound, which is an affinity protein magnetic microsphere);

independently and optionally, comprising (6) sedimenting the biomagnetic microspheres F by a magnet, removing a liquid phase and washing;

independently and optionally, comprising a step (7), replacing the avidin-affinity protein covalent ligation complex E.

A ninth aspect of the present application provides use of the biomagnetic microsphere described in the first to the fourth aspects of the present application in separating and purifying a protein substance;

preferably, use of the biomagnetic microsphere in the separating and purifying an antibody substance;

the antibody substance refers to a protein substance containing an antibody or an antibody fragment, including but not limited to, an antibody, an antibody fragment, an antibody fusion protein, and an antibody fragment fusion protein;

when the purification element connects to the end of the branched-chain of the polymer through the connection component containing the affinity complex, the use may optionally comprise a regeneration of the biomagnetic microsphere, that is, comprising a replacement of the purification element.

A tenth aspect of the present application provides a use on the biomagnetic microsphere described in the first to the fourth aspects of the present application in separating and purifying a protein substance, especially a use in separating and purifying an antibody, an antibody fragment, an antibody fusion protein, and an antibody fragment fusion protein;

the purification element is an affinity protein;

preferably, the affinity protein connects to the branched-chain of the polymer in a way of biotin-avidin-affinity protein;

when the affinity protein connects to the end of the branched-chain of the polymer through the connection component containing the affinity complex (such as the biomagnetic microsphere described in the fourth aspect), the use may further comprise optionally a regeneration and reuse of the biomagnetic microspheres, that is, comprising a replacement and reuse of the affinity protein.

An eleventh aspect of the present application provides a biomagnetic microsphere; an outer surface of the magnetic microsphere body has at least one polymer with a linear backbone and a branched-chain arranged, an end of the linear backbone is fixed onto the outer surface of the magnetic microsphere body, a plurality of other ends of the polymer is free from the outer surface of the magnetic microsphere body, and an end of the branched-chain of the polymer on the biomagnetic microsphere has a purification element connected; the purification element is selected from

an avidin-type tag, a polypeptide-type tag, a protein-type tag, an antibody-type tag, an antigen-type tag, or a combination thereof;

preferably, the avidin-type tag is avidin, a biotin-binding avidin analog, a biotin analog-binding avidin analog, or a combination thereof;

preferably, the avidin is a streptavidin, a modified streptavidin, a streptavidin analog, or a combination thereof;

preferably, the polypeptide-type tag is selected from any one of following tags or a variant thereof: a CBP tag, a histidine tag, a C-Myc tag, a FLAG tag, a Spot tag, a C tag, an Avi tag, a Streg tag, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof. The Streg tag comprises WSHPQFEK and a variant thereof;

preferably, the protein tag is selected from any one of following tags or a variant protein thereof: an affinity protein, a SUMO tag, a GST tag, an MBP tag and a combination thereof;

preferably, an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has an affinity protein connected;

preferably, further, a skeleton of the branched-chain between the affinity protein and the linear backbone of the polymer has a binding action of the affinity complex existing;

more preferably, the affinity protein is selected from Protein A, Protein G, Protein L, modified Protein A, modified Protein G, modified Protein L and a combination thereof.

A plurality of major advantages and active effects of the present application comprise:
one of a plurality of cores of the present application is a biomagnetic microsphere structure, the magnetic microsphere body has a plurality of polymer molecules with the linear backbones fixed covalently on an outer surface thereof, and the polymer molecules further have a large number of functional branched-chains, while the functional branched-chains have a plurality of purification elements connected (including but not limited to biotin, avidin, affinity protein, polypeptide tags, protein tags, and more.). Through the structures stated above, a large amount of purification elements suspended on a side end of the linear backbone of the polymer is provided on the outer surface of the biomagnetic microsphere, which not only avoids a high retention ratio caused by a traditional network structure, but also overcomes a limitation of a specific surface area, being able to provide a big number of binding sites for a target (such as an antibody). Wherein a type of the purification element can be selected according to a type of a substance to be purified. When the purification element is in a form of an affinity complex, and connects to a branched-chain of a polymer in a non-covalent strong interaction, further, the skeleton of the branched-chain between the purification element (such as avidin) and the linear backbone of the polymer will further have a binding action of the affinity complexes, making the purification elements (such as the affinity proteins) easily to be replaced. When a target for separation and purification is an antibody protein, the purification element is normally an affinity protein. It can be understood by combining with the part of preparing the magnetic microspheres and explaining of the principle.

The core of the present application further lies in a construction process (a preparation method) of the biomagnetic microsphere structure mentioned above: by a chemical modification to the outer surface, a plurality of binding sites are provided on the outer surface of the magnetic bead (the outer surface of the biomagnetic microsphere body), followed by connecting covalently a polymer molecule to a single binding site on the outer surface of the magnetic bead, and the polymer molecule connects covalently to the single binding site on the outer surface of the magnetic bead through one end of a linear backbone, there are a large number of side branched-chains distributed along the linear backbone, and the side branched-chains have a plurality of new binding sites carried, so as to achieve multiple, dozens, hundreds, or even thousands amplifications of the binding sites, followed by connecting a specific purification element to the new binding sites on the side branched-chains of the polymer, according to a specific purification requirement, so as to realize a capture of a corresponding specific target molecule (especially a biochemical molecule, including but not limited to an antibody-like protein molecule). In addition, the single binding site of the biomagnetic microsphere can connect covalently to only one linear polymer backbone or two or more linear backbones, as long as not causing a chain stacking and increasing a retention ratio.

Preferably, one binding site has only one linear backbone connected, this embodiment will provide a larger space for the linear backbone.

Or preferably, one binding site has only two linear backbones connected, this embodiment will provide a larger space for the linear backbone as possible.

The major advantages and active effects of the present application further comprise:

(1) A structural design of the present application, by the polymers carrying a big number of special structures of the branched-chain, covering the surface of the magnetic microsphere, overcomes a limitation of the specific surface area and provides a large number of binding sites for the purification element, amplifying a number of the purification element being able to bind onto the surface of the magnetic microsphere ten times, dozens of times, hundreds of times, or even thousands of times, so as to achieve binding a big number of a target, a preferred target is a protein; thus the biomagnetic microspheres can High-efficiently capture the targets (including the target proteins, including but not limited to the antibody, the antibody fragment, or a fusion protein thereof) from a mixed system onto the magnetic microspheres, achieving a high-throughput binding, or a high-throughput separation .

(2) A flexibility of a polymer chain can be utilized, the polymer chain is able to swing flexibly in a reaction and purification mixed system, expanding a movable space of the purification element, increasing a capture rate and a binding amount of the protein, and promoting a rapid and fully combination to a target, achieving a high efficiency and a high throughput.

(3) The structural design of the present application enables the biomagnetic microspheres to achieve an efficient elution of a target substance having been purified (such as a target protein, including but not limited to, an antibody, an antibody fragment, or a fusion protein thereof) during an elution, reducing effectively a retention time and a retention ratio of the target substance, and achieving a high efficiency and a high yield. The purification element is able to attach to the end of the branched-chain of the polymer, on one hand, a structure of the polymer will not form a network structure, thus will not cause branches stacking, which can avoid to have a discontinuous space or a dead end appear, and avoid a high retention period and a high retention ratio caused by a traditional network structure; on another hand, the branched-chain of the polymer further acts as a spacer, making the purification element be able to be fully distributed in a mixed system, and away from the surface of the magnetic microsphere or an internal skeleton of the polymer, which not only increases a target capture efficiency, in a subsequent elution step, It can also reduce effectively the retention period and the retention ratio of the target substance and achieve a separation having a high-throughput, a high-efficiency, and a high-proportion. The structural design of the present application can not only utilize a high flexibility of the linear backbone, but also have an advantage of a high magnification of the number of the branches, being able to realize a better combination of high speed and high throughout, as well as a separation of high efficiency and high ratio (high yield).

(4) The purification element (such as the affinity protein) of the biomagnetic microspheres of the present application can connect to the end of the branched-chain of the polymer on the outer surface of the magnetic bead, by a strong non-covalent binding force by means of an affinity complex. When it is needed to update or replace the purification element (such as the affinity protein), the purification element can be eluted from the microspheres and a new purification element can be recombined, easily and quickly, thus restoring the purification performance of the magnetic microspheres quickly, therefore, the biomagnetic microspheres can be regenerated and reused for a plurality of times, thereby reducing a cost of the separation and purification.

(5) The biomagnetic microspheres of the present application are convenient to operate and use. When separating the magnetic microspheres combined with a target object from a system, an operation is convenient, only a small piece of magnet is needed to efficiently manipulate a aggregation state and a position of the magnetic microspheres, so as to achieve a fast dispersion or a fast precipitation of the magnetic microspheres in a solution, making the separation and purification of a target substance (such as an antibody) simple and fast, no large-scale experimental equipments including a high-speed centrifuge is required, and the cost of separation and purification is greatly reduced.

(6) The biomagnetic microspheres provided by the present application has a wide usage, and the purification element is selectable. It is possible to carry a corresponding purification element on a magnetic microsphere system flexibly, according to a type of a specific purification substrate, before achieving a capture of a specific target molecule (especially a protein substance). For example, it is possible to select an affinity protein in a targeted manner, and it can be applied to the antibody substances generally on a large scale, including but not limited to the separation and purification of an antibody, an antibody fragment, or a fusion protein thereof.

BRIEF DESCRIPTION OF THE DRAWINGS

FIG. 1 illustrates a schematic structural diagram on a biological magnetic microsphere according to a first aspect of the present application. Wherein, the magnetic microsphere body is taking a $SiO_2$ encapsulated $Fe_3O_4$ as an example, and the purification element is taking biotin as an example. Wherein, a number of the polymer molecules (4) is merely for a purpose of simplicity and clarity, instead of meaning that a number of the polymer molecules on the outer surface of the magnetic microsphere is limited to 4, instead, the number shall be controlled and adjusted according to a content of each raw material in a preparation process. Similarly, a number of the branched-chains suspended at a side end of the linear backbone is for an illustration only, instead of a limitation to the number of the branched-chains on a side of a molecular of the polymer in the present application;

FIG. 2 illustrates a schematic structural diagram on the biomagnetic microsphere provided by the fourth aspect of the present application. Protein A is applied as the purification element, the Protein A is bound to an end of the branched-chain in a brush-like structure by a method of "biotin-avidin-Protein A". Wherein, the biomagnetic microsphere body is taken $Fe_3O_4$ encapsulated by $SiO_2$ as an example. Wherein, the number of polymer molecules and the number of the branched-chains at a side end of the backbone are for an illustration only, instead of limiting the number of the branched-chains in the polymer molecules of the present application.

FIG. 3 illustrates a flow chart on the preparation method for the biomagnetic microspheres provided by the fourth aspect of the present application. Wherein, a preparation process from an amino-modified magnetic microsphere A to a biomagnetic microsphere D is corresponding to the preparation method for the biomagnetic microspheres provided by the fifth aspect of the present application.

FIG. 4. Illustrates an experimental result on a comparison of the RFU values before and after binding a biomagnetic microsphere D (a biotin magnetic bead) to Protein A-eGFP-avidin and incubating once. Preparing a Protein A-eGFP-avidin (SPA-eGFP-avidin) by an *in vitro* protein synthesis system, and obtaining a supernatant after an IVTT reaction (abbreviated as IVTT supernatant), before comparing a change of a solution RFU value before and after binding to the biomagnetic microsphere D. Among the two Protein A-eGFP-avidin, the avidin corresponding to NO 1 is Streptavidin, and the avidin corresponding to NO 2 is Tamvavidin2. "Total" corresponds to the RFU value of the IVTT supernatant before the binding (before the biomagnetic microsphere treatment), and "Supernatant" corresponds to the RFU value of the IVTT supernatant after the binding (after the biomagnetic microsphere treatment).

FIG. 5 illustrates an experiment result of a biomagnetic microsphere F (a Protein A magneticsphere) prepared: a measuring result of the RFU values; the Protein A-eGFP-Avidin were bound in a saturation. The biomagnetic microsphere D was combined repeatedly with a solution obtained after an IVTT reaction for expressing the Protein A-eGFP-avidin (abbreviated as IVTT supernatant), before obtaining the biomagnetic microsphere F, which is combined with the Protein A-eGFP-avidin in a saturation. Wherein, 1 corresponds to an avidin of Streptavidin; 2 corresponds to an avidin of Tamvvidin2; the Supernatant corresponds to an IVTT supernatant without being processed by a biomagnetic microsphere; the flow-throughl, the flow-through2, and the flow-through 3 are flow-through liquids 1,2,3 obtained sequencially by the biomagnetic microsphere continuously incubating (capturing and binding) and eluting (decombination and releasing) with the avidin-Protein A three times, each time adopting a same source, a same amount of the IVTT supernatant.

FIG.6 illustrates an experimental result of the biomagnetic microsphere F separating and purifying an antibody. Specifically, the avidin in the biomagnetic microsphere F is Streptavidin; the biomagnetic microsphere F is incubated with an antibody IgG solution before performing an elution, the antibody IgG is captured and separated from an antibody solution, before being eluted and released into an eluent, and the eluent containing the antibody having been purified is then subjected to an SDS-PAGE test. Wherein lanes 1, 2, 3, and 4 correspond to a 4 antibody elution bands when a column bed volume of the SPA-magnetic microspheres is 2 μl, 6 μl, 18 μl, and 54 μl, respectively; and lane 5 is a positive control of an antibody eluted band obtained from a commercially available Sangon Bioengineering (Shanghai) Co., Ltd. (hereinafter referred to as: Sangon Company) with a Protein A agarose column bed volume of 7.5 microliters. Wherein an M corresponds to a Marker molecular weight marker.

FIG.7 illustrates an experimental result of a repeated use of the biomagnetic microsphere F. Specifically, the biomagnetic microsphere F and an antibody solution were encubated (combining the antibody), washed and eluted (releasing the antibody), repeating for 3 times, before an SDS-PAGE electrophoresis test to the eluent is performed to obtain the result. Wherein lanes 1, 2, 3, and 4 correspond to a 4 antibody elution bands when a column bed volume of the SPA-magnetic microspheres is 2 μl, 6 μl, 18 μl, and 54 μl, respectively; and lane 5 is a positive control of an antibody eluted band obtained from a commercially available Sangon Bioengineering (Shanghai) Co., Ltd. (hereinafter referred to as: Sangon Company) with a Protein A agarose column bed volume of 7.5 microliters. Wherein M corresponds to a Marker molecular weight marker.

FIG.8 illustrates an experimental result on regenerating the biomagnetic microsphere, specifically, a RFU test result and adopting a Protein A-eGFP-Tamvavidin2. The biomagnetic microsphere D was combined repeatedly with a solution obtained after the IVTT reaction of the Protein A-eGFP-avidin, to obtain a biomagnetic microsphere F(1) combined by and saturated with the Protein A-eGFP-avidin. The Protein A-eGFP-avidin was then eluted from the biomagnetic microsphere D, and the biomagnetic microsphere D was combined again with a fresh solution obtained after the IVTT reaction of the Protein A-eGFP-avidin, before a biomagnetic microsphere F(2) combined by and saturated with the Protein A-eGFP-avidin again is obtained. Repeating the steps stated above, and obtaining a biomagnetic microsphere F(3) combined by and saturated with the Protein A-eGFP-avidin a third time. Wherein, the Supernatant corresponds to an IVTT supernatant without being processed by a biomagnetic microsphere; the flow-throughl, the flow-through2, and the flow-through 3 are flow-through liquids 1,2,3 obtained sequencially by the biomagnetic microsphere continuously incubating with the avidin-Protein A three times, each time adopting a same IVTT supernatant (newly taken).

FIG.9 illustrates an experimental result on regenerating a biomagnetic microsphere and performing an antibody isolation and purification after the regeneration; adopting a Protein A-eGFP-Tamvavidin2. Specifically the biomagnetic microspheres F combined and saturated with the Protein A-eGFP-avidin were eluted by a denaturing buffer, and the eluent containing the Protein A-eGFP-avidin being eluted was tested by SDS-PAGE. Lanes 1, 2, and 3 represent three bands of the eluent containing the Protein A-eGFP-avidin being eluted from the biomagnetic microspheres F, after a first saturation, binding, and elution, a second saturation, binding, and elution, a third saturation, binding, and elution respectively. Lane 4 represents a band of a purified antibody protein eluted by an elution buffer after a third regenerated biomagnetic microsphere F was incubated with a commercial newborn calf serum. Wherein M corresponds to a Marker molecular weight marker.

FIG. 10 illustrates a loading test result of the RFU values of a biomagnetic microsphere G (proteinG magnetic bead) to Protein G. The biomagnetic microspheres D were incubated three times with the IVTT supernatant of the Protein G-eGFP-avidin to obtain the biomagnetic microspheres G having the Protein G-eGFP-avidin bound and saturated. Wherein, the Supernatant corresponds to an IVTT supernatant without being processed by a biomagnetic microsphere; three corresponding flow-through liquids were obtained after three incubations: FT1 (flow-through liquid 1), FT2 (flow-through liquid 2), FT3 (flow-through liquid 3).

FIG.11 illustrates an experimental result on separating and purifying an antibody by a biomagnetic microsphere G (a ProteinG magnetic bead): incubating the biomagnetic microspheres G with the antibody IgG solution before performing an elution, the antibody IgG is then captured and separated from the antibody solution, before being eluted and released into an eluent, the eluent containing the purified antibody is then subjected to an SDS-PAGE test. Wherein M corresponds to the Marker molecular weight marker.

FIG. 12 illustrates a RFU value test result of a biomagnetic microsphere H (a magnetic bead taking an antiEGFP nanobody) binding to the eGFP protein. Specifically, the biomagnetic microsphere H was incubated with an IVTT supernatant of the eGFP protein to bind the eGFP protein. Wherein, the Total corresponds to the IVTT supernatant without been processed by a biomagnetic microsphere; Flow-through corresponds to a flow-through liquid having been incubated once.

FIG. 13 illustrates an experimental result of a biomagnetic microsphere H (an antiEGFP magnetic bead) separating and purifying of the eGFP protein. Specifically, eluting after incubating the biomagnetic microsphere H with an eGFP protein solution, capturing and separating the eGFP protein from an original solution, before eluting and releasing into an eluent, then a SDS-PAGE test result for a corresponding eluent containing a purified eGFP protein is collected. Wherein M corresponds to the Marker molecular weight marker.

## DETAILED DESCRIPTION OF EMBODIMENTS

In order to make the purpose, technical solution and the advantages of the present disclosure clearer and more explicit, further detailed descriptions of the present application are stated hereafter, referencing to the attached drawings and some preferred embodiments of the present application. It should be understood that the detailed embodiments of the application described here are used to explain the present application only, instead of limiting the present application.

A plurality of experimental methods without listing a specific experimental condition in the following embodiments, are preferentially according to, or referencing to the conditions of the specific embodiment guidelines described above, then according to a regular condition, such as a plurality of experimental conditions described in Sambrook et al., Molecular Cloning: Laboratory Manual (New York: Cold Spring Harbor Laboratory Press, 1989); Cell-free Protein Synthesis Experiment Manual, edited by Alexander S.Spirin and James R. Swartz; Cell-free protein synthesis: methods and protocols [M]. 2008", or as suggested by the manufacturer.

Unless otherwise stated, the percentages and the parts mentioned in the present applicaiton are weight percentages and parts by weight.

Unless otherwise specified, the materials and reagents used in the embodiments of the present application are all commercially available products.

The temperature units in this application are all degrees Celsius (°C), unless otherwise specified.

Nucleotide and/or Amino Acid Sequence Listing

SEQ ID NO: 1, a nucleotide sequence of Protein A, 873 bases in length.

SEQ ID NO: 2, a nucleotide sequence of tamavidin2, 423 bases in length.

SEQ ID NO: 3, a nucleotide sequence of mEGFP, 714 bases in length.

SEQ ID NO: 4, a nucleotide sequence of an antibody binding region of Protein G, 585 bases in length.

SEQ ID NO: 5, an amino acid sequence of a Nanobody anti-eGFP, 117 amino acids in length.

SEQ ID NO: 6, a nucleotide sequence of mScarlet, 693 bases in length.

Nouns and terms

Following is an explanation or description of the meanings of some related "nouns" and "terms" used in the present application, so as to facilitate a better understanding of the present application. A corresponding explanation or illustration applies to an entirety of the application, both below and above. When a reference is involved in the present application, the definitions of related terms, nouns, and phrases in the cited documents are also cited together. However, in an event of having a conflict with the definitions in the present application, the definitions in the present application shall prevail. In an event of a conflict between the definitions in the cited documents and the definitions in the present application, it does not affect that, for the cited ingredients, substances, compositions, materials, systems, formulations, types, methods, equipment, and more, the content determined in the cited documents shall prevail.

Magnetic bead: a microsphere with a fine particle size having a strong magnetism or being able to be strongly magnified, which can also be described as magnetic bead, a preferred diameter size is 0.1 μm to 1000 μm. A plurality of embodiments of the magnetic bead of the present application comprises, but not limited to: a magnetic microsphere A, a magnetic microsphere B, a magnetic microsphere C, a magnetic microsphere D (a biotin magnetic bead), a biomagnetic microsphere F (a Protein A magnetic bead), a biomagnetic microsphere G (a Protein G magnetic bead), a biomagnetic microsphere H (a magnetic bead with antiEGFP nanobody), a biomagnetic microsphere K (an antibody magnetic bead).

Magnetic microsphere body: a magnetic bead with a plurality of modified sites (a magnetic microsphere having a plurality of binding sites), including a silica-encapsulated magnetic material particle, more particularly an aminated silica-encapsulated magnetic material particle.

Magnetic microsphere A: an amino-modified magnetic microsphere.

Magnetic microsphere B: a magnetic microsphere containing a plurality of carbon-carbon double bonds.

Magnetic microsphere C: an acrylic polymer modified magnetic microsphere.

Biotin Magnetic Bead: a magnetic bead having a biotin or a biotin analog bound, being able to bind specifically to a substance having an avidin-type tag. A plurality of advantages include being able to be expressed in an integrated manner in a form of a fusion protein, after a target protein is labeled with avidin or a protein mutant of the avidin, making an application method simple. This is also known as a biotin magnetic microsphere. The Biotin or the biotin analogs here may serve as a purification element or as a linking component.

Biomagnetic Microsphere D: A magnetic microsphere having a biotin or a biotin analog bound, a biotin magnetic bead. The biotin here can be applied as a purification element or as a linking component.

Avidin Magnetic Bead: a magnetic bead having an avidin or an avidin analog bound, being able to specifically bind to a substance with a biotin-type tag. This is also known as an avidin magnetic microsphere.

Biomagnetic Microspheres F: a magnetic microsphere having a Protein A bound, a Protein A magnetic bead, can be composed of a biotin-modified biomagnetic microspheres D and an avidin-Protein A covalent ligation complex E.

Affinity protein magnetic bead: a magnetic microsphere having a plurality of affinity proteins bound, can be applied to separating and purifying an antibody substance. This is also known as an affinity protein magnetic microsphere.

Biomagnetic Microsphere G: a magnetic microsphere having a Protein G-bound, a Protein G magnetic bead. In an embodiment, it can be composed of a biotin-modified biomagnetic microsphere D and an avidin-Protein G covalent ligation complex.

Biomagnetic Microsphere K: a magnetic bead having an antibody-type tag bound, may be applied to separating and purifying a target that can specifically bind to. This is also known as an antibody magnetic microsphere or an antibody magnetic bead.

Nanobody Magnetic Bead: a magnetic bead having a plurality of nanobodies bound, may be applied to separating and purifying a target that can specifically bind to, also known as a Nanobody Magnetic Microspher.

Biomagnetic Microsphere H: a nanobody magnetic bead, a magnetic microsphere having a nanobody of antiEGFP bound (an antiEGFP magnetic bead), can be combined by an avidin-antiEGFP covalent ligation complex.

A polymer, in a broad sense, comprises oligomers and polymers in the present application, having at least three structural units or a molecular weight of at least 500 Da (the molecular weight can be characterized by a suitable method, such as a number average molecular weight, a weight average molecular weight, a viscosity average molecular weight, and more.).

A polyolefin chain: referencing to a polymer chain that is only covalently linked by carbon atoms without heteroatoms. In the present application, a backbone of polyolefin in a comb-like structure is mainly involved; for example, the linear backbone of an acrylic polymer.

An acrylic polymer: referencing to a homopolymer or a copolymer having a unit-structure of -(COO-)-C-, and a copolymerization form of the copolymer is not particularly limited, as long as being able to provide a linear backbone and a metered side group COO-; the linear backbone of the acrylic polymer may have a heteroatom contained. Wherein a plurality of other substituents may exist on a carbon-carbon double bond, as long as it does not affect a progress of a polymerization reaction, such as a methyl substituent (corresponding to $-CH_3C(COO-)-C-$). Wherein, COO- may exist in a form of -COOH, or in a form of a salt (such as a sodium salt), may further be in a form of formate (preferably alkyl formate, such as a methyl formate $-COOCH_3$, an ethyl formate - $COOCH_2CH_3$; may further be a hydroxy ethyl formate $-COOCH_2CH_2OH$) and more. A specific structural form of the -C(COO-)-C-unit structure includes but not limited to - any one of: $CH(COOH)-CH_2-$, $-CH(COONa)-CH_2-$, $-MeC(COOH)-CH_2-$, $-MeC(COONa)-CH_2-$, $-CH(COOCH_3)-CH_2-$, $-CH(COOCH_2CH_2OH)-CH_2-$, $-MeC(COOCH_3)-CH_2-$, - $MeC(COOCH_2CH_2OH)-CH_2-$ and more or a combination thereof. Wherein, Me is methyl. On a linear backbone of a polymer molecule, there may be only one of the unit-structures mentioned above (corresponding to a homopolymer), or two or more unit-structures (corresponding to a copolymer).

An acrylic monomer molecule: a monomer molecule that can be applied to synthesizing the acrylic polymer mentioned above, having a basic structure of C(COO-)=C, for example, $CH(COOH)=CH_2$, $CH(COONa)=CH_2$, $CH_3C(COOH)=CH_2$, $CH_3C(COONa)=CH_2$, $CH(COOCH_3)=CH_2$, $CH(COOCH_2CH_2OH)=CH_2$, $CH_3C(COOCH_3)=CH_2$, $CH_2C( COOCH_2CH_2OH)=CH_2$ and more.

A branched-chain: a chain in the present appliction that is attached to a branch point and has an independent end. In the present application, a branched-chain and a side branched-chain have a same meaning and can be used interchangeably. In the present application, the branched-chain refers to a side chain or a side group bonded on the linear backbone of the polymer. There is no special requirement to a length and a size of the branched-chain, may be a plurality of short branched-chains including carboxyl, hydroxyl, amino and more, may further be a long branched-chain containing relatively more atoms. There is no special requirement for a structure of the branched-chain, which may be linear or a branched-chain having a branched structure. The branched-chain may further contain a plurality of additional side chains or side groups. A plurality of structural charaters including a number, a length, a size, a degree of re-branching and more of the branch chain have no limitations as long as not forming a network structure, not causing an accumulation of the branched-chains to increase a retention ratio, so as to allow a flexible swing of the linear backbone smoothly..

A branched-chain skeleton: the branched-chain skeleton consists of a plurality of skeleton atoms connected in a sequence by a covalent bond or a non-covalent bond, and is sequentially connected to the backbone of the polymer from the end of the branched-chain. Through the branched-chain skeleton, the functional groups at the ends of the polymer are attached to the backbone of the polymer. An intersection of the branched-chain skeleton and the backbone is also a branched point from which the branched-chain is drawn out. In an embodiment, the branched-chain skeleton between the purification element and the linear backbone of the polymer, taking the avidin protein as the purification element as an example, the avidin protein at the end of the branched-chain of the polymer may connect to a polyolefin backbone of the polymer through avidin, biotin, a propylenediamine residue (-NH-$CH_2CH_2CH_2$-NH-), a carbonyl group (a residue after a carboxyl group undergoes an amidation reaction) in turn.

A plurality of ends of the branched-chains, comprise the ends of all branched-chains. For the linear backbone, in addition to the end being fixed at the magnetic microsphere body, another end of the linear backbone must connect to a branched point, therefore, it is also broadly included in a category of "end of a branched-chain" of the present application. Therefore, the polymer attached to the outer surface of the magnetic microsphere body of the present application has at least one branched point.

A functional group of the branched-chain of the polymer: refers to a group having a reaction activity, or having the reaction activity after being activated, being able to have a covalent react directly with a reactive group of a plurality of other raw materials, or have the covalent react directly with the reactive group of the other raw materials after being activated, before generating a covalent bond connection. The functional group of the branched-chain of the polymer, in a preferred embodiment, is a specific binding site.

A direct connection method refers to a connection method in which an interaction occurs directly without an aid of a plurality of spacer atoms. A form of the interaction comprises, but not limited to: covalent, non-covalent, or a combination thereof.

An indirect connection method, refers to a connection method with an aid of at least one connection component, wherein at least one spacer atom is involved. The connection component comprises, but not limited to, a linking peptide, an affinity complex, and more.

A plurality of "fixing" methods including "fixing", "fixed", "fixed onto", fixed on", means of a covalent binding method.

A plurality of connection methods, including "with", "connected with", "connected to", "binding", "bound", "capturing", "captured", and more, have no specifical limitations, including but not limited to, a covalent method, a non-covalent method and more.

A covalent method: directly bonded by a covalent bond. The covalent method comprises, but not limited to, a dynamic covalent method, and the dynamic covalent method refers to a manner of directly bonding with a dynamic covalent bond.

A covalent bond: comprising a plurality of common covalent bonds including an amide bond and an ester bond, further comprising a dynamic covalent bond with a reversible property. The covalent bond comprises a dynamic covalent bond. The dynamic covalent bond is a chemical bond with a reversible property, including but not limited to, an imine bond, an acylhydrazone bond, a disulfide bond, or a combination thereof. Those skilled in the chemical arts shall understand the meaning.

A non-covalent method: comprising a method of a supramolecular interaction including but not limited to, a coordination binding, an affinity complex interaction, an electrostatic adsorption, a hydrogen bonding, a $\pi$-$\pi$ overlap, and a hydrophobic interaction.

The supramolecular interaction: including but not limited to, the coordination binding, the affinity complex interaction, the electrostatic adsorption, the hydrogen bonding, the $\pi$-$\pi$ overlapping interaction, the hydrophobic interaction, and a combination thereof.

A connection component, also referred to as a linking group, refers to a component applied to connecting two or more non-adjacent groups, comprising at least one atom. A connection method between the connection component and an adjacent group is not particularly limited, including but not limited to, a covalent method, a non-covalent method and more. An internal connection method for the connection component is not particularly limited, including but not limited to, a covalent method, a non-covalent method and more.

A covalent connection component: a plurality of spaced atoms from one end of a connection component to another end are connected covalently.

A specific binding site: In the present application, the specific binding site refers to a group or a structural site on a branched-chain of the polymer having a binding function, the group or the structural site has a specific recognition and binding function to a specific target, a specific binding may be achieved through a plurality of binding actions or other interactions including coordination, complexation, an electrostatic force, a van der Waals force, a hydrogen bond, a covalent bond and other.

A covalent ligation complex: a compound that is directly or indirectly linked by a covalent means, also known as a covalent linker.

An avidin-purification element covalent ligation complex: a compound that is formed by a covalent linking method, with an avidin at one end and a purification element at another end, which are linked by a covalent bond directly, or through a covalent connection component connected indirectly.

An avidin-affinity protein covalent ligation complex E: an avidin-purification element covalent ligation complex with an affinity protein as the purification element; or an avidin-affinity protein complex E; a compound formed in a covalent connection method, has the avidin at one end and the affinity protein at another end, which are connected by a covalent bond directly or by a covalent linking group indirecty. The covalent connection method comprises, but not limited to, a covalent bond, a linking peptide, and more. In an embodiment, it ma be: a streptavidin-Protein A complex, a streptavidin-Protein A fusion protein, a streptavidin-enhanced green fluorescent protein-Protein A fusion protein (Protein A-eGFP-Streptavidin), a Protein A-eGFP-Tamvavidin2, a ProteinG-eGFP-avidin fusion protein, a ProteinG-eGFP-Tamvavidin2 fusion protein, and more.

An affinity complex: a non-covalent ligation complex formed by two or more molecules through a specific binding, relying on an extremely strong affinity, in an embodiment, which is a compound formed by an interaction of a biotin (or a biotin analog) and an avidin (or an avidin analog). A binding method for an affinity complex between the biotin and the avidin has been well known to those skilled in the art.

A purification substrate, also known as a target, a substance to be separated from a mixed system. The purification substrate in the present application is not particularly limited, but a preferred purification substrate is a protein substance (also referred to as a target protein now).

A purification element, is able to make a specific binding with a purification substrate, so as to capture the purification substrate, and further to separate the purification substrate from a mixed system. The purification element linked to the branched end of the polymer of the present application is a functional component having a function of binding the purification substrate. When the purification element is covalently linked to an adjacent group, it behaves as a functional group with a function of binding the purification substrate.

An affinity protein: binding specifically to a target protein with a relatively high affinity binding force. In an embodiment, it may be Protein A, Protein G, Protein L, modified Protein A, modified Protein G, modified Protein L, and more.

Protein A: a surface protein of 42 kDa, was originally found in cell wall of Staphylococcus aureus, which is encoded by a spa gene, and a regulation thereof is controlled by a DNA topology, a cellular osmolality, and a two-component system called ArlS-ArlR. Due to an ability thereof to bind an immunoglobulin, Protein A has been applied in a plurality of related researches in the field of biochemistry. The Protein A is able to bind to an antibody Fc specifically, and mainly used for an antibody purification, which can be selected from any commercially available products. "Protein A" and "SPA" are used interchangeably herein.

Protein G: an immunoglobulin-binding protein, expressed in *Streptococci* of a groups C and a group G, similar to the Protein A, but has a different binding specification. The Protein G is a cell surface protein of 65kDa (G148 Protein G) and 58kDa (C40 Protein G), through a specific binding to an antibody or a functional protein, Protein G is mainly used for antibody purification and can be selected from any commercially available products.

Protein L: restricted to binding to a plurality of antibodies containing a kappa ($\kappa$) light chain specifically. In humans and mice, most antibody molecules contain the kappa light chains and a remaining lambda ($\lambda$) light chain, which is mainly applied for an antibody purification, and can be selected from any commercially available products.

Biotin: being able to combine with the avidin, with a strong binding force and a good specificity.

Avidin: being able to combine with the biotin, with a strong binding force and a good specificity, including a streptavidin (SA), an analog thereof (including a Tamvavidin2, referred to as Tam2), a modified product thereof, a mutant thereof, and more.

A biotin analog: referring to a non-biotin molecule that can form a specific binding with the avidin, similar to "avidin-biotin", preferably the biotin analog is a polypeptide or a protein, including a polypeptide containing a sequence of WSHPQFEK used in a series of Strep-tag® developed by the IBA Company (for example, ccccC Strep®II, Twin-Strep-tag® and more.), and a plurality of similar polypeptides containing the WNHPQFEK sequence. Wherein WNHPQFEK may be regarded as a mutated sequence of WSHPQFEK.

An avidin analog, referring to a plurality of non-avidin molecules that can form a specific binding with the biotin, which is similar to "avidin-biotin", and a preferred embodiment is a polypeptide or a protein. The avidin analog comprises, but not limited to, a plurality of derivatives of the avidin, a plurality of homologs of the avidin (homologous substances), a plurality of variants of the avidin, and more. In an embodiment, the avidin analog is Tamavidin1, Tamavidin2, and more. (refer to: FEBS Journal, 2009, 276, 1383-1397).

A biotin-type tag: the biotin-type tag contains a plurality of following units: biotin, a biotin analog being able to bind the avidin, a plurality of biotin analogs being able to bind an avidin analog, and a combination thereof. The biotin-type tag is capable of binding the avidin, the avidin analogs, or a combination thereof specifically. Therefore, the biotin-type tag can be applied to separating and purifying, including but not limited to, a plurality of proteins labeled by an avidin-type tag.

The avidin-type tag: the avidin-type tag contains a plurality of following units: avidin, an avidin analog being able to bind the biotin, a plurality of avidin analogs being able to bind the biotin analogs, and a combination thereof. The avidin-type tag is capable of specifically binding the biotin, the biotin analogs, or a combination thereof. Therefore, the avidin-type tag can be applied to separating and purifying, including but not limited to, a plurality of proteins being labeled by a biotin-type tag.

A polypeptide-type tag: the polypeptide-type tag of the present application refers to a tag containing a polypeptide tag or a derivative of a polypeptide tag. The polypeptide tag refers to a tag of a polypeptide structure composed of a plurality of amino acid units, and the amino acid may be a natural amino acid or an unnatural amino acid.

A protein-type tag: the protein-type tag of the present application comprises a tag containing a protein tag or a derivative of a protein tag. The protein tag refers to a tag of a protein structure composed of a plurality of amino acid units, and the amino acid may be a natural amino acid or an unnatural amino acid.

An antibody-type tag: the antibody-type tag of the present application refers to a tag containing an antibody-like substance, being able to bind to a corresponding target specifically, the target comprises an antigen. An embodiment of the antibody-type tag further comprises an antiEGFP nanobody that can bind to an eGFP protein specifically.

An antigen-type tag: The antigen-type tag of the present application refers to a tag containing an antigen-like substance, which can specifically bind to an antibody-like substance.

A peptide is a compound comprising two or more amino acids linked by a peptide bond. In the present application, the peptide and a peptide segment have a same meaning and can be used interchangeably.

A polypeptide, a peptide comprising 10 to 50 amino acids.

A protein, a peptide comprising more than 50 amino acids. A fusion protein is also a protein.

A derivative of a polypeptide, a derivative of a protein: any polypeptide or protein involved in the present application, unless otherwise specified (for example, a specific sequence is specified), should be understood that a derivative thereof is also included. The derivative of a polypeptide, the derivative of a protein, comprises at least a C-terminus tag, an N-terminus tag, or a C-terminus and an N-terminus tag. Wherein, the C-terminus refers to a COOH terminus, and the N-terminus refers to an NH2-terminus, those skilled in the art shall understand the meaning. The tag may be a polypeptide tag or a protein tag. An example of the tag comprises, but not limited to, a histidine tag (generally containing at least 5 histidine residues; such as 6×His, HHHHHH, or a 8×His tag), Glu-Glu, c-myc epitopes (EQKLISEEDL), a FLAG® Tag (DYKDDDDK), Protein C (EDQVDPRLIDGK), Tag-100 (EETARFQPGYRS), V5 epitope tag (V5epitope, GKPIPNPLLGLDST), VSV-G (YTDIEMNRLGK), Xpress (DLYDDDDK), Hemagglutinin (YPYDVPDYA) , β-galactosidase, thioredoxin, His-patch thioredoxin, IgG-binding domain, an intein-chitin binding domain, T7 gene 10, glutathione-S-transferase, (GST), green fluorescent protein (GFP), maltose binding protein (MBP) and more.

A protein substance, in the present application, broadly referring to a substance containing a polypeptide or a protein fragment. For example, a polypeptide derivative, a protein derivative, a glycoprotein, and more, are also included in a category of the protein substance.

An antibody, an antigen: the antibody and the antigen involved in the present application, unless otherwise specified, shall be understood that, further comprising domains, subunits, fragments, single chains, single chain fragments, and variants. In an embodiment, when it is related to the "antibody", unless otherwise specified, a fragment, a heavy chain, a heavy chain lacking a light chain (such as a nanobody), a complementarity determining region (CDR), and more, are further comprised. In an embodiment, when it is related to the "antigen", unless otherwise specified, an epitope and an epitope peptide are further comprised.

An antibody substance: in the present application, comprising, but not limited to, an antibody, an antibody fragment, a single chain of antibody, a fragment of a single chain, an antibody fusion protein, a fusion protein of an antibody fragment, and more, as well as a plurality of derivatives and variants thereof, as long as a specific binding action of the antibody-antigen can be generated.

An antigenic substance: in the present application, comprising but not limited to, an antigen known to those skilled in the art and a plurality of substances being able to exert an antigenic function and bind an antibody substance specifically.

An anti-protein antibody: referring to an antibody that can specifically bind to a protein.

An anti-fluorescent protein nanobody: referring to a nanobody being able to bind to a fluorescent protein specifically.

A homology, unless otherwise specified, referring to having at least 50% homology; preferably at least 60% homology, more preferably at least 70% homology, more preferably at least 75% homology, more preferably at least 80% homology, more preferably at least 85% homology, more preferably at least 90% homology; also such as at least 91%, at least 92%, at least 93%, at least 94%, at least 95%, at least 96%, At least 97%, at least 98%, at least 99% homology. In an embodiment, an object described is a homologous sequencs of an Ω sequence mentioned in the present specification. Homology here refers to a similarity in a sequence, and may be equivalent to an identity in a numerical value.

A Homologue: referring to a substance having a homologous sequence.

"Variant": referring to a plurality of substances having a different structure (including but not limited to a minor variation), but still retaining or substantially retaining an original function or performance. The variant comprises, but not limited to, a nucleic acid variant, a polypeptide variant, and a protein variant. A method to obtain a related variant comprises, but not limited to, a recombination of a structural unit, deletion or loss, insertion, translocation, substitution and more. The variant comprises, but not limited to, a modified product, a genetically modified product, a fusion product, and more. In order to obtain the genetically modified product, a method performing a genetic modification comprises but not limited to, a gene recombination (corresponding to a gene recombination product), a gene deletion or loss, an insertion, a frameshift, a base substitution and more. A gene mutation product, also known as a gene mutant, belongs to a type of the genetic modification product. A preferred way of the variant is a homologue.

A modified product: including but not limited to, a chemically modified product, an amino acid modification, a polypeptide modification, a protein modification, and more. The chemically modified product refers to a product modified by adopting a plurality of chemical synthesis methods including organic chemistry, inorganic chemistry, and polymer chemistry. An embodiment of a modification method comprises ionization, saltation, desalination, complexation, decomplexation, chelation, dechelation, addition reaction, substitution reaction, elimination reaction, insertion reaction, oxidation reaction, reduction reaction, post-translational modification, and more. A specific example of the modification method comprises a plurality of modification methods including oxidation, reduction, methylation, demethylation, amination, carboxylation, and sulfuration.

A mutant, unless otherwise specified in the present application, refers to a mutant product being able to still maintain or substantially maintain an original function or performance, and a number of a plurality of mutant sites is not particularly limited. The mutant comprises, but not limited to, a gene mutant, a polypeptide mutant, and a protein mutant. The mutant is a type of the variant. A plurality of methods obtaining a related mutant comprises, but not limited to, a recombination of a plurality of structural units, deletion or loss, insertion, translocation, substitution, and more. A structural unit of a gene is a base, a structural unit of a polypeptide and a protein is an amino acid. A plurality of types of the genetic mutations comprises, but not limited to, a gene deletions or loss, an insertion, a frameshift, a base substitution, and more.

A "modified" product: comprising but not limited to, a derivative, a modified product, a genetically modified product, a fusion product, and more, of the present application, can maintain an original function or performance, or can optimize or change a function or a performance thereof.

An eluent (take a target protein as an example): eluting a target protein; after an elution, the target protein exists in the eluent.

A washing solution (take the target protein as an example): eluting a plurality of impurities including an impurity protein; after an elution, the impurity protein is taken away by the washing solution.

Binding capacity: such as a binding capacity of a magnetic microsphere to a certain protein.

Affinity: A substrate concentration that a magnetic microsphere binds only 50% of the substrate, when using a plurality of substrate solutions having different concentration gradients.

IVTT: In vitro transcription and translation. An in vitro transcription and translation system is a cell-free protein synthesis system. The cell-free protein synthesis system uses exogenous target mRNA or DNA as a protein synthesis template, and by artificially controlling an addition of a plurality of substrates required for a protein synthesis and a plurality of substances related to the transcription and translation including a protein factor, achieves a target protein synthesis. The cell-free protein synthesis system of the present application is not particularly limited, and can be any cell-free protein synthesis system based on a yeast cell extract, an *Escherichia coli* cell extract, a mammalian cell extract, a plant cell extract, and an insect cell extract species or a combination thereof.

The present application, wherein "translation-related enzymes", (TRENs), refers to a plurality of enzyme substances required in a synthesis process from a nucleic acid template to a protein product, and not limited to a plurality of enzymes required in a translation process. A nucleic acid template: also known as a genetic template, refers to a nucleic acid sequence acting as a template for a protein synthesis, comprising a DNA template, an mRNA template and a combination thereof.

Flow-through liquid: a supernatant collected after the magnetic beads were incubated with a target protein-containing system, which has a plurality of residual target proteins contained that were not captured by the magnetic beads. For example, in Embodiment 2, after adding a solution containing a complex of avidin-affinity protein to an affinity column, collecting a solution passed through the column, including: flow-through liquid 1, flow-through liquid 2 and flow-through liquid 3 , represent respectively a solution of a first penetration, a solution of a second penetration and a solution of a third penetration.

RFU: Relative Fluorescence Unit.

eGFP: enhanced Green Fluorescence Protein. In the present application, the eGFP broadly comprises a wild type and a plurality of variants, including but not limited to, the wild type and a mutant.

mEGFP: an A206K mutant of the eGFP.

"Optionally", means that there may or may not be, and taking a technical solution being able to realize the present application as a selection criterion. In the present application, "optional mode" means that, a technical solution, as long as it is applicable to the present application, can be applied to implementing the present application.

In the present application, a plurality of preferred embodiments, including "prefer (including prefer, preferable, preferably, preferred, and more)", "preferred", "more preferred", "better", "most preferred" and more, do not constitute a limitation in a coverage scope and a protection scope of the present application, not intended to limit the scope and the embodiments of the present application, instead, it is only used to provide some embodiments as examples.

In the description of the present application, for "preferably one", "a preferred embodiment", "one preferred embodiment", "a preferred embodiment", "preferred embodiment", "in a preferred embodiment", "preferably", "more preferably", "further preferably", "for example", "as an example", "for example", "such as", "for example", and more, in a plurality of embodiments in the present application, do not constitute a limitation on the coverage scope and the protection scope of the present application, and the specific features described in various ways are included in at least one embodiment of the present application. In the present applicaiton, the specific features described in the various manners may be combined in any suitable manner in any one or more embodiments. In the present application, the technical features or technical solutions corresponding to a preferred manner may also be combined in any suitable manners.

The present application, wherein "any combination thereof' means, "greater than 1" in number and is intended to mean a group consisting of "optionally one, or optionally at least two".

In the present invention, "one or more", and the like, "at least one", "a combination thereof', "or a combination thereof', "combinations thereof', "or any combinations thereof', and the like, may be used interchangeably and refer to a quantity equal to "1" or "greater than 1".

In the present invention, "or/and", "and/or" means "optionally one or a combination thereof', and at least one.

A plurality of technical means in the prior art, as described in terms "usually", "conventional", "generally", "often", and more in the present application are all considered as reference to the content of the present application, and are all considered to be one of the preferred ways of part of the technical features of the present application, and it should be noted that no limitation on the coverage scope and the protection scope of the present application is made.

All documents referred to in the present application, and documents cited directly or indirectly, are incorporated by reference in this application as if each document is individually incorporated by reference.

It should be understood that, within the scope of the present application, the above technical features of the present application and the technical features specifically described in the following (including but not limited to the embodiments) may be combined with each other to form a new or a preferred technical solution as long as it can be used to practice the present application. Due to a space limitation, no more descriptions one by one will be stated again.

1. A first aspect of the present application provides a biomagnetic microsphere, the biomagnetic microsphere comprises a magnetic microsphere body, an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of biotins or biotin analogs connected.

The biomagnetic microsphere in the first aspect is also called a biotin biomagnetic microsphere or a biotin biomagnetic bead.

A typical structure of the biomagnetic microsphere is shown in FIG.1.

The biotin or the biotin analogue can be applied as a purification element, and can also be applied as a connection component to further connect a plurality of other types of purification elements.

Compared to a plurality of currently commonly used gel-like porous materials, most commercially available microspheres are adopting an agarose material. A porous material has a rich pore structure, thereby providing a large specific surface area, providing a high binding capacity for purifying a substrate, but correspondingly, when absorbing or eluting a protein, a protein molecule is necessary to enter or escape a complex pore channel inside the porous material additionally, which costs more time and the protein molecules are more easily retained. Comparing with the prior art, a plurality of binding sites for capturing a target protein provided by the present application uses only an outer surface space of the biological magnetic microsphere, and when carrying out an adsorption and an elution, does not need to pass through a complex mesh channel, and can be directly released into an eluent, thus greatly reducting a elution time, and improving an elution efficiency, reducing a retention ratio, and improving a purification yield.

1.1 Magnetic microsphere body

In the present application, a volume of the magnetic microsphere body may be any feasible particle sizes.

A smaller particle size helps to achieve a suspension of the magnetic microspheres in a mixing system, in contact with a protein product more sufficiently, increasing a capture efficiency and a binding rate of the protein product. In a plurality of preferred embodiments, a diameter size of the magnetic microsphere body is any one of particle size scales (a deviation may be ±25%, ±20%, ±15%, ±10%), or a range between any two particle size scales: 0.1 μm, 0.15 μm, 0.2 μm, 0.25 μm, 0.3 μm, 0.35 μm, 0.4 μm, 0.45 μm, 0.5 μm, 0.55 μm, 0.6 μm, 0.65 μm, 0.7 μm, 0.75 μm, 0.8 μm, 0.85 μm, 0.9 μm, 0.95 μm, 1 μm, 1.5 μm, 2 μm, 2.5 μm, 3 μm, 3.5 μm, 4 μm, 4.5 μm, 5 μm, 5.5 μm, 6 μm, 6.5 μm, 7 μm, 7.5 μm, 8 μm, 8.5 μm, 9 μm, 9.5 μm, 10 μm, 15 μm, 20 μm, 25 μm, 30 μm, 35 μm, 40 μm, 45 μm, 50 μm, 55 μm, 60 μm, 65 μm, 70 μm, 75 μm, 80 μm, 85 μm, 90 μm, 95 μm, 100 μm, 150 μm, 200 μm, 250 μm, 300 μm, 350 μm, 400 μm, 450 μm, 500 μm, 550 μm, 600 μm, 650 μm, 700 μm, 750 μm, 800 μm, 850 μm, 900 μm, 950 μm, 1000 μm; unless specifically stated, the diameter size refers to an average size.

A volume of the magnetic microsphere body may be any feasible particle size.

In a preferred embodiment, a diameter of the magnetic microsphere body is selected from 0.1-10 μm.

In a preferred embodiment, a diameter of the magnetic microsphere body is selected from 0.2-6 μm.

In a preferred embodiment, a diameter of the magnetic microsphere body is selected from 0.4-5 μm.

In a preferred embodiment, a diameter of the magnetic microsphere body is selected from 0.5-3 μm.

In a preferred embodiment, a diameter of the magnetic microsphere body is selected from 0.2-1 μm.

In a preferred embodiment, a diameter of the magnetic microsphere body is selected from 0.5-1 μm.

In a preferred embodiment, an average diameter of the magnetic microsphere body is around: 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, 1000 nm, an approximation may be ±25%, ±20%, ±15%, ±10%.

In a preferred embodiment, a diameter of the magnetic microsphere body is selected from 1 μm-1 mm.

In a preferred embodiment, a diameter of the magnetic microsphere body is 1 μm, 10 μm, 100 μm, 200 μm, 500 μm, 800 μm, 1000 μm, with a deviation range ±25%, ±20%, ±15%, ±10%.

Different magnetic materials can provide different types of activation sites, can produce a difference in a way of combining the purification element, and a ability to disperse and settle with a magnet is also different, and can further be selective for a type of a purified substrate.

The magnetic microsphere body and the magnetic microsphere comprising the magnetic microsphere body, on one hand, can be quickly positioned, guided and separated under an action of an applied magnetic field, on another hand, can be given a plurality of active functional groups on the surface of the magnetic microsphere by a surface modification or a chemical polymerization, including hydroxyl groups, carboxyl groups, aldehyde groups, amino groups, and more. In addition, the magnetic microsphere can also bind a plurality of antibodies, DNA and other bioactive substances through a covalent bond or a non-covalent bond.

In a preferred embodiment, the magnetic microsphere body is a magnetic material encapsulated by $SiO_2$. Wherein, a $SiO_2$ encapsulation layer may comprise a silane coupling agent with an active site taken.

In a preferred embodiment, the magnetic material is selected from: an iron compound (such as iron oxides), an iron alloy, a cobalt compound, a cobalt alloy, a nickel compound, a nickel alloy, a manganese oxide, a manganese alloy, a zinc oxide, a gadolinium oxide, a chromium oxide, and a combination thereof.

In a preferred embodiment, the iron oxide is a magnetite ($Fe_3O_4$), a magnetite ($\gamma$-$Fe_2$O3) or a combination thereof, preferably ferric oxide.

In a preferred embodiment, the magnetic material is selected from one of $Fe_3O_4$, $\gamma$-$Fe_2O_3$, Iron Nitride, $Mn_3O_4$, FeCrMo, FeAlC, AlNiCo, FeCrCo, ReCo, ReFe, PtCo, MnAlC, CuNiFe, AlMnAg, MnBi, FeNiMo, FeSi, FeAl, FeSiAl, MO•6$Fe_2O_3$, GdO or a combination thereof; wherein, the Re is a rare earth element Rhenium.

1.2 a polymer structure providing a large number of branched-chains.

An outer surface of the magnetic microsphere body has at least one polymer with a linear backbone and a branched-chain arranged, an end of the linear backbone is fixed onto the outer surface of the magnetic microsphere body, a plurality of other ends of the polymer is free from the outer surface of the magnetic microsphere body.

The "fixed onto" means fixing onto the outer surface of the magnetic microsphere body in a covalent connection method.

In a preferred embodiment, the polymer is covalently coupled to the outer surface of the magnetic microsphere body directly, or covalently coupled to the outer surface of the magnetic microsphere body indirectly through a connection component.

The polymer has a linear backbone, thus the polymer has not only a high flexibility of the linear backbone, but also an advantage of a high magnification of the number of branches, which can better achieve a high-speed and high-throughput binding, as well as a high efficient and high-ratio (high-yield) separations.

For the magnetic microspheres of the present application, one end of the polymer is covalently coupled to the outer surface of the magnetic microsphere body, and a plurality of remaining ends, including all branches and all functional groups are dissolved in the solution and distributed in an outer space of the magnetic microsphere body, the molecular chain can be fully stretched and wiggled, making the molecular chain be able to fully contact with a plurality of other molecules in the solution, further enhancing a capture to the target protein. When eluting the target protein from the magnetic microspheres, the target protein can be directly freed from a shackle of the magnetic microspheres and directly enter the eluent. Comparing with a polymer physically wound on the outer surface of the magnetic microsphere body or formed integrally with the magnetic microsphere body, this kind of polymer covalently fixed by one end of the linear backbone (in some preferred embodiments, a single linear backbone is covalently fix, in a plurality of other preferred embodiments, 2 or 3 linear backbones are covalently drawn from a fixed end of the backbone), is able to reduce effective a stacking of a plurality of molecular chains, enhancing a stretching and wiggling of the molecular chains in a solution, and enhancing a capture of the target proteins, reducing a retention ratio and a retention period of the target protein during an elution.

1.2.1 the backbone of the polymer on the biomagnetic microspheres provided by the present application.

In a preferred embodiment, the linear backbone is a polyolefin backbone or an acrylic polymer backbone.

In another preferred embodiment, the linear backbone of the polymer is a polyolefin backbone, including a polyolefin backbone (the linear backbone contains a plurality of carbon atoms only), and a linear backbone containing a plurality of hetero atoms (a hetero atom is a non-carbon atom).

In a preferred embodiment the backbone of the polymer is a polyolefin backbone. A monomer unit of the acrylic polymer is an acrylic monomer molecule inlcuding acrylic acid, acrylate, acrylic ester, methacrylic acid, methacrylate, methacrylate ester or a combination thereof. The acrylic polymer can be obtained by polymerizing one of the monomers stated above or by copolymerizing a suitable combination of the monomers stated above.

In a preferred embodiment, the linear backbone of the polymer is a polyolefin backbone. Specifically, for example, the polyolefin backbone is a backbone provided by a polymerization product composed of one monomer of acrylic acid, acrylate, acrylic ester, methacrylic acid, methacrylate, methacrylate ester, or a combination thereof (a backbone provided by a copolymerization product thereof), or a backbone provided by a copolymerization product having the monomers mentioned above perticipated. An example of the polymerization product combined by the monomers mentioned above is an acrylic acid-acrylic easter copolymer, or a methyl methacrylate-2-hydroxyethyl methacrylate copolymer (MMA-HEMA copolymer), and acrylic acid-hydroxypropyl acrylate copolymer. An example of the copolymerization product formed by the monomers mentioned above participated in the polymerization is maleic anhydride-acrylic acid copolymer.

In a preferred embodiment, the linear backbone is a polyolefin backbone and is provided by a backbone of an acrylic polymer.

In a preferred embodiment, the linear backbone is an acrylic polymer backbone.

In another preferred embodiment, the backbone of the polymer is an acrylic polymer backbone. A polyolefin backbone (only contains carbon atoms) may be used, or a hetero atom (hetero atom: a non-carbon atom) may also be contained in the backbone.

In another preferred embodiment, a backbone of the polymer is a backbone of a block copolymer containing a polyolefin block, for example, polyethylene glycol-b-polyacrylic acid copolymer (belonging to the scope of the acrylic copolymer). As long as it is able to exert a flexible swing of the linear backbone smoothly without causing an accumulation of the branched-chains or increasing a retention period or/and retention ratio.

In another preferred embodiment, the backbone of the polymer is a polycondensation-type backbone. The polycondensation-type backbone refers to a linear backbone that can be formed by a polycondensation reaction between a plurality of monomer molecules or oligomers; the polycondensation-type backbone may be a homopolymerization type or a copolymerization type. For example, a polypeptide chain, a polyamino acid chain, and more. Specifically, for example, an $\varepsilon$-polylysine chain, an $\alpha$-polylysine chain, a $\gamma$-polyglutamic acid chain, a polyaspartic acid chain, and more, an aspartic acid/glutamic acid copolymer, and more.

A number of the linear backbone being able to covalently couple to one binding site on the outer surface of the magnetic microsphere body can be one or more.

In a preferred embodiment, a binding site on the outer surface of the magnetic microsphere body has only one linear backbone drawn out, being able to provide a relatively large activity space for the linear backbone.

In another preferred embodiment, a binding site on the outer surface of the magnetic microsphere body has only two linear backbones drawn out, being able to provide an activity space for the linear backbone as large as possible.

The backbone of the polymer, wherein one end is covalently coupled to the outer surface of the magnetic bead (the outer surface of the biomagnetic microsphere), and a plurality of remaining ends including all branches and all functional groups, are dissolved in the solution and distributed in the outer space of the magnetic bead, the molecular chain can be fully stretched and wiggled, making the molecular chain be able to fully contact a plurality of other molecules in the solution, thereby enhancing a capture of the target protein. When eluting the target protein from the magnetic beads, the target protein can be directly freed from a shackle of the magnetic beads and directly enter the eluent. Comparing to a polymer physically wound on the outer surface of the magnetic beads or formed integrally with magnetic beads, the polymer provided herein is covalently fixed by one end of the linear backbone (most preferably, covalently fixing a single polymer linear backbone, and preferable, an fixed end of the backbone has 2 or 3 linear backbones drawn out covalently), which is able to reduce a stacking of a plurality of molecular chains effectively, enhance a stretching and swinging of the molecular chains in the solution, enhance capturing of a target protein, and reduce an amount of a retaintion ratio and a retaintion period of the target protein during an elution.

1.2.2. The polymer branched-chains of a biomagnetic microsphere provided by the present application

A number of the branched-chains is related to a size of the magnetic microsphere body, a type of a skeleton structure type of the polymer, a chain density (specifically, a density of the branched-chain) of the polymer on the outer surface of the magnetic microsphere body.

A number of the branched-chains of the polymer is a plurality, and at least 3. A number of a plurality of side branched-chains is related to a plurality of factors including a size of the magnetic microsphere, a length of the polymer backbone, a linear density of the side branched-chains along the polymer backbone, a chain density of the polymer on the outer surface of the magnetic microsphere, and more. The number of the branched-chains of the polymer can be controlled by controlling a feed ratio of a plurality of raw materials.

A branched-chain type polymer has at least 3 branched-chains.

An end of each of the branched-chains is or is not binding to a purification element independently.

When the end of the branched-chain is binding with a purification element, the end of each branched-chain binds to the purification element independently, or binds to the purification element indirectly through a connection component.

When the end of the branched-chain is binding with a purification element, a number of the purification element may be one or a plurality.

In a preferred embodiment, one molecule of the branched-chain polymer is binding with at least three purification elements.

1.3. a plurality of binding methods of the biotin or the biotin analogs

A method of the biotin or the biotin analogue connecting to the end of the branched-chain of the polymer is not particularly limited.

A method of the biotin or the biotin analog connecting to the end of the branched-chain of the polymer comprises, but not limited to, a covalent bond, a supramolecular interaction, or a combination thereof.

In a preferred embodiment, the covalent bond is a dynamic covalent bond; more preferably, the dynamic covalent bond comprises an imine bond, an acylhydrazone bond, a disulfide bond or a combination thereof.

In a preferred embodiment, the supramolecular interaction is selected from: a coordination binding, an affinity complex interaction, an electrostatic adsorption, a hydrogen bonding, a $\pi$-$\pi$ overlapping interaction, a hydrophobic interaction, and a combination thereof.

In a preferred embodiment, the branched-chain of the polymer covalently bonds to the biotin or the biotin analog through a functional group-based covalent bond, and bonds the biotin or the biotin analog covalently to the end of the branched-chain of the polymer. It can be obtained by a covalent reaction between a functional group contained in the branched-chains of polymer molecules on the outer surface of the biomagnetic microspheres with the biotin or the biotin analogs. Wherein, one of the preferred embodiments on the functional group is a specific binding site (a definition thereof is defined in the "Nouns and Terms" section of the detailed description of embodiments).

In a preferred embodiment, the branched-chain of the polymer covalently bonds to the biotin or the biotin analog through a functional group-based covalent bond, and bonds the biotin or the biotin analog covalently to the end of the branched-chain of the polymer. It can be obtained by a covalent reaction between a functional group contained in the branched-chains of polymer molecules on the outer surface of the biomagnetic microspheres with the biotin or the biotin analogs. Wherein, one of the preferred embodiments on the functional group is a specific binding site (a definition thereof is defined in the "Nouns and Terms" section of the detailed description of embodiments).

The functional group-based covalent bond refers to a covalent bond formed by a functional group participating in a covalent coupling. Preferably, the functional group is a carboxyl group, a hydroxyl group, an amino group, a sulfhydryl group, a salt form of a carboxyl group, a salt form of an amino group, a formate group, or a combination thereof. A preferred embodiment of the salt form of the carboxyl group is a sodium salt form such as COONa; a preferred embodiment of the salt form of the amino group may be an inorganic salt form, or an organic salt form, including but not limited to, a form of hydrochloride, hydrofluorid, and more. The "combination of functional groups" refers to all branched-chains of all polymer molecules on the outer surface of the magnetic microsphere, allowing different functional groups to participate in a formation of a covalent bond; taking the biotin as an example, that is, all biotin molecules on the outer surface of a magnetic microsphere with the biotin are able to covalently link with a plurality of different functional groups respectively, while one biotin molecule is able to link with one functional group only.

2. A second aspect of the present application provides a biomagnetic microsphere, on a basis of the biomagnetic microsphere provided by the first aspect of the present application, the biotin or the biotin analog, acting as a connection component, further connects with a purification element. That is, the end of the branched-chain of the polymer connects to the purification element through a connection component, and the connection component comprises the biotin or the biotin analog.

Purification Element

The purification element is a functional element being able to capture specifically a target from a mixed system, that is, the purification element and a target molecule to be separated and purified are able to perform a specifical binding, before the target molecule being captured can further be eluted under a suitable condition, so as to achieve a purpose of separation and purification.

When the purification element takes a protein substance as a target, a specific binding effect can be formed with respect to a target protein itself or a purification tag carried by the target protein. Therefore, a substance being able to be used as a purification tag of a target protein can be applied as an optional manner of the purification element; and a peptide or a protein applied as the purification element can also be used as an optional method for purifying the tag in the target protein.

2.1 A TYPE OF THE PURIFICATION TAG

The purification element may contain, but is not limited to, an antibody-type label, a polypeptide-type label, a protein-type label, an antibody-type label, an anti-prototype label, or a combination thereof

In a preferred embodiment, the purification element may comprise, but not limited to, an avidin-type tag, a polypeptide-type tag, a protein-type tag, an antibody-type tag, an antigen-type tag, or a combination thereof.

In a preferred embodiment, the avidin-type tag is avidin, a biotin-binding avidin analog, a biotin analog-binding avidin analog, or a combination thereof.

In a preferred embodiment, the purification element is: an avidin, an avidin analog being able to bind to a biotin or a biotin analog, a biotin, a biotin analog being able to bind to an avidin or an avidin analog, avidin protein, antibody, antigen, DNA, or a combination thereof.

In a preferred embodiment, an end of the branched-chain of the polymer on the biomagnetic microsphere connects with biotin; the purification element is avidin.

In a preferred embodiment, the avidin is any one of streptavidin, modified streptavidin, a streptavidin analog or a combination thereof.

The avidin analog, such as tamavidin 1, tamavidin 2 and more, wherein the Tamavidin 1 and the Tamavidin2 are a kind of proteins having an ablity of biotin-binding ability discovered by Yamamoto et al. in 2009 (Takakura Y et al. Tamavidins: Novel avidin-like biotin-binding proteins from the Tamogitake mushroom [J]. FEBS Journal, 2009, 276, 1383-1397), which have a strong affinity for the biotin similar to a streptavidin. A thermal stability of the Tamavidin2 is better than that of the streptavidin, and an amino acid sequence thereof can be retrieved from a plurality of relevant databases, including UniProt B9A0T7, or be transferred by a codon conversion, and be optimized by a program to obtain a DNA sequence can be optimized by a codon conversion and an optimize program.

In a preferred embodiment, a thermal stability of the Tamavidin2 is better than that of streptavidin, and an amino acid sequence thereof can be retrieved from a relevant database, such as UniProt B9A0T7, or a DNA sequence thereof can be optimized by an optimization program after a codon conversion.

The biotin analog, comprises a WSHPQFEK sequence or a variant sequence thereof, a WRHPQFGG sequence or a variant sequence thereof, and more.

In a preferred embodiment, the purification element is an affinity protein.

Examples of the affinity protein comprises but not limited to: Protein A, Protein G, Protein L, modified Protein A, modified Protein G, modified Protein L and more.

A plurality of definitions of the antibody and the antigen refer to the term section, and it should be understood that, the antibody and the antigen further comprise, but not limited to, a plurality of domains thereof, subunits, fragments, heavy chains, light chains, single-chain fragments (including Nanobodies, heavy chains lacking light chains, heavy chains variable regions, complementarity determining regions, and more.), epitopes, epitope peptides, a plurality of variants thereof, and more.

In a preferred embodiment, the polypeptide tag is selected from any one of following tags or a variant thereof: a CBP tag, a histidine tag, a C-Myc tag, a FLAG tag, a Spot tag, a C tag, an Avi tag, a tag comprising a sequence of WSHPQFEK, a tag comprising a variant sequence of WSHPQFEK, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof.

In a preferred embodiment, the protein tag is selected from any one of following tags or a variant protein thereof: an affinity protein, a SUMO tag, a GST tag, an MBP tag and a combination thereof; more preferably, the affinity protein is selected from Protein A, Protein G, Protein L, modified Protein A, modified Protein G, modified Protein L and a combination thereof.

In a preferred embodiment, the antibody-type tag is any one of an antibody, a fragment of an antibody, a single chain of an antibody, a fragment of a single chain, an antibody fusion protein, a fusion protein of an antibody fragment, a derivative thereof, or a variant thereof.

In a preferred embodiment, the antibody-type tag is an anti-protein antibody.

In a preferred embodiment, the antibody-type tag is an antibody against a fluorescent protein.

In a preferred embodiment, the antibody-type label is a nanobody.

In a preferred embodiment, the antibody-type tag is an anti-protein nanobody.

In a preferred embodiment, the antibody-type label is an anti-fluorescent protein nanobody.

In a preferred embodiment, the antibody-type label is a nanobody against green fluorescent protein or a mutant thereof.

In a preferred embodiment, the antibody-type tag is an Fc fragment.

2.2 A LOADING METHOD FOR THE PURIFICATION ELEMENT

A method for the purification element connecting to the biotin or the biotin analog has no particular limitations.

A connection method for the purification element connecting to the biotin or the biotin analog comprises, but not limited to, through a covalent bond, through a non-covalent bond (including a supramolecular interaction), through a connection component, or through a combination thereof.

In a preferred embodiment, the covalent bond is a dynamic covalent bond; more preferably, the dynamic covalent bond comprises an imine bond, an acylhydrazone bond, a disulfide bond or a combination thereof.

In a preferred embodiment, the supramolecular interaction is: a coordination binding, an affinity complex interaction, an electrostatic adsorption, a hydrogen bonding, a $\pi$-$\pi$ overlapping interaction, a hydrophobic interaction or a combination thereof.

In a preferred embodiment on the biomagnetic microsphere, the purification element connects to an end of the branched-chain of the polymer through a connection component containing an affinity complex.

In a preferred embodiment the biotin or the biotin analog has the avidin or the avidin analog connected through the affinity complex interaction, the purification element connects to the avidin or the avidin analog directly or indirectly.

In a preferred embodiment, the affinity complex interaction is selected from: a biotin-avidin interaction, a biotin analog-avidin interaction, a biotin-avidin analog interaction, a biotin analog-avidin analog interaction.

In a preferred embodiment, an affinity complex selection criteria is: having a good specificity and a strong affinity, further providing a site for a chemical bonding, making the affinity complex be able to connect covalently to the end of the branched-chain of the polymer, or be able to connect covalently to the outer surface of the magnetic microsphere body, after a chemical modification, including a binding site on the outer surface, an end of the backbone of the linear polymer, and an end of the branched-chain of the branched-chain type polymer. Such as a combination of a plurality of following substances: biotin or an analog thereof and avidin or an analog thereof, antigen and antibody, and more.

When the loading mode comprises the dynamic covalent bonds and the supramolecular interactions (especially the affinity complex interactions), a reversible loading mode is formed, and the purification element can be unloaded from the end of the branched-chain under a certain condition, before being renewed or replaced.

The renewal of the purification element corresponds to a regeneration of the magnetic microsphere, and a type of the purification element before and after the renewal are same.

The renewal of the purification element corresponds to a variation of the magnetic microsphere, and a type of the purification element before and after the renewal are different.

In a preferred embodiment, when the purification element is an avidin, further comprising a biotin combined with the avidin, wherein the biotin is a connection component; wherein a binding action of an affinity complex between the biotin and the avidin is formed.

In a preferred embodiment, when the purification element is an affinity protein, further comprising an avidin linked to the affinity protein, and biotin linked to the avidin; wherein, a binding action of an affinity complex is formed between the biotin and the avidin, the affinity complex is acting as a connection component.

In a preferred embodiment, the ends of the branched-chain of the polymer on the biomagnetic microspheres are connecting with biotin, avidin, and a purification element sequentially. More preferrably, the purification element is an antibody or an antigen. A connection method between the avidin and the purification element comprises but not limited to: a covalent bond, a non-covalent bond, a connection component or a combination thereof.

In a preferred embodiment, the purification element connects to the end of the branched-chain of the polymer on the biomagnetic microspheres through one or a combination of a plurality of following connection components, including but not limited to: nucleic acid, oligonucleotide, peptide nucleic acid, nucleic acid aptamer, deoxyribonucleic acid, ribonucleic acid, leucine zipper, helix-turn-helix motifs, zinc finger motifs, biotin, avidin, streptavidin, anti-hapten antibodies, and more. Of course, the connection component can also be a double-stranded nucleic acid construct, a duplex, a homohybrid or a heterohybrid (a homohybrid or a heterohybrid selected from DNA-DNA, DNA-RNA, DNA-PNA, RNA-RNA, RNA-PNA or PNA-PNA), or a combination thereof.

2.3. AN ACTION MECHANISM OF THE PURIFICATION ELEMENT

An action force of the purification element to capture a target molecule in a reaction and purification mixed system can be selected from: including but not limited to, a covalent bond, a supramolecular interaction, and a combinatios thereof.

In a preferred embodiment, the affinity complex interaction is selected from: a biotin-avidin interaction, a biotin analog-avidin interaction, a biotin-avidin analog interaction, a biotin analog-avidin analog interaction.

In a preferred embodiment, the target substance is bound to the end of the branched-chain of the polymer of the biomagnetic microsphere by a plurality of following forces: a biotin-avidin binding force, a Streg tag-avidin binding force, an avidin-affinity protein binding force, a histidine tag-metal ion affinity, an antibody-antigen binding force, or a combination thereof. The Streg tag mainly comprises, but not limited to, a peptide tag developed by IBA that can form a specific binding with the avidin or an analog thereof, usually containing a WSHPQFEK sequence or a variant sequence thereof.

2.4 REGENERATION AND REUSE OF THE PURIFICATION ELEMENT

When the purification element connects reversibly to the end of the branched-chain of the polymer on the biomagnetic microspheres of the present application through a plurality of reservable methods including an affinity complex, a dynamic covalent bond, and more, the purification element may be eluted from the end of the branched-chain of the polymer under an appropriate condition, before recombining with a new purification element.

The affinity complex interaction is taken as the affinity complex interaction between the biotin and the streptavidin as an example.

An extremely strong affinity between the biotin and the streptavidin is a typical binding effect of an affinity complex, which is stronger than a general non-covalent bond but weaker than a covalent bond, making a purification element be able to firmly bind to the end of the branched-chain of the plymer on the outer surface of the magnetic bead, and achieve a synchronous separation of the purification element by eluting the streptavidin from the specific binding site of the biotin when the purification element needs to be replaced, before releasing a plurality of activation sites that can re-associate a new avidin-purification element covalent ligation complex (e.g., a purification element with a streptavidin tag), further enabling a rapid recovery of a magnetic bead purification performance, and reducing a cost of separating and purifying a target substance (such as an antibody) dramatically. A process of eluting the biomagnetic microspheres modified with the purification element to remove the covalent ligation complex of the avidin-purification element, before regaining the biotin or the biotin analog-modified biomagnetic microspheres, is called a regeneration of the biotin magnetic microspheres. A regenerated biotin magnetic microsphere has a plurality of biotin active sites released, being able to rebind the avidin-purification element covalent ligation complex, and regaining the purification element-modified biomagnetic microspheres (corresponding to the regeneration of the biomagnetic microspheres), which is able to provide a plurality of fresh purification elements and provide a plurality of new target substance binding sites. This allows the biotin magnetic microspheres of the present application to be regenerated, that is, replacing before reusing the purification element.

2.4. PURIFICATION SUBSTRATE (PREFERABLY A PROTEIN TYPE SUBSTANCE)

The purification substrate of the present application refers to a substance that being able to be captured and separated by the magnetic microspheres of the present applicaiton, and there is no any particular limitation, as long as the purification substrate is able to specifically bind to the purification element of the magnetic microspheres of the present application.

When the purification substrate is a protein type substance, the purification substrate is also called a target protein.

2.4.1 A PURIFICATION TAG IN THE TARGET PROTEIN

The target protein may not carry a purification tag. In this case, the target protein itself should be able to be captured by the purification element on the magnetic microsphere. For example, the "target protein, purification element" is a combination of "an antibody, an antigen", "an antigen, an antibody", "an avidin or an analog thereof, a biotin or an analog thereof' and more.

In a preferred embodiment, the target protein carrying a purification tag, the purification tag is able to bind specifically to the purification element. In a target protein molecule, a number of the purification tags is one, two or more; when there are two or more purification tags contained, a type of the purification tags is one, two or more. It should be stated that, as long as an amino acid sequence of a tag differs, it is regarded as a different kind of tag.

The purification tag in the target protein can be selected from a group including but not limited to a plurality of following tags: a histidine tag, an avidin, an avidin analog, a Streg tag (a tag comprising the WSHPQFEK sequence or a variant thereof), a tag containing WRHPQFGG sequence or a variant thereof, a tag containing RKAAVSHW sequence or a variant thereof, a FLAG tag or a variant thereof, a C-tag and a variant thereof, a Spot tag and a variant thereof, a GST tag and a variant thereof, an MBP tag and a variant thereof, a SUMO tag, and a variant thereof, a CBP tag and a variant thereof, an HA tag and a variant thereof, an Avi tag and a variant thereof, an affinity protein, an antibody-like tag, an antigen-like tag, and a combination thereof. It can also be selected from a purification tag disclosed in US6103493B2, US10065996B2, US8735540B2, US20070275416A1, including but not limited to a streg tag and a variant thereof.

The purification tag may be fused with the N-terminus or the C-terminus.

The histidine tag generally contains at least 5 histidine residues, including 5×His tag, 6×His tag, 8×His tag and more.

A octapeptide of WRHPQFGG can specifically bind to core streptavidin.

The Streg tag is able to form a specific binding effect with the avidin or the analog thereof, and the Streg tag contains WSHPQFEK or a variant thereof. In an embodiment, WSHPQFEK-$(XaaYaaWaaZaa)_n$-WSHPQFEK, wherein each of the Xaa, the Yaa, the Waa, the Zaa is any one amino acid, independent to each other, XaaYaaWaaZaa comprises at least one amino acid and $(XaaYaaWaaZaa)_n$ comprises at least 4 amino acids, wherein n is selected from 1 ~15 (such as 1, 2, 3, 4, 5, 6, 7, 8, 9, 10, 11, 12, 13, 14, 15); a specific embodiment of $(XaaYaaWaaZaa)_n$ is $(G)_8$, $(G)_{12}$, GAGA, $(GAGA)_2$, $(GAGA)_3$, $(GGGS)_2$, $(GGGS)_3$. An example of the Streg tag is: WSHPQFEK, WSH-PQFEK-$(GGGS)_n$-WSHPQFEK, WSHPQFEK-GGGSGGGSGGSA-WSHPQFEK, SA-WSHPQFEK-$(GGGS)_2$GGSA-WSHPQFEK, WSHPQFEK-GSGGG-WSHPQFEK-GL-WSHPQFEK, GGSA-WNHPQFEK-GGGSGSGGSA-WSHPQFEK-GS, GGGS-WSHPQFEK-GGGSGGGSGGSA-WSHPQFEK, and more.

A sequence of the FLAG tag is DYKDDDDK. In an embodiment, a sequence of the variant of the FLAG tag is DYKDHD- G-DYKDHD-I-DYKDDDDK.

A sequence of the Spot tag is PDRVRAVSHWSS.

The C-tag comprises an EPEA sequence.

The GST tag refers to a glutathione S-transferase tag.

The MBP tag refers to a maltose binding protein tag.

The SUMO tag is a known Small ubiquitin-like modifier, and one of a plurality of important members of an ubiquitin-like polypeptide chain superfamily. In a primary structure, an SUMO shares only 18% homology with an ubiquitin, although a tertiary structure and a biological function of both is pretty similar.

A sequence of the CBP tag is KRRWKKNFIAVSAANRFKKISSSGAL.

A sequence of the HA tag is YPYDVPDYA.

The Avi tag is a known small tag consisting of 15 amino acid residues, the small tag can be recognized by a biotin ligase BirA specifically.

A antibody-like tag, comprises but not limited to, a complete structure of an antibody (an intact immunogolobin), a domain, a subunit, a fragment, a heavy chain, a light chain, a single chain fragment (e.g. a nanobody, a heavy chain without a light chain, a variable heavy chain region, a complementarity determining region, and more), and more.

A antigen-like tag, comprises but not limited to, a complete structure of an antigen (a complete antigen), a domain, a subunit, a fragment, a heavy chain, a light chain, a single-chain fragment (eg, an epitope, and more.), and more..

In a preferred embodiment, an N-terminus or a C-terminus of the target protein connects to a purification tag, or each of both terminus is connecting to a purification tag.

Various purification tags described in this section can be a candidate for the purification element in the magnetic microspheres of the present application.

## 2.4.2 TYPES OF THE TARGET PROTEIN

The target protein can be a natural protein or a modified product thereof, or an artificial synthetic sequence. A source of the natural protein is not particularly limited, including but not limited to: an eukaryotic cell source, a prokaryotic cell source, a pathogen source; wherein the eukaryotic cell source comprises but not limited to: a mammalian cell source, a plant cell source, a yeast cell source, an insect cell source, a nematode cell source, and a ombination thereof; the mammalian cell source may comprise but not limited to a murine source (including a rat source, a mice source, a guinea pig source, a golden hamster source, a hamster source, and more), a rabbit source, a monkey source, a human sources, a pig source, an ovine source, a bovine source, a dog source, a horse source, and more. The pathogen comprises a viruse, a chlamydia, a mycoplasma, and more. The viruse comprises HPV, HBV, TMV, coronavirus, rotavirus, and more.

A type of the target protein comprises but not limited to a polypeptide (the "target protein" in the present application broadly comprises a polypeptide), a fluorescent protein, an enzyme and a corresponding zymogen, an antibody, an antigen, an immunoglobulins, a hormone, a collagen, a polyamino acid, a vaccine, and more, a partial domain thereof, a subunit or a fragment thereof, and a variant thereof. The "subunit or fragment thereof' comprises a subunit or a fragment of the "partial domain thereof'. The "variant thereof' comprises a variant of the "partial domain thereof, subunit or fragment thereof'. The "variant thereof' comprises, but not limited to, a mutant thereof. In the present application, a situation of two or more consecutive "thereof' in other positions shall be interpreted similarly.

A structure of the target protein can be either a complete structure or selected from a plurality of corresponding partial domains, subunits, fragments, dimers, multimers, fusion proteins, glycoproteins, and more. A plurality of examples of an incomplete antibody structure comprises: nanobody (a heavy chain antibody loosing a light chain, or a $V_HH$, which retains a complete antigen-binding ability of the heavy chain antibody), a heavy chain variable region, a complementarity determining region (CDR), and more.

The target protein that can be synthesized by the *in vitro* protein synthesis system of the present application can be selected from, but not limited to, any of a plurality of following proteins, fusion proteins in any combinations, and compositions in any combinations: luciferase (such as firefly luciferase) ), green fluorescent protein (GFP), enhanced green fluorescent protein (eGFP), yellow fluorescent protein (YFP), aminoacyl-tRNA synthetase, glyceraldehyde-3-phosphate dehydrogenase, catalase (Catalase, such as mouse catalase), actin, antibodies, a variable region of an antibody (such as a single-chain variable region of an antibody, scFV), a single chain of an antibody and a fragment thereof (such as a heavy chain of an antibody, a nanobody, a light chain of an antibody), alpha-amylase, enterobactin A, hepatitis C virus E2 glycoprotein, insulin and a precursor thereof, a glucagon-like peptide (GLP-1), an interferon (including but not limited to interferon alpha, interferon alpha A, interferon beta, interferon gamma, and more), an interleukin (including an interleukin-1 beta, interleukin 2, interleukin 12, and more), a lysozyme, a serum albumin (including but not limited to a human serum albumin, a bovine serum albumin), a transthyretin, a tyrosinase, a xylanase, a β-galactosidase (β-galactosidase, LacZ, such as an *escherichia coli* β-galactosidase), and more, an aforementioned A partial domain of any proteins, a subunit or a fragment thereof, or a variant thereof (as defined above, the variant comprises a mutant, such as a luciferase mutant, a mutant of the eGFP, the mutant may also be a homolog). A plurality of examples of the aminoacyl-tRNA synthetase comprises a human lysine-tRNA synthetase, a human leucine-tRNA synthetase, and more. A plurality of examples of the glyceraldehyde-3-phosphate dehydrogenase comprises, an arabidopsis glyceraldehyde-3-phosphate dehydrogenase, a glyceraldehyde-3-phosphate dehydrogenase. References can further be made to a patent document of CN109423496A. The compositions in any combinations may comprise any of the proteins stated above, or may comprise a fusion protein thereof.

In a preferred embodiment, adopting a plurality of target proteins with a fluorescent property including one of GFP, eGFP, mScarlet, or a similar substance thereof, or a mutant thereof, to evaluate a protein synthesis ability of the *in vitro* protein synthesis system.

A plurality of application fields of the target protein comprises but not limited to biomedicine, molecular biology, medicine, *in vitro* detection, medical diagnosis, regenerative medicine, bioengineering, tissue engineering, stem cell engineering, genetic engineering, polymer engineering, surface engineering, nanoengineering, cosmetics, food, food additives, nutritional agents, agriculture, feed, daily necessities, washing, environment, chemical dyeing, fluorescent marking and more.

2.4.3 A MIXING SYSTEM COMPRISING A TARGET PROTEIN

The magnetic microspheres of the present application can be applied to separating a target protein from a mixed system thereof. The target protein is not limited to one substance, instead, a combination of a plurality of substances is allowed, as long as a purpose of the purification is obtaining the composition, or a form of the composition can meet a purification requirement.

A mixed system containing the target protein is not particularly limited, as long as the purification element of the magnetic microspheres of the present application can specifically bind to the target protein; generally, it is also required that the purification element and other substances other than the target protein in the mixed system have no specific binding actions or non-specific binding actions.

In the embodiments of the present application, the mixed system containing the target protein may be from a natural source, or may be an artificially constructed or obtained mixed system.

In an embodiment, it is possible to separate and purify a specific protein from a commercially available serum.

In an embodiment, it is possible to separate a target protein from a post-reaction system of an *in vitro* protein synthesis system.

One of a plurality of specific embodiments of the *in vitro* protein synthesis system further comprises, but not limited to, an E. coli-based cell-free protein synthesis system described in WO2016005982A1. A plurality of *in vitro* cell-free protein synthesis systems described in other citations of the present application, and a plurality of direct and indirect citations thereof, comprises but not limited to, an *in vitro* cell-free protein synthesis system based on wheat germ cells, rabbit reticulocytes, *Saccharomyces cerevisiae, Pichia pastoris,* and *Kluyveromyces marxianus,* are also incorporated into the present application as an embodiment of the *in vitro* protein synthesis system of the present application. In an embodiment, a plurality of *in vitro* cell-free protein synthesis systems described in a document "Lu, Y. Advances in Cell-Free Biosynthetic Technology. Current Developments in Biotechnology and Bioengineering, 2019, Chapter 2, 23-45", including, but not limited to, pages 27-28 of the "2.1 Systems and Advantages" section, can all be applied as the *in vitro* protein synthesis system for implementing the present application. In an embodimen (unless conflicting with the present application, the following documents and a plurality of citations thereof are cited in their entirety and for all purposes), including CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622, CN110938649A, CN110964736A, CN111378706A, CN111378707A, CN111378708A, CN111718419A, CN111748569A, CN2019107298813, CN2019112066163, CN2018112862093(CN111118065A), CN2019114181518, CN2020100693833, CN2020101796894, CN202010269333X, CN2020102693382, and a plurality of cited literatures thereof recording an *in vitro* cell-free protein synthesis system, a DNA template construction and amplification method, can all be applied as an embodiment of the *in vitro* protein synthesis system, the DNA template construction and amplification method, of the present application.

A source of the cell for the cell extract of the *in vitro* protein synthesis system is not particularly limited, as long as the target protein can be expressed *in vitro.* A plurality of exogenous proteins disclosed in the prior art that are suitable for a plurality of *in vitro* protein synthesis systems based on a prokaryotic cell extract, an eukaryotic cell extract (preferably a yeast cell extract, and more preferably *Kluyveromyces lactis*), or an endogenous protein suitable for an internal synthesization, including a prokaryotic cell system and an eukaryotic cell system (preferably a yeast cell system, more preferably a *Kluyveromyces lactis* system) can both be synthesized by the *in vitro* protein synthesis system of the present application, or by the *in vitro* protein synthesis system provided by the present application as a try.

One of a preferred method for the *in vitro* protein synthesis system is an IVTT system. A liquid after an IVTT reaction (referred to as an IVTT reaction solution), in addition to the target protein being expressed, further contains a plurality of residual raw materials for a reaction of the IVTT system, especially various factors from a cell extract (including a ribosome, a tRNA, a translation-related enzyme, a plurality of initiation factors, elongation factors, termination factors, and more.). The IVTT reaction solution, on one hand, is able to provide the target protein for binding to the magnetic beads, on another hand, can also provide a mixed system applied to testing a separation effect of the target protein.

3. A third aspect of the present application provides a biomagnetic microsphere, on a basis of the biomagnetic microsphere provided by the first aspect of the present application, further, the biotin or the biotin analog is applied as a connection component, further connects to the avidin or the avidin analog by a binding action of the affinity complex.

The biomagnetic microsphere in the third aspect is also called an avidin magnetic microsphere or an avidin magnetic bead.

In a preferred embodiment, the avidin or the avidin analog can be applied either as a purification element or as a connection component to further connect a plurality of other types of purification elements. Wherein, a binding action of the affinity complex is formed between the biotin or the biotin analog and the avidin or the avidin analog

In a preferred embodiment, on a basis of the biomagnetic microsphere provided in the first aspect of the present application, further comprising an avidin combined with the biotin. Wherein, the binding action of the biotin and the avidin forms an affinity complex. That is: the end of the branched-chain of the polymer on the biomagnetic microspheres connects with the biotin; the purification element is the avidin, and forms the binding action of the affinity complex with the biotin.

In a preferred embodiment, the avidin is any one of streptavidin, modified streptavidin, and streptavidin analogs or a combination thereof.

4. A fourth aspect of the present application provides a biomagnetic microsphere, on a basis of the biomagnetic microsphere provided by the third aspect of the present application, further comprising an affinity protein connecting to the avidin or the avidin analog. In this case, the biotin or the biotin analog, the avidin or the avidin analog can all be applied as a connection component, forming a binding action of the affinity complex in between; the affinity protein can be applied as a purification element or a connection component, preferably as a purification element.

The biomagnetic microsphere in the fourth aspect is also called an affinity protein magnetic microsphere or an affinity protein magnetic bead.

A typical structure of the biomagnetic microsphere is shown as FIG.2.

In a preferred embodiment, on a basis of the biomagnetic microspheres provided by the second aspect of the present application, the purification element is an affinity protein, the biomagnetic microsphere further comprises an avidin connected to the affinity protein, and a biotin connected to the avidin; wherein the purification element connects to an end of the branched-chain of the polymer through a connection element, the connection element comprises an affinity complex formed by the biotin and the avidin.

In a preferred embodiment, the affinity protein is one of Protein A, Protein G, Protein L, or a modified protein thereof. A corresponding biomagnetic microsphere may be called respectively as a Protein A magnetic microsphere or a Protein A magnetic bead, a Protein G magnetic microsphere or a Protein G magnetic bead, a Protein L magnetic microsphere or a Protein L magnetic bead, and more.

The biomagnetic microspheres provided by the fourth aspect of the present application, taking a connection method of biotin-avidin-avidin as an example, is able to make the avidin bind firmly to the end of the branched-chain of the polymer on the outer surface of the magnetic bead, can also elute the avidin (such as a streptavidin) from a specific binding site of the biotin to achieve a synchronous disengagement of the affinity protein, when an affinity protein needs to be replaced, further releasing an activation site being able to rebind a new avidin-affinity protein covalently linked complex E of (for example, an avidin has a streptavidin tagged), so as to realize a rapid recovery of a purification performance of the magnetic beads and greatly reduce a cost of antibody separation and purification. A process of eluting a biomagnetic microsphere modified with the avidin and removing the avidin-affinity protein covalently linked complex E, so as to regain a biotin-modified biomagnetic microsphere, is called a regeneration of the biotin magnetic microspheres. A regenerated biotin magnetic microsphere has a plurality of biotin active sites being released, being able to rebind the avidin-affinity protein covalently linked complex E, and obtain an avidin-modified biomagnetic microsphere F (corresponding to a regeneration of the biomagnetic microsphere F), being able to provide a fresh affinity protein and provide a newly generated antibody binding site. This enables the biotin magnetic microspheres of the present application to be able to regenerate and reuse, that is, the affinity protein can be replaced before reused.

5. A fifth aspect of the present application provides a preparation method for the biomagnetic microsphere provided by the first aspect of the present application;

5.1. A preparation and a principle of the biomagnetic microsphere provided by the first aspect of the present application.

The first aspect of the present application provides a biotin magnetic microsphere, having been modified with the biotin or the biotin analogs.

The biotin magnetic microsphere being modified with the biotin is taken as an example.

The biomagnetic microspheres provided in the first aspect can be prepared through a plurality of following steps: providing a $SiO_2$-encapsulated magnetic bead (commercially available or self-made), activating and modifying the $SiO_2$, connecting the polymer to the $SiO_2$ covalently (the polymer connects to the $SiO_2$ covalently through one end of the linear backbone, while a large number of side branched-chains are distributed along the backbone of the polymer), followed by connecting the biotin covalently to the ends of the branched-chains of the polymer. It should be noted that the steps stated above are not required to be totally isolated, instead, two or three steps are allowed to be combined into one step. In an embodiment, a plurality of activated silica-encapsulated magnetic beads (commercially available or homemade) may be directly provided. The step of activating and modifying the $SiO_2$, in an embodiment, is the step (1) of the preparation method of biomagnetic microspheres according to the fifth to eighth aspects provided by the present application. The step of connecting the polymer to the $SiO_2$ covalently, in an embodiment, is the step (2) and the step (3) of the preparation method of biomagnetic microspheres according to the fifth to eighth aspects provided by the present application. The step of connecting the biotin covalently to the ends of the branched-chains of the polymer, in an embodiment, is the step (4) of the preparation method of biomagnetic microspheres according to the fifth to eighth aspects provided by the present application.

The biomagnetic microspheres can be prepared by a plurality of following steps: (1) providing or preparing a magnetic microsphere body, an outer surface of the magnetic microsphere body has a reactive group $R_1$ arranged; (2) connecting a polymer with a linear backbone and a large number of branched-chains on a basis of the reactive group $R_1$, while one end of the linear backbone is covalently connected to the reactive group $R_1$; (3) connecting the biotin or the biotin analogs at an end of the branched-chain.

Taking a $SiO_2$-encapsulated magnetic material as the magnetic microsphere body as an example, a preparation process of the biomagnetic microspheres can be prepared by a plurality of following steps: (1) providing a magnetic microsphere encapsulated by $SiO_2$ (commercially available or self-made), activating and modifying the $SiO_2$ to generate a reactive group $R_1$; (2) performing a polymerization reaction on the reactive group $R_1$ (such as taking acrylic acid or sodium acrylate as a monomer molecule), before generating a polymer with a linear backbone and a large number of branched-chains, and the branched-chain has a plurality of functional groups $F_1$ at the end; (3) connecting the biotin or the biotin analog to the functional group $F_1$ at the end of the branched-chain. At this time, the polymer covalently connected to the magnetic microsphere body has a linear backbone, and one end of the linear backbone is covalently fixed to the reactive group $R_1$, and a large number of side branched-chains are distributed along the polymer backbone.

5.2 a plurality of preferred embodiments

A preferred embodiment on the preparation method for the biomagnetic microsphere (referencing to FIG.3), comprises following steps:

Step (1): providing a magnetic microsphere body, before performing a chemical modification to the magnetic microsphere body, by introducing an amino group to an outer surface of the magnetic microsphere body to form an amino-modified magnetic microsphere A.

In a preferred embodiment, performing the chemical modification to the magnetic microsphere by adoping a coupling agent;

In a preferred embodiment, the coupling agent is preferred to be an aminated silane coupling agent

In a preferred embodiment, the magnetic microsphere body is a magnetic material encapsulated by $SiO_2$, a silane coupling agent may be applied to performing the chemical modification to the magnetic microsphere body; the silane coupling agent is preferred to be an aminated silane coupling agent.

Step (2): covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B.

Step (3): by a polymerization of a carbon-carbon double bond, parforming a polymerization to a plurality of acrylic monomer molecules (such as a sodium acrylate), an acrylic polymer obtained is a branched-chain type polymer, having a linear backbone and a plurality of branched-chains containing the functional group $F_1$, the polymer is covalently coupled to the outer surface of the magnetic microsphere B through one end of the linear backbone, forming an acrylic polymer modified magnetic microsphere C. The present step may be carried out under a condition of no crossing agent added.

A definition of the acrylic monomer molecule and the functional group in the branched-chain of the polymer are shown in the "Nouns and Terms" section.

the functional group is a carboxyl group, a hydroxyl group, an amino group, a sulfhydryl group, a formate, an ammonium salt, a salt form of a carboxyl group, a salt form of an amino group, a formate group, or a combination of the functional groups thereof; the "combination of the functional groups " refers to the functional groups contained in all branched-chains of all the polymers on the outer surface of the magnetic microsphere, may have one or more types, which is consistent with a meaning of "combination of functional groups" as defined in the first aspect;

In another preferred embodiment, the functional group is a specific binding site.

Step (4): covalently coupling the biotin or the biotin analog to the end of the branched-chain of the polymer through the functional group $F_1$ contained in the branched-chain of the polymer, to obtain a biomagnetic microsphere combined with the biotin or the biotin analog (a biotin magnetic microsphere). The prepared biomagnetic microspheres, wherein providing a large number of biotin-binding sites by an acrylic polymer (having a polyacrylic acid backbone).

5.3 EMBODIMENTS

A specific embodiment on preparing the biotin magnetic microspheres is as follows:

Specifically, taking the acrylic polymer providing a linear backbone and a large number of the branched chains as an example, the present application provides a specific embodiment as following: taking a silicon dioxide-encapsulated ferric oxide magnetic bead as the biomagnetic microsphere body; Using a coupling agent of 3-aminopropyltriethoxysilane (APTES, CAS: 919-30-2, an aminated coupling agent, also a silane coupling agent, more specifically an aminated silane coupling agent) to perform a chemical modification to a silica-encapsulated ferrite magnetic bead, and introducing an amino group to the outer surface of the magnetic bead to complete activating and modifying the $SiO_2$ before obtaining an amino-modified magnetic microsphere A; followed by using a covalent reaction between the carboxyl groups and the amino groups, to couple a fixed molecule (an acrylic acid molecule, providing a carbon-carbon double bond and a reactive group of the carboxyl group) covalently to the outer surface of the magnetic bead, thereby introducing a carbon-carbon double bond to the outer surface of the magnetic bead, before obtaining a magnetic microsphere B containing the carbon-carbon double bond; further by using a polymization reaction of the carbon-carbon double bond, to carry out a polymerization of the acrylic monomer molecules (such as a sodium acrylate), and at a same time of the polymerization, a polymerization product is coupled covalently onto the outer surface of the magnetic bead, so as to achieve a connection of a polymer onto the $SiO_2$ (in a covalent connection method), and obtaining an acrylic polymer modified magnetic microsphere C; wherein the fixed molecule is an acrylic molecule, and one fixed molecule draws out only one polymer molecule, while also draws out only one linear polymer backbone; taking sodium acrylate as a monomer molecule in an embodiment, a polymerization product is sodium polyacrylate, and a backbone thereof is a linear polyolefin backbone, and there are a large number of side branched-chains COONa covalently connected along the backbone, while the functional group contained in the branched-chain is also COONa; the polymerization reaction herein does not use a plurality of cross-linking agents including N,N'-methylenebisacrylamide (CAS: 110-26-9) and more, so as to avoid cross-linking a molecular chain and forming a network polymer, instead of making the polymerization prouduct generate the linear backbone under a condition of no crossing linking agents added. If the molecular chains are cross-linked and forming a network polymer, a porous structure will be formed, affecting an elution efficiency of the target protein.

In a preferred embodiment, an amount of the acrylic acid used in the preparation of the magnetic microspheres B is 0.002-20 mol/L.

In a preferred embodiment, an amount of the sodium acrylate used in the preparation of the magnetic microspheres C is 0.53-12.76 mol/L.

The outer surface of the biomagnetic microspheres can further be modified by a plurality of other activation and modification methods. In an embodiment, the aminated biomagnetic microsphere (the amino-modified magnetic microsphere A) can further react with an acid anhydride or a plurality of other modified molecules, so as to realize a chemical modification of the outer surface of the biomagnetic microspheres by a carboxylation or other activation methods.

The fixed molecule is a small molecule that covalently fixes the backbone of the polymer to the outer surface of the magnetic bead. The fixed molecule is not particularly limited, as long as one end thereof is covalently coupled to the outer surface of the magnetic bead, and another end can initiate a polymerization reaction, including a homopolymerization, a copolymerization or a polycondensation, or another end thereof can copolymerize with the end of the backbone of the coupled polymer.

In a preferred embodiment, the fixed molecule allows drawing out only one single polymer linear backbone, also allows drawing out two or more polymer linear backbones, as long as it does not lead to a chain stacking and/or an increase in a retention ratio. Preferably, one fixed molecule draws out only one polymer molecule, and only one linear polymer backbone.

In another preferred embodiment, the acrylic monomer molecule, acting as a monomer unit for a polymerization, may also be one monomer molecule of acrylic acid, acrylate, acrylic ester, methacrylic acid, methacrylate, methacrylate ester, or a combination thereof.

As other embodiments of the present invention, the acrylic polymer may also be replaced by a plurality of other polymers. A selection criterion is: a polymer formed has a linear backbone, a large number of side branched-chains are distributed along the backbone, and the side branched-chains are carrying a plurality of functional groups for a subsequent chemical modification; that is, for a binding site on the outer surface of the magnetic beads, there are a large number of functional groups provided through the branched-chains distributed at a side end of the linear backbone of the polymer, comprising a plurality of structures including an ε-polylysine chain, an α-polylysine chain, a γ-polyglutamic acid, a polyaspartic acid chain, an aspartic acid/glutamic acid copolymer and more.

A method of introducing the polymer molecules of other alternatives of the polymers mentioned above to the outer surface of the biomagnetic microspheres: according to a chemical structure of a polymer alternative and a type of an active group on a side branched-chain thereof, selecting a suitable activation and modification method for the outer surface of the biomagnetic microsphere, a type of the fixed molecular, and a type of the monomer, before carrying out a suitable chemical reaction and introducing a plurality of active groups locating on the branched-chains to the outer surface of the biomagnetic microspheres.

After coupling covalently the acrylic polymer molecules (such as: a sodium polyacrylate linear molecular chain) to the outer surface of the magnetic bead, the functional group at the end of the branched-chain provides an activation site, or before connecting to a plurality of molecules including the biotin and the biotin analogs, according to a need of the reaction, it is possible to active the functional groups on the branched-chain of the polymer molecule, to make it reactive and form an activation site; coupling 1,3-propanediamine covalently to the activation site of the branched-chain of the polymer (each acrylic monomer unit structure will provide one activation site) to form a new functional group (an amino group), followed by using an amidation covalent reaction between the carboxyl group and the amino group to couple a biotin molecule or a biotin analog molecule covalently to the new functional group at the end of the branched-chain of the polymer, before completing a covalent attachment of the biotin or the biotin analogs to the end of the branched-chain of the polymer. Taking biotin as the purification element as an example, obtaining biotin-modified biomagnetic microspheres D; wherein one biotin molecule can provide a specific binding site. In an embodiment, taking the biotin as the purification element, obtaining a biotin-modified biomagnetic microsphere D; wherein one biotin molecule can provide a specific binding site; taking COONa as the functional group of the branched-chain of the polymer, wherein a sodium acrylate is applied as the monomer molecule, before a covalent reaction with the 1,3-propanediamine, the carboxyl group can be activated first, and an existing carboxyl group activation method can be adopted, in an embodiment, adding EDC•HCl and NHS.

5.3.1. Prepararing an acrylic polymer-modified magnetic microsphere

Preparing the magnetic microspheres A: for an aqueous solution of the silica-encapsulated ferroferric oxide magnetic microspheres, washing the magnetic microspheres with absolute ethanol, adding an ethanol solution of 3-aminopropyltriethoxysilane (APTES, a coupling agent), followed by reacting and cleaning, so as to introduce a large number of amino groups on the outer surface of the magnetic microspheres.

Preparing the magnetic microspheres B: adding 1-(3-dimethylaminopropyl)-3-ethylcarbodiimide hydrochloride (EDC•HCl) and N-hydroxysuccinimide (NHS) to an aqueous solution of the acrylic acid, for an activation of the carboxyl group, and after the activation, adding into an aqueous solution containing the magnetic microspheres A. A plurality of activated carboxyl groups on the acrylic acid forms a covalent bond (an amide bond) with the amino group on the outer surface of the magnetic microspheres, so as to introduce a large number of the carbon-carbon double bonds on the outer surface of the magnetic microspheres.

Preparing the magnetic microspheres C: adding an aqueous solution of acrylic monomer molecules into the magnetic microspheres B, adding an initiator, and carrying out a polymerization reaction of the carbon-carbon double bonds. The carbon-carbon double bonds in the acrylic monomer molecules and the carbon-carbon double bonds on the surfaces of the magnetic microspheres undergo a bond opening polymerization, and the acrylic polymer molecule is bonded covalently to the outer surface of the magnetic microsphere, wherein the acrylic polymer contains a functional group of the carboxyl group; and the functional group of the carboxyl group can exist in a form of carboxyl, formate, formate and more. In a preferred embodiment, the functional group of the carboxyl group is existing in a form of sodium formate, and adopting sodium acrylate or sodium methacrylate as a monomer molecule. In another preferred embodiment, the functional group of the carboxyl group is existing in a form of formate ester, and adopting the acrylate or methacrylate as the monomer molecule. Both the formate and the formate ester can obtain a better reactivity after being activated by the carboxyl group.

5.3.2 Preparing the biotin-modified biomagnetic microspheres D

A solution of the magnetic microspheres C: adding 1-(3-dimethylaminopropyl)-3-ethylcarbodiimide hydrochloride (EDC•HCl) and N-hydroxysuccinimide (NHS), to activate the carboxyl functional group of the side branched-chain of the polymer molecule on the outer surface of the microsphere by a carboxyl group, followed by adding an aqueous solution of propylene diamine to carry out a coupling reaction, and grafting a propylene diamine at a position of the carboxyl group on the side branched-chain of the acrylic polymer molecule, before transferring the functional groups on the side brained-chains of the polymer from a carboxyl group to an amino group.

Aqueous solution of biotin: adding 1-(3-dimethylaminopropyl)-3-ethylcarbodiimide hydrochloride and N-hydroxysuccinimide to activate the carboxyl group in the biotin molecule, before adding to an aqueous solution containing the magnetic microspheres C, the biotin is bound covalently to a position of a newly-born functional group (an amino group) at the side branched-chain of the polymer on the outer surface of the magnetic microsphere C, and obtaining the biomagnetic microsphere D having the biotin molecules connected to a large number of the side branched-chains of the acrylic polymer, respectively.

5.3.3 Embodiments

In a preferred embodiment, a method for preparing the biomagnetic microspheres D stated above is as follows: First, measuring 0.5-1000mL (20%, v/v) aqueous solution of the ferroferric oxide magnetic microspheres encapsulated by the silica, washing the magnetic microspheres with an absolute ethanol, adding 10-300mL ethanol solution with (3-Aminopropyl)triethoxysilane (APTES, CAS: 919-30-2) (5%-50%, v/v) to the magnetic microspheres having been cleaned as stated above, and reacting for 2-72 hours, followed by washing the magnetic microspheres with absolute ethanol and distilled water, to obtain the amino-modified magnetic microspheres A.

Pipette $1.0\times10^{-4}$~1 mol acrylic acid before adding to a solution X with a pH4~6 (solution X: an aqueous having 2-morpholineethanesulfonic acid (CAS: 4432-31-9) in a final concentration of 0.01-1 mol/L, and 0.1~2 mol/L NaCl), adding 0.001~0.5 mol 1-(3-dimethylaminopropyl)-3-ethylcarbodiimide hydrochloride (EDC·HCl, CAS: 25952-53-8) and 0.001~0.5 mol N-hydroxysuccinimide (NHS, CAS: 6066-82-6), react for 3~60 min. The solution stated above is then added to a PBS buffer solution with a pH 7.2-7.5 and having 0.5-50 mL of the magnetic microspheres A mixed, reacting for 1-48 hours, and the magnetic microspheres are then washed with distilled water to obtain the magnetic microspheres B modified with the carbon-carbon double bonds.

Take 0.5-50 mL magnetic microsphere B, add 0.5-20 0mL 5%-30% (w/v) sodium acrylate solution, then add 10 μL-20 mL 2%-20% (w/v) ammonium persulfate solution and 1 μL-1 mL tetramethylethylenediamine, react for 3-60 min, before washing the magnetic microspheres with distilled water and obtaining the magnetic microspheres C modified with the sodium polyacrylate.

Transfer 0.5~50 mL magnetic microspheres C to the solution X with a pH 4~6, add 0.001-0.5 mol 1-(3-dimethylaminopropyl)-3-ethylcarbodiimide hydrochloride (EDC•HCl) and 0.001~0.5 mol N-hydroxysuccinimide (NHS), react for 3~60 min. Followed by adding a PBS buffer solution having 0.0001-1 mol of 1,3-propanediamine dissolved and a pH 7.2-7.5, reacting for 1-48 hours. Wash with distilled water before adding a PBS buffer solution to convert the COONa at the side branched-chains of the polymer on the magnetic microsphere C into an amino functional group; weight $1.0\times10^{-6}$~$3.0\times10^{-4}$ mol biotin and adding into the solution X, add $2.0\times10^{-6}$~$1.5\times10^{-3}$ mol 1-(3-dimethylaminopropyl)-3-ethylcarbodiimide hydrochloride and $2.0\times10^{-6}$~$1.5\times10^{-3}$ mol N-hydroxysuccinate imide, before reacting for 3 to 60 min. Followed by being added into the magnetic microsphere solution having been washed as mentioned above, and reacting for 1 to 48 hours, before being washed with distilled water to obtain the biotin-modified magnetic microsphere D.

5.4. The biomagnetic microspheres provided in the third aspect of the present application can be obtained by reacting the biomagnetic microspheres provided in the first aspect directly with the avidin or the avidin analogs.

6. A sixth aspect of the present application provides a method for preparing the biomagnetic microspheres provided by the second aspect of the present application, comprising a plurality of following steps: (i) providing the biomagnetic microspheres prepared in the second aspect; which may be prepared by the steps (1) to (4) in the fifth aspect.; (ii) connecting the purification element to the biotin or the biotin analog at the end of the branched-chains of the polymer on the biomagnetic microspheres.

In a preferred embodiment, the biomagnetic microspheres are prepared through a plurality of following steps: steps (1) to (4) are as same as the fifth aspect; step (5): connecting the purification element with the biotin at the end of the branched-chains of the polymer on the biomagnetic microspheres.

7. A seventh aspect of the present application provides a method for preparing the biomagnetic microspheres provided by the second aspect of the present application, comprising a plurality of following steps: (i) provide the biomagnetic microspheres prepared in the second aspect; which may be prepared by the steps (1) to (4) in the fifth aspect.; (ii) take a covalent ligation complex of the avidin or the avidin analog and the purification element (for example, an avidin-purification element covalent ligation complex) as a raw material to provide the purification element, before being bound to the end of the branched-chains of the polymer, the biotin or the biotin analogs and the avidin or the avidin analogs are forming a binding action of an affinity complex, so as to obtain the biomagnetic microspheres with the purification element.

Independently and optionally, further comprising a step (6): sedimenting the biomagnetic microspheres by a magnet, removing a liquid phase, and cleaning;

Independently and optionally, further comprising a replacement of the purification element, which may be achieved by eluting the covalent ligation complex of the avidin or the avidin analogs and the purification element under an appropriate condition.

In a preferred embodiment, the biomagnetic microspheres are prepared through following five steps: steps (1) to (4) are as same as the fifth aspect; step (5) is as same as the step (ii) stated above.

8. An eighth aspect of the present application provides a method for preparing the biomagnetic microspheres provided by the fourth aspect of the present invention (referencing to FIG.3).

The fourth aspect of the present application provides an affinity protein magnetic microsphere.

The biomagnetic microspheres provided in the fourth aspect of the present application can be obtained by using the biomagnetic microspheres provided in the first aspect as a raw material, before combining with a covalent ligation complex of the avidin or the avidin analogs and the affinity protein, so as to load the affinity protein onto the branched-chains of the polymer on the biomagnetic microspheres through an action of the affinity complex between the biotin or the biotin analogs and the avidin or the avidin analogs.

In a preferred embodiment, the biomagnetic microspheres provided in the fourth aspect of the present application can be obtained by using the biomagnetic microspheres provided in the first aspect as a raw material, then combining with a covalent ligation complex E of the avidin-affinity protein.

The covalent ligation complex E of the avidin-affinity protein: also called an avidin-affinity protein complex E, which is a complex formed by a covalent linking, having the avidin or the avidin analogs at one end, and the affinity protein at another end, both are directly connected by a covalent bond, or indirectly connected by a covalent linking element. A covalent linking group comprises a covalent bond, a linking peptide, and more. An example of the avidin-affinity protein complex E is an affinity protein with streptavidin, wherein the affinity protein is selected from, but not limited to, Protein A, Protein G and/or Protein L, and more. Examples of the avidin-affinity protein complex E further comprise: a streptavidin-Protein A complex, a streptavidin-Protein A fusion protein, a fusion protein of Protein A-eGFP-streptavidin, Protein A-eGFP-tamavidin2, Protein A-eGFP-tamavidinl, and more; wherein, the eGFP comprises broadly a plurality of mutants thereof, both the tamavidin1 and the tamavidin2 are avidin analogs.

The avidin binds to biotin specifically to form an affinity complex. The binding action of the affinity complex of avidin and biotin mentioned above can also be replaced by a binding action of a plurality of other affinity complexes, and an effect of reusing after replacing the affinity protein can also be achieved. However the action of the affinity complex provided by the avidin and the biotin is more preferred, due to a good specificity and a strong affinity between the two, also the biotin, besides a binding domain with the aviton, further has an additional carboxyl group available for bonding, and the avidin can easily bind with the affinity protein to form a fusion protein.

An selection criterion for the affinity complex: having a good specificity and a strong affinity, and further providing a site for chemical bonding, which enables a covalent connection to the end of the branched-chain of the polymer, or a covalent connection to the end of the branched-chain of the polymer after a chemical modification.

8.1 preparation process

In a preferred embodiment, on a basis of the biomagnetic microspheres (a biotin magnetic microsphere) prepared in the fifth aspect, a following step (5) is carried out to obtain a magnetic microsphere system using the affinity protein as a purification element.

Step (5): Binding the covalent ligation complex of the avidin or the avidin analogs and the affinity protein (eg, a covalent ligation complex E of avidin-affinity protein). By a specific binding action between the biotin or the biotin analogs and the avidin or the avidin analogs, binding the covalent ligation complex (such as the covalent ligation complex E of avidin-affinity protein) to the end of the branched-chain of the polymer, before forming a binding action of an affinity complex between the biotin or the biotin analogs and the avidin or the avidin analogs, to obtain an affinity protein magnetic microsphere.

In an embodiment, adding the covalent ligation complex E of avidin-affinity protein (eg, an affinity protein with the streptavidin, wherein the affinity protein is selected from a group comprising, but not limited to, Protein A, Protein G and/or Protein L, and more.), to a system of the biotin-modified biomagnetic microspheres D, by an extremely strong specific affinity between the biotin and the avidin (such as the streptavidin), the affinity protein is non-covalently linked to the end of the branched-chains of the polymer on the outer surface of the magnetic bead, to obtain an affinity protein-modified magnetic bead being able to be applied to separating and purifying an antibody substance, taking the affinity protein as the purification element, and providing the binding sites applied to capturing a target protein.

8.2 embodiments on the preparation process

In an embodiment, the biomagnetic microspheres provided by the fourth aspect of the present application, connecting to the end of the branched-chain of the polymer by a connection method of biotin-avidin-affinity protein, can be prepared by a plurality of following steps:

(1) performing a chemically modification to the magnetic microsphere body, introducing an amino group to the outer surface of the magnetic microsphere body to form an amino-modified magnetic microsphere A; when the magnetic microsphere body is a magnetic material encapsulated by $SiO_2$, preferably a coupling agent is an aminated silane coupling agent;

In a preferred embodiment, performing the chemical modification to the magnetic microsphere by adopting a coupling agent;

when the magnetic microsphere body is a magnetic material encapsulated by $SiO_2$, a silane coupling agent may be applied to performing the chemical modification to the magnetic microsphere body. The silane coupling agent is preferred to be an aminated silane coupling agent;

(2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B;

(3) under a condition of not adding a cross-linking agent, by a polymerization of a carbon-carbon double bond, parforming a polymerization to a plurality of acrylic monomer molecules (such as a sodium acrylate), and an acrylic polymer obtained has a linear backbone and a branched-chain containing a functional group. The polymer covalently couples to the outer surface of the magnetic microsphere B through one end of the linear backbone to form an acrylic polymer modified magnetic microsphere C;

preferably, the functional group is a specific binding site;

a plurality of other preferred embodiments on the functional group are as same as that in the first aspect;

(4) covalently coupling the biotin through the functional group contained in the branched-chain of the polymer, to obtain a biotin-modified biomagnetic microsphere D.

(5) by a specific binding between the biotin and the avidin, binding an avidin-affinity protein covalent ligation complex E to the end of the branched-chain of the polymer, by a binding action of the affinity complex between the biotin and the avidin, an affinity protein modified biomagnetic microsphere is obtained (an affinity protein modified magnetic microsphere);

independently and optionally, comprising (6) sedimenting the affinity protein modified magnetic microsphere by a magnet, removing a liquid phase and washing;

independently and optionally, comprising a step (7), replacing the avidin-affinity protein covalent ligation complex E, which may be achieved by eluting the avidin-affinity protein covalent ligation complex E.

8.3. biomagnetic microsphere: preparing a biomagnetic microsphere F having the avidin-Protein A bound.

(a biomagnetic microsphere F having an affinity protein bound, a Protein A modified biomagnetic microsphere F, a Protein A modified magnetic microsphere)

Add the biotin-modified biomagnetic microspheres D to a fusion protein solution of an avidin-Protein A ligation complex E (eg, ProteinA-eGFP-Streptavidin, ProteinA-eGFP-Tamvavidin2), mix and incubate. Through a specific binding of the avidin (such as Streptavidin or Tamvavidin2) to the biotin, the proteinA is fixed to an end group of the branched-chains of the polymer on the outer surface of the biomagnetic microsphere D, and a biomagnetism microsphere F having the avidin-Protein A bound is obtained. In a structure of the biomagnetic microsphere F obtained, the side branched-chain of the acrylic polymer contains an affinity complex structure of biotin-avidin-Protein A, which is covalently linked to a branched point in the linear backbone of the polymer through an end having the biotin. A strong non-covalent specific binding action of an affinity complex between the biotin and the avidin is formed, a covalent connection exists between the avidin and the Protein A, and a fluorescent tag can be inserted between the avidin and the Protein A, as well as a plurality of other linker peptides can also be inserted.

Wherein, the avidin-Protein A fusion proteins, including a ProteinA-eGFP-Streptavidin fusion protein and a ProteinA-eGFP-Tamvavidin2 fusion protein, can be obtained by an IVTT reaction for a cell-free protein synthesis *in vitro.* At this time, the biomagnetic microspheres D is mixed with a supernatant obtained after a reaction of the IVTT, and by a specific binding action between the biotin on the outer surface of the biomagnetic microspheres D and the avidin fusion protein in the solution, a binding of the avidin-Protein A. is achieved.

8.4. A binding amount of the affinity protein on the outer surface of the biomagnetic microspheres can be determined by a following method (taking a fluorescent protein eGFP labeling as an example):

First, after a binding reaction between the affinity protein solution and the magnetic beads is completed, the biomagnetic microspheres having the affinity protein bound are adsorbed and sedimented by a magnet. Then, a liquid phase was separated and collected, recorded as a flow-through liquid. At this time, a concentration of the affinity protein in the liquid phase decreases. By measuring a change in a fluorescence value of the fluorescent protein eGFP in the supernatant obtained from an IVTT reaction before and after binding the biomagnetic microspheres, a fluorescence intensity of the fluorescent protein eGFP bound on the biomagnetic microspheres was calculated, and a concentration of the affinity protein was obtained by a conversion. When an affinity protein concentration in the flow-through liquid has basically no more changes compared with an affinity protein concentration in an IVTT solution before incubating with the biomagnetic microspheres, it means that an adsorption of the affinity protein by the biomagnetic microspheres tends to be saturate, and accordingly, a fluorescence value of the fluorescent protein eGFP is no longer changed significantly. It is possible to establish a standard curve between the fluorescence value and the concentration of the eGFP protein, by adopting a pure product of the eGFP protein, so as to quantitatively calculate an amount and a concentration of the avidin-affinity protein (such as a streptavidin-Protein A) bound to the biomagnetic microspheres.

Adopting the Protein A-modified biomagnetic microspheres for separating and purifying the antibodies, it is possible to calculate a binding amount of the antibody by a following method (taking a bovine serum antibody as an example): encubating the Protein A modified biomagnetic microspheres and a solution of bovine serum antibody (such as obtained by adopting an *in vitro* protein synthetic system to express the antibody or commercially available), after the reaction, the bovine serum antibody is eluted from the magnetic beads by using an elution buffer, and the bovine serum antibody separated exists in an eluate. A concentration of the bovine serum albumin in the eluate was determined by a Bradford method. At a same time, adopting BSA as a standard protein, and performing a test by a microplate reader, taking a standard protein as a reference, a protein concentration of a purified antibody could be calculated, further a yield amount and a yield of a separation and a purification could be calculated.

8.5. regenerating the biomagnetic microspheres: replacing the purification element (taking the protein A as the affinity protein for an example)

Elute and replace the Protein A: at a same time of eluting the avidin, a simultaneous detachment of the Protein A is achieved, so it is also a replacement of the avidin-Protein A.

In an embodiment, adding a denaturing buffer (containing urea and sodium dodecyl sulfate) to the Protein A-modified biomagnetic microspheres F, incubating in a metal bath at 95°C, and eluting the avidin-Protein A fusion protein (such as the SPA-eGFP-Tamvavidin2) binding with the biotin on the biomagnetic microspheres D, obtaining a regenerated biomagnetic microsphere D (with a plurality of binding sites of the biotin on the ends of the branched-chain of the polymer), followed by adding a fresh fusion protein solution (such as a supernatant of SPA-eGFP-Tamvavidin2 after an IVTT reaction) containing the avidin-Protein A into the biomagnetic microspheres D having been regenerated, to make the binding sites of the biotin of the biomagnetic microspheres D released rebind a new avidin-Protein A (such as the SPA-eGFP-Tamvavidin2), regenerating a non-covalent specific binding action between the biotin and the avidin (eg, Tamvavidin2), thereby realizing the replacement of the Protein A, and obtaining the biomagnetic microspheres F having been regenerated.

9. position control of the magnetic microspheres

After preparing the biomagnetic microspheres (including but not limited to the biomagnetic microspheres D and the biomagnetic microspheres F) described in the first to fourth aspects of the present application, it is possible to sediment the magnetic microspheres easily by a magnet, remove a liquid phase, before washing and removing a plurality of impurity proteins and/or other impurities absorbed.

By controlling a size of the magnetic microspheres and a plurality of chemical and structural parameters of the polymer, the magnetic microspheres can be suspended stably in the liquid phase, without a settle down for two days or more. Moreover, the magnetic microspheres are possible to be stably suspended in a liquid system without any continuous stirring. On one hand, the magnetic microspheres can be controlled to a nanoscale size of several micrometers or even less than 1 micrometer; on another hand, a graft density of the polymer on the outer surface of the magnetic microspheres can be adjusted, and a plurality of characters including a hydrophilicity thereof, a structure type, a hydrodynamic radius, a chain length, a number of the branches, a length of the branches, and more, are also adjustable, so as to better control a suspension performance of a magnetic microsphere system in a system, and realize a sufficient contact of the magnetic microsphere system and a mixed system of the *in vitro* protein synthesis reaction. A preferred size of the magnetic microspheres is about 1 micron.

10. A ninth aspect of the present application provides use of the biomagnetic microsphere described in the first to the fourth aspects of the present application in separating and purifying a protein substance;
preferably, use of the biomagnetic microsphere in the separating and purifying an antibody substance;

Referencing to the "Nouns and Terms" section for a definition of an antibody substance.

The present application particularly provides an application of the biomagnetic microspheres in separating and purifying the antibodies, an antibody fragment, an antibody fusion protein, and an antibody fragment fusion protein.

When the purification element connects to the end of the branched-chain of the polymer through a connection component containing an affinity complex, the application may further optionally comprise a regeneration of the biomagnetic microspheres, that is, the replacement before reuse of the purification element.

11. A tenth aspect of the present application provides a use on the biomagnetic microsphere described in the first to the fourth aspects of the present application in separating and purifying a protein substance, especially a use in separating and purifying an antibody, an antibody fragment, an antibody fusion protein, and an antibody fragment fusion protein;

the purification element is an affinity protein;

preferably, the affinity protein connects to the branched-chain of the polymer in a way of biotin-avidin-affinity protein;

when the affinity protein connects to the end of the branched-chain of the polymer through the connection component containing the affinity complex (such as the biomagnetic microsphere described in the fourth aspect), the use may further comprise optionally a regeneration and reuse of the biomagnetic microspheres, that is, comprising a replacement and reuse of the affinity protein.

12. An eleventh aspect of the present application provides a biomagnetic microsphere; an outer surface of the magnetic microsphere body has at least one polymer with a linear backbone and a branched-chain arranged, an end of the linear backbone is fixed onto the outer surface of the magnetic microsphere body, a plurality of other ends of the polymer is free from the outer surface of the magnetic microsphere body, and an end of the branched-chain of the polymer on the biomagnetic microsphere has a purification element connected; the purification element is selected from an avidin-type tag, a polypeptide-type tag, a protein-type tag, an antibody-type tag, an antigen-type tag, or a combination thereof;

preferably, the avidin-type tag is avidin, a biotin-binding avidin analog, a biotin analog-binding avidin analog, or a combination thereof;

preferably, the avidin is a streptavidin, a modified streptavidin, a streptavidin analog, or a combination thereof;

preferably, the polypeptide-type tag is selected from any one of following tags or a variant thereof: a CBP tag, a histidine tag, a C-Myc tag, a FLAG tag, a Spot tag, a C tag, an Avi tag, a Streg tag, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof. The Streg tag comprises WSHPQFEK and a variant thereof;

preferably, the protein tag is selected from any one of following tags or a variant protein thereof: an affinity protein, a SUMO tag, a GST tag, an MBP tag and a combination thereof;

preferably, an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has an affinity protein connected;

preferably, further, a skeleton of the branched-chain between the affinity protein and the linear backbone of the polymer has a binding action of the affinity complex existing;

more preferably, the affinity protein is selected from Protein A, Protein G, Protein L, modified Protein A, modified Protein G, modified Protein L and a combination thereof.

The *in vitro* protein synthesis system (IVTT system) used in the *in vitro* cell-free protein synthesis method in the embodiments 2-6 described below comprises following components (final concentration): 9.78 mM Tris-Hydroxymethylaminomethane hydrochloride (Tris-HCl) with a pH 8.0, 80 mM potassium acetate, 5 mM magnesium acetate, 1.8 mM mixture of nucleoside triphosphates (adenosine triphosphate, guanosine triphosphate, cytosine triphosphate and uridine triphosphate, a concentration of each nucleoside triphosphate is 1.8 mM), 0.7 mM amino acid mixture (glycine, alanine, valine, leucine, isoleucine, phenylalanine, proline, tryptophan, serine , tyrosine, cysteine, methionine, asparagine, glutamine, threonine, aspartic acid, glutamic acid, lysine, arginine, and histidine, a concentrationat of each amino acid is 0.1 mM respectively), 15 mM glucose, 320 mM maltodextrin (molar concentration in glucose units, corresponding to about 52 mg/mL), 24 mM tripotassium phosphate, 2% (w/v) polyethylene glycol 8000, and finally adding 50% Volume of a cell extract (specifically a yeast cell extract, more specifically a *Kluyveromyces lactis* cell extract).

Wherein, the *Kluyveromyces lactis* extract comprises an endogenously expressed T7 RNA polymerase. The *Kluyveromyces lactis* extract is transformed in a following manner: adopting a modified strain based on *Kluyveromyces lactis* strain ATCC8585; using the method described in CN109423496A, integrating the encoding gene of T7 RNA polymerase into a genome of the *Kluyveromyces lactis* strain, to obtain a modified strain, which is able to endogenously express the T7 RNA polymerase; culturing the cell raw material with the modified strain, before preparing the cell extract. A preparation process of the *Kluyveromyces lactis* cell extract adopts a conventional technical means, referencing to the method described in CN109593656A. In general, a plurality of preparation steps comprises: providing an appropriate amount of raw materials from a fermented *Kluyveromyces lactis* cell, quick-freezing the cells with liquid nitrogen, breaking the cells, and collecting the supernatant by centrifugation to obtain the cell extract. A protein concentration in the *Kluyveromyces lactis* cell extract being obtained was 20-40 mg/mL.

IVTT reaction: adding 15 ng/$\mu$L DNA template (the encoded protein contains a fluorescent label) to the *in vitro* protein synthesis system stated above, carrying out an *in vitro* protein synthesis reaction, mixing well before placing in a 25-30°C environment for a reaction, a reaction time is 6~18h, to synthesize a protein encoded by the DNA template, and obtain an IVTT reaction solution containing the protein. Measuring the RFU value by adopting a UV absorption method, and calculating a content of the protein by combining a concentration thereof and the standard curve of the the RFU

Embodiment 1: preparing the biomagnetic microspheres D (binding to biotin)

Preparing silica encapsulated magnetic microspheres (also known as magnetic microsphere body, magnetic beads, glass beads)

Place 20 g $Fe_3O_4$ microspheres into a mixed solvent of 310 mL ethanol and 125 mL water, adding 45 mL 28% (wt) ammonia water, add 22.5 mL ethyl orthosilicate drop by drop, stir and react at a room temperature for 24 h, wash with ethanol and water for cleaning after a reaction. A plurality of ferroferric oxide microspheres with different particle sizes (around 1 $\mu$m, 10 $\mu$m, and 100 $\mu$m) were used as a raw material to control a particle size of a glass bead obtained. The ferroferric oxide microspheres with different particle sizes can be prepared by a conventional technical means.

The magnetic microsphere prepared is applied as a basic raw material to be modified by a purification element or a connection component-purification element, thus also being called a magnetic microsphere body.

The magnetic microsphere prepared has a magnetic core, being able to be controled to a position through an action of a magnetic force, before realizing a plurality of operations including moving, dispersing, and sedimenting, thus it is a generalized magnetic bead.

The magnetic microsphere prepared has a silica coating layer, thus also being called a glass bead, which can reduce an adsorption of the magnetic core to a plurality of following ingradients or components: polymers, purification elements, all components of the *in vitro* protein synthesis system, nucleic acid templates, protein expression products, and more.

After a plurality of experiments, it has been shown that when the particle size of the magnetic microspheres is about 1 μm, a plurality of properties including an ease of suspension, a persistence of suspension, and a binding efficiency to the proteins are best. Using an IVTT reaction solution to provide a mixed system for a target protein, for the binding efficiency of the target protein, when the particle size of the magnetic microspheres is about 1 μm, comparing with a particle size of 10 μm, it has increased by more than 50%, and comparing with a particle size of 100 μm, it has increased by over 80%.

Using the silica-coated magnetic microspheres to prepare the biotin magnetic beads is achieved by a plurality of following steps:

First, measure 50 mL of an aqueous solution of silica encapsulated ferroferric oxide magnetic microspheres (a particle size of the magnetic microsphere is about 1 μm) having a solid content of 20% (v/v), sediment the magnetic microspheres with a magnet and remove a liquid phase, wash the magnetic microspheres with 60 mL of anhydrous ethanol each time, for a total 5 times. Add 100 mL ethanol solution (25%, v/v) in excessive with 3-aminopropyltriethoxysilane (APTES, CAS: 919-30-2) to the magnetic microspheres after washing as stated above, stirr mechanically in a water bath at 50°C for 48 hours, followed by stirring mechanically in a water bath at 70°C for 2 hours; sedimenting the magnetic microspheres with a magnet and removing a liquid phase, washing the magnetic microspheres with 60 mL of anhydrous ethanol each time, for a total 2 times, followed by washing the magnetic microspheres with 60 mL of distilled water each time, repeating the washing for 3 times, and obtaining the magnetic microspheres A.

Next, pipette 0.01 mol of acrylic acid into 100 mL of solution × (solution X: an aqueous having 2-morpholineethanesulfonic acid (CAS: 4432-31-9) in a final concentration of 0.1 mol/L, and 0.5 mol/L NaCl), before adding 0.04 mol 1-(3-dimethylaminopropyl)-3-ethylcarbodiimide hydrochloride (CAS: 25952-53-8) and 0.04 mol N-hydroxysuccinimide (CAS: 6066-82 -6), stir and mix well at a room temperature, stir and react for 15 min, adjust a pH value of the solution to 7.2 by using $NaHCO_3$ solid powder, add the solution stated above having the pH value adjusted well to 100 mL of PBS buffer solution having 10 mL of the magnetic microspheres A, carry out a mechanical stirring in a 30°C water bath for 20 hours, followed by sedmenting the magnetic microspheres by a magnet, removing the liquid phase, and washing the magnetic microspheres with 60 mL of distilled water each time, repeating the washing for 6 times, before obtaining the magnetic microspheres B.

Third, take 1 mL of the magnetic microspheres B, add 12 mL of 15% (w/v) sodium acrylate solution, add 450 μL of 10% ammonium persulfate solution and 45 μL of tetramethylethylenediamine, reacting at the room temperature for 30 min, followed by sedmenting the magnetic microspheres by a magnet, removing the liquid phase, and washing the magnetic microspheres with 10 mL of the distilled water each time, repeating the washing for 6 times, before obtaining the magnetic microspheres C (crylic polymer-modified magnetic microspheres C).

Fourth, transfer the magnetic microspheres C having been synthesized to 10 mL of the solution X, adding 0.004 mol 1-(3-dimethylaminopropyl)-3-ethylcarbodiimide hydrochloride and 0.004 mol N- Hydroxysuccinimide, stir and mix well at the room temperature, stir and react for 15 min, sediment the magnetic microspheres with a magnet and remove a liquid phase, wash the magnetic microspheres with 10 mL of distilled water each time, for a total 3 times; pipette $4.0\times10^{-4}$ mol 1,3-propanediamine and dissolve in 10 mL of PBS buffer solution, before adding into the magnetic microspheres having been washed, stirring mechanically for 20 hours in a 30°C water bath, sediment the magnetic microspheres with a magnet, remove the liquid phase, wash 6 times with 10 mL of distilled water each time, add 10 mL PBS buffer solution; weight $2.5\times10^{-4}$ mol biotin, add 10mL solution X, then add $1.0\times10^{-3}$ mol 1-(3-dimethylaminopropyl)-3-ethylcarbodiimide hydrochloride and 0.001 mol N-hydroxysuccinimide, stir and mix at the room temperature, stir and react for 15 min, adjust the pH of the solution to 7.2 by using $NaHCO_3$ solid powder, add to the magnetic microspheres containing 10 mL of PBS buffer solution after being washed. carry out a mechanical stirring in a 30°C water bath for 20 hours, followed by sedmenting the magnetic microspheres by a magnet, removing the liquid phase, and washing the magnetic microspheres with 10 mL of distilled water each time for 10 times, before obtaining the biotin modified biomagnetic microspheres D.

Embodiment 2: preparing the biomagnetic microspheres F having the avidin-Protein A combined (an affinity protein combined biomagnetic microsphere F) (taking the Protein A as a purification element, and taking the biotin-avidin affinity complex as a connection component)

2.1 synthesizing a ProteinA-eGFP-avidin fusion protein

Construct a DNA sequence of the fusion protein ProteinA-eGFP-Streptavidin and Protein A-eGFP-Tamvavidin2 composed by three genes respectively.

Wherein a sequence of the ProteinA comes from Staphylococcus Aureus, SPA in short. A total length of the amino acid sequence of the SPA is 516 amino acid residues, and a plurality of amino acids 37-327 thereof were selected as a gene sequence used in constructing the fusion protein, that is, an antibody binding domain of the SPA. After the sequence is optimized with an optimization program, a nucleotide sequence is obtained, referencing to SEQ ID NO: 1.

Tamvavidin2, an avidin analog, is a protein having an ability of binding the biotin. Discovered in 2009 by Yamamoto et al. (2009_FEBS_Yamanomo T_Tamavidins--novel avidin-like biotin-binding proteins from the Tamogitake mushroom), having a strong affinity to biotin, similar to Streptavidi, and in addition, a thermal stability thereof is better than that of Streptavidin.

The amino acid sequence of the Tamavidin2 can be retrieved from a plurality of relevant databases, including UniProt B9A0T7, which contains a total of 141 amino acid residues. After a codon conversion and an optimization program optimization, the DNA sequence is obtained, and an optimized nucleotide sequence is shown in SEQ ID NO: 2.

In addition, a nucleotide sequence of the enhanced fluorescent protein eGFP involved in the present embodiment is shown in SEQ ID NO: 3, which is a mutant at A206K of the eGFP, also referred to as mEGFP.

Construct the DNA templates of two fusion proteins ProteinA-eGFP-Streptavidin and Protein A-eGFP-Tamvavidin2 by adopting a recombinant PCR method. Then adopt an *in vitro* cell-free protein synthesis method to synthesize the two fusion proteins ProteinA-eGFP-Streptavidin and Protein A-eGFP-Tamvavidin2 respectively. Adopt a published in vitro cell-free protein synthesis method, referencing to a plurality of patent documents, including CN 201610868691.6, WO2018161374A1, KR20190108180A, CN108535489B, and more. Express the SPA-eGFP-Streptavidin and the SPA-eGFP-Tamavidin2 respectively in an IVTT system. In a summary, add a plurality of components to an IVTT system, including a cell extract (comprising a RNA polymerase integrated in the genome), a DNA template, an energy system (such as: a phosphocreatine-phosphocreatine kinase system), magnesium ions, sodium ions, polyethylene glycol and more, and the reaction is carried out under a condition of 28-30°C. The reaction is terminated after 8 to 12 hours. The IVTT reaction solution being obtained contains the ProteinA-eGFP-avidin fusion protein corresponding to the DNA template.

The IVTT reaction solutions of ProteinA-eGFP-Streptavidin and Protein A-eGFP-Tamvavidin2 were obtained respectively.

Express the SPA-eGFP-Streptavidin (corresponding to "1" in FIG.4) and the SPA-eGFP-Tamavidin2 (corresponding to "2" in FIG.4), before measuring the RFU values for the proteins being synthesized, and a result is shown as "total" in FIG.4.

Centrifugate the IVTT reaction solutions of the ProteinA-eGFP-Streptavidin and the ProteinA-eGFP-Tamavidin2 at 4000 rpm and 4°C for 10 min, respectively, retain a supernatant, denoted as an IVTT supernatant.

2.2 Preparing a protein A-binding biomagnetic microsphere F

Incubate the IVTT supernatants of the two fusion proteins of ProteinA-eGFP-Streptavidin and ProteinA-eGFP-Tamavidin2 together with the biomagnetic microspheres D obtained in the embodiment 1, and react for 1 hour, before meausing a content amount of the fusion protein in the biomagnetic microspheres F respectively, according to the method stated above, and comparing a binding ability of the two fusion proteins. A result is shown as "supernatant" in FIG.4.

Pipette 30 μL of 10% (v/v) suspension of the biomagnetic microspheres D, wash 3 times by a binding/washing buffer (10 mM $Na_2HPO_4$ with a pH 7.4, 2 mM $KH_2PO_4$, 140 mM NaCl, 2.6 mM KCl) for use.

Take 2 mL of the IVTT supernatant containing the fusion protein of the avidin-protein A and incubate in a rotation with the biomagnetic microspheres D stated above at 4 °C for 1 hour, collect an unbound supernatant, which is a flow-through liquid, and repeat this step for three times, before obtaining three different flow-through liquids. That is, a same batch of the biomagnetic microspheres D will continuously incubate with the avidin-protein A for three times. Each time, use 2 mL of the IVTT supernatant described in step 2.1, and a corresponding flow-through liquid obtained each time is numbered as 1, 2, and 3 in a sequence. Detecting a plurality of eGFP fluorescence values of the IVTT supernatant and the three flow-through liquids by a fluorophotometry. A result is shown in FIG.5, an excitation wavelength (Ex) was 488 nm, and an emission wavelength (Em) was 507 nm. The closer the RFU value of the three flow-through liquids solution is to the RFU value of the IVTT supernatant, the more saturated a binding of the biomagnetic microspheres D to the avidin-avidin A, and a plurality of corresponding RFU values are shown in Table 1 below:

Table 1 A comparison of the eGFP fluorescence values of the IVTT supernatant without a biomagnetic microsphere D treatment and three flow-through liquids obtained after the treatment.

| RFU value | IVTT supernatant | Flow through 1 | Flow through 2 | Flow through 3 |
|---|---|---|---|---|
| Protein A-eGFP-Streptavidin | 1324.3 | 950.7 | 1316.5 | 1338.0 |
| Protein A-eGFP-Tamavidin2 | 1264.0 | 915.3 | 1158.0 | 1247.0 |

The RFU values of the SPA-eGFP-Streptavidin and the SPA-eGFP-Tamvavidin2 bound to the biomagnetic microsphere D are shown in Table 2 and Table 3 below, respectively. Wherein for the Streptavidin, a first binding has saturated a protein A binding capacity of the biomagnetic microsphere D, for Tamvavidin2, after a second binding, a binding amount of the protein A on the biomagnetic microsphere D has basically saturated. A first binding amount was calculated by subtracting the protein amount in the flow-through liquid 1 from the protein amount in the supernatant, and a second binding amount was calculated by the protein amount in the supernatant minus the protein in the flow-through liquid 2; a third binding amount was calculated by subtracting the protein amount in the flow-through liquid 3 from the protein amount in the supernatant, as shown in Table 2 and Table 3 below. Wherein, a binding ablility refers to the ProteinA fusion protein/biomagnetic microsphere D, which is a mass to volume ratio, having a unit of (mg/mL)

Table 2 Physicochemical comparison of the flow-through liquid of the SPA-eGFP-Streptavidin treated with the biomagnetic microspheres D

| Component | RFU value | Concentration (μg/mL) | Vol. (mL) | Mass (μg) | Protein A fusion protein (μg) | Total pro. bound (μg) | Binding ability (mg /mL) |
|---|---|---|---|---|---|---|---|
| IVTT supernatant | 1324.3 | 239.9 | 2 | 479,9 | / | 144.5 | 48.2 |
| Flow-through 1 | 950.7 | 169.2 | ’2 | 338.4 | 141.5 | | |
| Flow-through 2 | 1316.5 | 238.4 | 2 | 476.8 | 3.0 | | |
| Flow-through 3 | 1338.0 | 242.5 | 2 | 485.0 | 0.0 | | |

Table 3 Physicochemical comparison of the flow-through liquid of the SPA-eGFP-Tamvavidin2 treated with the biomagnetic microspheres D

| Component | RFU value | Concentration (μg/mL) | Vol. (mL) | Mass (μg) | Protein A fusion protein (μg) | Total pro. bound (μg) | Binding ability (mg/mL) |
|---|---|---|---|---|---|---|---|
| IVTT supernatant | 1264.0 | 234.6 | 2 | 469.3 | / | 183.3 | 61,1 |
| Flow-through 1 | 915.3 | 167.0 | 2 | 333.9 | 135.4 | | |
| Flow-through 2 | 1158.0 | 214.0 | 2 | 428.0 | 41.3 | | |
| Flow-through 3 | 1247.0 | 231.3 | 2 | 462.6 | 6.6 | | |

According to a standard curve of the fluorescence value and the protein mass concentration, the protein concentration of each binding was calculated, and a total protein amount of each binding was calculated according to an incubation volume (2 mL).

According to a purified eGFP simulation standard curve, a formula converting the RFU value of the eGFP into a protein concentration is:

$$X = -\frac{1}{0.0005} \times \frac{\ln\left(1 - \frac{Y - 38.089}{31692}\right)}{M} \times N$$

wherein X is a protein concentration (μg/mL), Y is a RFU fluorescence reading number, M is a molecular weight of the eGFP (26.7kDa), and N is a molecular weight of the SPA-eGFP-Streptavidin (77.3kDa) or a molecular weight of the SPA-eGFP-Tamvavidin2 (79.4kDa).

By taking the RFU fluorescence values having been measured, a mass concentration (μg/mL) of the target protein SPA-eGFP-Streptavidin or SPA-eGFP-Tamvavidin2 can be converted and obtained.

Wherein, a RFU value of the IVTT supernatant and the flow-through liquid of the avidin-protein A fusion protein is Y, taking a value of Y into the formula above, before obtaining an X as a mass concentration of a corresponding fusion protein, followed by multiplying with a volume of the protein A solution, a total protein amount of the avidin-protein A fusion protein will be obtained. The protein amount of the avidin-protein A in the IVTT supernatant minus the protein amount of the avidin-protein A in the flow-through liquids, a difference obtained is a protein amount W of the avidin-protein A being bound by the biomagnetic microspheres. Divide W by a column bed volume of the magnetic microspheres, a mass of a fusion protein of the avidin-protein A bound to the magnetic microspheres per unit volume is then calculated and obtained, which is a binding force, having a unit of mg/mL.

In the present embodiment, 30 μL of 10% (v/v) biomagnetic microsphere D suspension was used, so a binding amount by converting into 1 mL of 100% biomagnetic microsphere D is shown in Table 2 and Table 3. Wherein a binding force of the Tamvavidin2 to the biomagnetic microsphere D was slightly stronger than that of the Streptavidin.

Embodiment 3: applying the Protein A-modified biomagnetic microspheres F to a serum antibody purification

Different volumes of the biomagnetic microspheres having the SPA-eGFP-Streptavidin fusion protein bound were rotated and incubated with 1 mL of neonatal bovine serum at 4°C for 1 hour, followed by being washed three times with 1 mL of binding/washing buffer, each washing process was incubated at 4°C and rotated for 10 minutes. Taking 100 μL of 0.1 M glycine in pH 2.8 to eluate the antibody and the antibody being eluted was present in the eluate, to which one tenth volume (10 μL) of 1 M Tris-HCl in a pH 8.0 was added immediately. Taking a Protein A agarose column of Sangon biotech as a control (Cat. NO C600957-0005).

Take 40 μL of an eluate and add into 10 μL of 5×SDS loading buffer (without a reducing agent), and take a metal bath at 95°C for 10 minutes, before taking a detection of SDS-PAGE electrophoresis. A detection result is shown in FIG. 6.

It can be seen from FIG.6 that, by adiopting the biomagnetic microspheres combined with the SPA-eGFP-Streptavidin fusion protein, together with a 6 microliters column bed volume, a separation and purification effect superior to that of a commercially available product will be obtained.

Wash the biomagnetic microspheres having been eluted three times with 1 mL of binding/washing buffer, and repeat the steps above including antibody incubating, washing, and eluting; that is, after a first antibody incubation, followed by washing, eluting, and releasing the antibody being bound, the protein A-modified magnetic microspheres are obtained again; then perform the incubation, washing, and elution with the antibody again, to obtain the protein A-modified magnetic microspheres again, followed by incubating, washing, and eluting for a third time. After incubating the biomagnetic microspheres with the antibody for a total of 3 times, take 40 μL of the eluate containing the antibody obtained after the third incubation with the antibody, add 10 μL of 5× loading buffer (without a reducing agent), and perform a 95°C metal bath for 10 minutes, before performing a detection by an SDS-PAGE electrophoresis, and a detection result were shown in FIG. 7.

It can be seen from FIG.6 and FIG.7 that, the biomagnetic microspheres F combined with SPA-eGFP-Streptavidin can be used repeatedly. Antibodies in bovine serum can still be extracted, and the overall effect is no worse than that of Sanko's ProteinA agarose magnetic beads. For 6 microliter column bed volume, the present invention also has better effect. The biomagnetic microspheres F combined with the affinity protein (herein, protein A) provided by the present invention can be used repeatedly.

Embodiment 4: recyclability of a binding of the biomagnetic microspheres D and the affinity complex E (avidin-protein A)

4.1 Incubating

Incubate to prepare a SPA-eGFP-Tamvavidin2-binding biomagnetic microsphere F:
Take 50 μL of 10% biomagnetic microsphere D suspension (5 μL in volume), and wash 3 times with 2 mL of binding/washing buffer, add 2 mL of SPA-eGFP-Tamvavidin2 IVTT supernatant, incubate at 4°C and rotate for 1 hour, before discarding a supernatant (collect the supernatant to obtain a flow-through liquid 1), add fresh IVTT supernatant containing the SPA-eGFP-Tamvavidin2 repeatedly, incubate at 4°C and rotate for 1 hour, discard the supernatant (collect the supernatant to obtain a flow-through liquid 2), then add fresh IVTT supernatant containing the SPA-eGFP-Tamvavidin2 for a third time, incubate with rotate at 4°C for 1 hour, discard the supernatant (collect the supernatant to obtain a flow-through liquid 3). Measure the RFU value of the IVTT supernatant and the RFU values of three flow-through liquids of the SPA-eGFP-Tamvavidin2, and calculate a remaining content of the SPA-eGFP-Tamvavidin2 in the solution according to the above formula. The results are shown in FIG.8 and Table 4.

According to the standard curve based on the purified eGFP, the calculated RFU value of the eGFP is converted into the calculation formula of protein concentration:

$$X = -\frac{1}{0.0005} \times \frac{\ln\left(1 - \frac{Y - 38.089}{31692}\right)}{M} \times N$$

Wherein, X is the protein concentration (μg/mL), Y is the RFU fluorescence reading, M is the molecular weight of eGFP (26.7kDa), and N is the molecular weight of SPA-eGFP-Tamvavidin2 (79.4kDa).

By substituting the measured RFU fluorescence value into the calculation formula, the mass concentration (μg/mL) of the target protein SPA-eGFP-Tamvavidin2 can be obtained. For example: when the value of Y is 1330.3, the corresponding value of X is calculated by the above formula to be 247.6, as shown in Table 4.

4.2 eluting and replacing

Avidin-avidin complex E: SPA-eGFP-Tamvavidin2 was eluted and replaced.

Wash the biomagnetic microspheres F for 3 times with 2 mL of binding/washing buffer, add 200 μL of denaturing buffer (1M Urea and 10% SDS), and incubate in a metal bath at 95°C for 10 minutes to elute the Avidin-Protein A binding on the biomagnetic microspheres. Take 20 μL of the eluate and add 5 μL of 5×SDS loading buffer, before performning a SDS-PAGE electrophoresis to obtain a plurality of bands in lane 1 as shown in FIG.9. The biomagnetic microspheres D after the elution are washed three times with 2 mL of the binding/washing buffer to obtain the first regenerated biomagnetic microspheres D. The binding sites of the biotin on the branched-chains of the polymer on the outer surface of the magnetic microspheres are released.

4.3 a second incubation (a regeneration)

Repeat the process stated above of incubating the SPA-eGFP-Tamvavidin2 and eluting the avidin-protein A to achieve a first regeneration of the biomagnetic microspheres F, that is, a first replacement of the avidin-protein A. The biomagnetic microspheres D regenerated for the first time are combined with SPA-eGFP-Tamvavidin2, and the RFU values of the corresponding IVTT supernatant and three times flow-through liquids were measured, and a data in Table 5 is obtained. The biomagnetic microspheres D having been eluted are washed with 2 mL of the binding/washing buffer for three times, before obtaining the biomagnetic microspheres D regenerated for a second time.

4.4 a third incubation (a second regeneration)

Repeat again the process stated above of incubating the SPA-eGFP-Tamvavidin2 and eluting the avidin-protein A to achieve a second regeneration of the biomagnetic microspheres F, that is, a second replacement of the avidin-protein A. The biomagnetic microspheres D regenerated for the second time are combined with SPA-eGFP-Tamvavidin2, and the RFU values of the corresponding IVTT supernatant and both flow-through liquids are measured, and a data in Table 6 is obtained. The tables 4-6, wherein the binding force is referencing to ProteinA fusion protein/biomagnetic microsphere D, mass to volume ratio, unit (mg/mL).

Table 4 Calculation on a loading capacity of the first prepared biomagnetic microspheres D combined with the Protein A fusion protein

| Component | RFU value | Concentration (μg/mL) | Vol. (mL) | Mass (μg) | Protein fusion protein | A Total pro. bound (μg) | Binding ability (mg/mL) |
|---|---|---|---|---|---|---|---|
| IVTT supernatant | 1330.3 | 247.6 | 2 | 495.2 | / | 291.5 | 58.3 |
| Flow-through 1 | 886.7 | 161.4 | 2 | 322.8 | 172.4 | | |
| Flow-through 2 | 1172.3 | 216.8 | 2 | 433.5 | 61.7 | | |
| Flow-through 3 | 1183.3 | 218.9 | 2 | 437.8 | 57.4 | | |

Table 5 Calculation on the capacity of the first regenerated biomagnetic microspheres D combined with Protein A fusion protein

| Component | RFU value | Concentration (μg/mL) | Vol. (mL) | Mass (μg) | Protein fusion protein | A Total pro. bound (μg) | Binding ability (mg/mL) |
|---|---|---|---|---|---|---|---|
| IVTT supernatant | 1334.7 | 248.5 | 2 | 497.0 | / | 251.1 | 50.2 |
| Flow-through 1 | 955.3 | 174.7 | 2 | 349.4 | 147.7 | | |
| Flow-through 2 | 1211.3 | 224.4 | 2 | 448.7 | 48.3 | | |
| Flow-through 3 | 1193.7 | 220.9 | 2 | 441.8 | 55.2 | | |

Table 6 Calculation on the capacity of the second regenerated biomagnetic microspheres D combined with Protein A fusion protein

| Component | RFU value | Concentration (μg/mL) | Vol. (mL) | Mass (μg) | Protein A fusion protein (μg) | Total pro. bound (μg) | Binding ability (mg/mL) |
|---|---|---|---|---|---|---|---|
| IVTT supernatant | 1534.3 | 287.6 | 2 | 575.3 | / | 355.6 | 71.1 |
| Flow-through 1 | 990.0 | 181.4 | 2 | 362.8 | 212.5 | | |
| Flow-through 2 | 1292.0 | 240.1 | 1.8 | 432.2 | 143.1 | | |

Measure an ability of the regenerated biomagnetic microspheres D to re-bind the protein A-eGFP-avidin.

The biomagnetic microspheres D prepared for a first time were saturated with SPA-eGFP-Tamvavidin2 for a first time, the biomagnetic microspheres D regenerated for a first time were saturated with SPA-eGFP-Tamvavidin2 for a second time, the biomagnetic microspheres D regenerated for a second time were saturated with SPA-eGFP-Tamvavidin2 for a third time, and the biomagnetic microspheres D were regenerated for the second time The third saturation binds SPA-eGFP-Tamvavidin2, the protein was eluted with a denaturing buffer respectively, and three eluates containing the SPA-eGFP-Tamvavidin2 were collected and subjected to an SDS-PAGE. A result is shown in FIG.9.

Lanes 1, 2, and 3 represent respectively three electrophoretic bands of three eluates containing SPA-eGFP-Tamvavidin2, after the first, the second, and the third times saturated binding the SPA-eGFP-Tamvavidin2, elute a protein from the biomagnetic microspheres F prepared for the first time, the biomagnetic microspheres F regenerated for the first time, the biomagnetic microspheres F regenerated for the second time.

4.5 detecting an ability of a regenerated biomagnetic microsphere F to bind to a bovine serum antibody

When the biomagnetic microsphere D was saturated with SPA-eGFP-Tamvavidin2 for a third time, the biomagnetic microsphere F regenerated for a second time was obtained. Add 2 mL of newborn bovine serum (commercially available product, same as below) to the biomagnetic microspheres F obtained for a second time, incubate at 4°C and rotate for 1 hour, discard the supernatant (collect the supernatant to obtain a flow-through liquid 1). Add 2 mL of the newborn calf serum again and incubate at 4°C, rotate for 1 hour, and discard the supernatant (collect the supernatant to obtain a flow-through liquid 2). Wash 3 times with 1 mL of binding/washing buffer, during each wash, incubate at 4°C and rotate for 10 min. Elute a bovine serum antibody (the antibody is bound to a terminus of the protein A in the magnetic microspheres) with 100 μL of 0.1M glycine solution at pH 2.8, and immediately add 1/10 volume (i.e., 10 μL) 1 M pH 8.0 Tris-HCl solution to obtain 110 μL of eluate.

Take 40 μL of the eluate stated above, add 10 μL of 5×SDS loading buffer (no reducing agent), mix well, and perform an SDS-PAGE electrophoresis detection after 95°C metal bath for 10 minutes. The detection results are shown in FIG.9. According to a plurality of experimental results of FIG.7, FIG.9 and Table 6, it can be seen that a combination of the biomagnetic microsphere D and the SPA-eGFP-Tamvavidin2 is reproducible, and the biomagnetic microsphere F provided by the present application can be used regeneratively. Moreover, a step of adding the denaturing solution and heat treating does not affect a binding efficiency of the biomagnetic microspheres.

Embodiment 5: preparing the biomagnetic microspheres G having the protein G bound (taking the protein G as the purification element and taking the biotin-avidin affinity complex as the connection component)

5.1. Synthesizing a ProteinG-eGFP-avidin fusion protein (an avidin-affinity protein covalently linked complex E)

Take the method of the Embodiment 2, synthesize a ProteinG-eGFP-Tamvavidin2 fusion protein (also abbreviated as a ProteinG fusion protein), before preparing an IVTT reaction solution containing the ProteinG-eGFP-Tamvavidin2 fusion protein and an IVTT supernatant sequentially.

First, construct a DNA sequence of a ProteinG-eGFP-Tamvavidin2 fusion protein containing three gene sequences. Wherein, the nucleotide sequences of the "eGFP" segment and the "Tamvavidin2" segment are as same as those in the Embodiment 2, shown as SEQ ID NO: 3 and SEQ ID NO: 2, respectively. Wherein, the nucleotide sequence of the "ProteinG" segment (SEQ ID NO: 4) is from *Streptococcus sp.* group G, specifically the gene sequence of an antibody binding region thereof.

Adopt a recombinant PCR method and construct a DNA template of the ProteinG-eGFP-Tamvavidin2 fusion protein. Adopt a RCA method and performing an *in vitro* amplification. And adopt the *in vitro* cell-free protein synthesis method stated above (specifically adopting a cell extract based on *Kluyces lactis* cell), to synthesize the ProteinG-eGFP-Tamvavidin2 fusion protein.

Carry out an IVTT reaction before obtaining an IVTT reaction solution containing the ProteinG-eGFP-Tamvavidin2 fusion protein and an IVTT supernatant sequentially.

5.2. Preparing the biomagnetic microsphere G having the protein G bound

Pipette 30 μL of 10% (w/v) biotin-modified biomagnetic microspheres D prepared in the Embodiment 1, and wash with a binding/washing buffer (10 mM $Na_2HPO_4$ with a pH 7.4, 2 mM $KH_2PO_4$, 140 mM NaCl, 2.6 mM KCl) for three times as a backup.

Take 2 mL of the IVTT supernatant containing the ProteinG-eGFP-Tamvavidin2 fusion protein, rotate and incubate with the biomagnetic microspheres D stated above at 4°C for 1 hour, before collecting the supernatant, which is the flow-through liquid. The flow-through liquid contains a plurality of the fusion proteins left without binding to the spheres; repeat for three times, each time take and incubate a new IVTT supernatant with the biomagnetic microspheres D to obtain three different flow-through liquids. That is, incubating a same batch of the biomagnetic microspheres D three times continuously with the ProteinG-eGFP-Tamvavidin2 fusion protein in the IVTT supernatant. Each time, take 2 mL of the IVTT supernatant obtained in the step 5.1, and record three flow-through liquids correspondingly as a flow-through liquid 1 (FT1), a flow-through liquid 2 (FT2), and a flow-through liquid 3 (FT3) sequencially. Adopt fluorophotometry to detect a fluorescence value of the eGFP (measured by a RFU value) of the IVTT supernatant and the three flow-through liquids, the results are shown in FIG. 10.

The closer the RFU value of the flow-through liquid to the RFU value of the IVTT supernatant is, the more saturated a binding of the biomagnetic microspheres D to the ProteinG-eGFP-Tamvavidin2 fusion protein is. A corresponding RFU value is shown in Table 7 below. A first binding amount was calculated by subtracting a protein amount in the flow-through liquid 1 from a protein amount in the supernatant, a second binding amount was calculated by subtracting a protein amount in the flow-through liquid 2 from the protein amount in the supernatant, and a third binding amount was calculated by subtracting a protein amount in the flow-through 3 from the protein amount in the supernatant.

A concentration of the ProteinG-eGFP-Tamvavidin2 fusion protein was calculated according to a formula below using the method in the Embodiment 2.

$$X = -\frac{1}{0.0005} \times \frac{\ln\left(1 - \frac{Y - 38.089}{31692}\right)}{M} \times N$$

Wherein, X is a mass concentration (μg/mL) of the proteinG-eGFP-Tamvavidin2 fusion protein, Y is a RFU fluorescence reading value, M is a molecular weight of the eGFP (26.7 kDa), and N is a molecular weight of the ProteinG-eGFP-Tamvavidin2 fusion protein.

Table 7: Data on the eGFP fluorescence values of the IVTT supernatant and three flow-through liquids containing the ProteinG-eGFP-Tamvavidin2 fusion protein

| Bind. times | Object for test | RFU value | Con. of fusion protein (μg/mL) | Vol. (mL) | Mass (μg) | Fusion protein bound (μg) | Total fusion pro. bound (μg) | Binding ability (mg /mL) |
|---|---|---|---|---|---|---|---|---|
| 1 | IVTT super. | 576 | 85.7 | 2 | 171.4 | 23.8 | 59.0 | 19.7 |
| | F-T 1 | 502 | 73.8 | 2 | 147.6 | | | |
| 2 | IVTT super. | 576 | 85.7 | 2 | 171.4 | 33.6 | | |
| | F-T 2 | 472 | 68.9 | 2 | 137.8 | | | |
| 3 | IVTT super. | 576 | 85.7 | 2 | 171.4 | 1.6 | | |
| | F-T 3 | 571 | 84.9 | 2 | 169.8 | | | |

Wherein the binding force is a mass of the ProteinG fusion protein bound to the magnetic beads to a volume of the magnetic beads used.

Through a process of incubating the biomagnetic microspheres D with the IVTT supernatant containing the ProteinG fusion protein as stated above, the biomagnetic microspheres G having the ProteinG bound by a connection of the affinity complex (biotin-avidin) are obtained, also denoted as ProteinG magnetic beads.

5.3. Purify a serum antibody (using an IgG antibody as a target protein) by the ProteinG magnetic beads

Rotate and incubate a plurality of different volumes of the ProteinG magnetic beads having the ProteinG-eGFP-Tamvavidin2 fusion protein bound with 1 mL of neonatal bovine serum for 1 hour at 4°C, before washing with 1 mL of the binding/washing buffer (3 times), each wash is incubated and rotated at 4°C for 10 min. Elute the antibody with 100 μL of 0.1 M glycine having a pH 2.8, and add a tenth volume (10 μL) of 1 M Tris-HCl having a pH 8.0 to the eluate immediately. Detect an IgG antibody purity in the eluate by SDS-PAGE, and a result is shown in FIG. 11. Perform a quantitative analysis according to a gray value, and a purity was greater than 95%.

6. Embodiment 6: preparing a biomagnetic microsphere H having a nanobody of antiEGFP bouind (taking the nanobody of anti-eGFP as the purification element and taking the biotin-avidin affinity complex as the connection component)

6.1. Synthesizing an antiEGFP-mScarlet-avidin fusion protein (an antiEGFP-mScarlet-Tamvavidin2 fusion protein, a nanobody fusion protein)

Take the method of the Embodiment 2, synthesize the antiEGFP-mScarlet-Tamvavidin2 fusion protein (also abbreviated as the antiEGFP fusion protein, having a molecular weight of 59kDa), and prepare the IVTT reaction solution containing the antiEGFP-mScarlet-Tamvavidin2 fusion protein and the IVTT supernatant in turn.

First, construct the DNA sequence of the antiEGFP-mScarlet-Tamvavidin2 fusion protein containing the three gene sequences.

Wherein, the nucleotide sequence of the "Tamvavidin2" segment is as same as that in the Embodiment 2, referencing to SEQ ID NO:2.

Wherein, the antiEGFP is a nanobody having an amino acid sequence as shown in SEQ ID NO:5.

Wherein, the mScarlet is a bright red fluorescent protein, having an amino acid sequence as shown in SEQ ID NO:6 accordingly.

Adopt a recombinant PCR method to construct the DNA template of the antiEGFP-mScarlet-Tamvavidin2 fusion protein. Adopt a RCA method to carry out an *in vitro* amplification. And adopt the *in vitro* cell-free protein synthesis method indicated before to synthesize the antiEGFP-mScarlet-Tamvavidin2 fusion protein.

Carry out an IVTT reaction before obtaining an IVTT reaction solution containing the antiEGFP-mScarlet-Tamvavidin2 fusion protein and an IVTT supernatant sequentially.

6.2 Preparing the biomagnetic microsphere H having the nanobody of antiEGFP bound

Pipette 30 μL of 10% (w/v) biotin-modified biomagnetic microspheres D prepared in the Embodiment 1, and wash with a binding/washing buffer (10 mM $Na_2HPO_4$ with a pH 7.4, 2 mM $KH_2PO_4$, 140 mM NaCl, 2.6 mM KCl) for three times as a backup.

Take 2 mL of the IVTT supernatant containing the antiEGFP-mScarlet-Tamvavidin2 fusion protein (with a RFU value of 2400) and the biomagnetic microspheres D mentioned above, rotate and incubate at 4°C for 1 hour, and collect the supernatant, which is the flow-through liquid (with a RFU value of 1700). The flow-through liquid contains a plurality of remaining fusion proteins not bound by the microspheres. A test condition of the RFU value is: an excitation wavelength (Ex) of 569 nm, an emission wavelength (Em) of 593 nm.

Through a process of incubating the biomagnetic microspheres D with the IVTT supernatant containing the antiEGFP fusion protein stated above, an incubated magnetic bead binds with the nanobody of anti-eGFP through connecting the affinity complex (biotin-Tamvavidin2), which is denoted as a biomagnetic microsphere H, and also denoted as an antiEGFP magnetic bead (a nanobody magnetic bead).

6.3 a loading test of the antiEGFP magnetic bead binding to the eGFP protein (taking the eGFP as the target protein, and the eGFP is excess)

Pipette 30 μL of 10% (w/v) antiEGFP magnetic beads prepared in the Embodiment 6.2. and wash 3 times with a binding/washing buffer, for a backup.

Take 2 mL of the IVTT reaction solution containing the eGFP protein (the nucleotide sequence encoding the eGFP in the DNA template is shown as SEQ ID NO: 3), measure a fluorescence value thereof, and record as a fluorescence value of Total. Mix with 3 μL of the antiEGFP magnetic beads washed previously, rotate and incubate for 1 hour. Denote a supernatant not bound to the magnetic beads as Flow-through, and measure a fluorescence value thereof. According to a test result of the fluorescence value in Table 8, an amount of the eGFP protein is obtained correspondingly by a formula of the eGFP, and a load of the antiEGFP magnetic beads was calculated to be 17.7 mg/mL (a mass of the eGFP protein bound to each milliliter of the antiEGFP magnetic beads).

Table 8 a loading test result of the antiEGFP magnetic beads binding to the eGFP protein

| Object for test | Mark | RFU avg. | Mass con. of eGFP protein (μg/mL) | Vol. (mL) | eGFP protein (μg) |
|---|---|---|---|---|---|
| IVTT supertanant | Total | 2300 | 166.4 | 2 | 332.8 |
| F-T | Flow-through | 1950 | 139.8 | 2 | 279.7 |

6.4. a binding efficiency of the antiEGFP magnetic beads purifying the eGFP protein (taking the eGFP as the target protein, and the magnetic beads are excess)

Take the antiEGFP magnetic beads prepared in the Embodiment 6.2, wash three times with the binding/washing buffer, as a backup.

Take 1 mL of the IVTT reaction solution containing the eGFP protein (the nucleotide sequence encoding the eGFP in the DNA template is shown as SEQ ID NO:3), measure a fluorescence value thereof, and denote as Total. Mix with excess antiEGFP magnetic beads, rotate and incubate for 1 hour, collect a supernatant, record as a Flow-through, and measure a fluorescence value thereof. Wash the magnetic beads twice with 1 mL of the binding/washing buffer, incubate and rotate at 4°C for 10 min during each wash. Record a plurality of washing solutions obtained as Washing1 and Washing2, respectively, and measure the fluorescence values thereof. A magnetic bead having been incubated has the target protein of eGFP bound. A measurement result of the fluorescence values mentioned above is shown in FIG. 12 and Table 9 respectively. The result has shown that the anti-eGFP magnetic beads prepared by a protocol stated in the present application is able to bind and elute the eGFP protein effectively. According to the fluorescence values of the IVTT supernatant and the flow-through liquid, it was calculated that, the binding efficiency of the antiEGFP magnetic beads to the target protein eGFP after 1 hour incubation was 98.2%.

Table 9 binding efficiency test of the antiEGFP magnetic beads purifying the eGFP protein

| Object for test | Mark | RFU avg. | Mass con. of eGFP protein (μg/mL) | Vol. (mL) | eGFP protein (μg) |
|---|---|---|---|---|---|
| IVTT supertanant | Total | 430 | 27.96 | 1 | 27.96 |
| F-T | Flow-through | 45 | 0.49 | 1 | 0.49 |

Elute the eGFP with 100 μL of 0.1 M glycine having a pH 2.8, and add one-tenth volume (10 μL) of 1 M Tris-HCl having a pH 8.0 to an eluate immediately. Measure a fluorescence value of the eluate and record as Elution, before checking a purity by the SDS-PAGE. A result is shown in FIG.13, and a purity is about 95%.

It should be understood that, the application of the present application is not limited to the above examples listed. Ordinary technical personnel in this field can improve or change the applications according to the above descriptions, all of these improvements and transforms should belong to the scope of protection in the appended claims of the present application.

## Claims

1. A biomagnetic microsphere, comprises a magnetic microsphere body, wherein an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of biotins or biotin analogs connected.

2. The biomagnetic microsphere according to claim 1, wherein an end of the branched-chain of the polymer connects to a purification element through a connection component, the connection component comprises the biotins or the biotin analogs.

3. The biomagnetic microsphere according to claim 2, wherein the purification element comprises an avidin-type tag, a polypeptide-type tag, a protein-type tag, an antibody-type tag, an antigen-type tag, or a combination thereof; preferably, the avidin-type tag is avidin, a biotin-binding avidin analog, a biotin analog-binding avidin analog, or a combination thereof; more preferably, an end of the branched-chain of the polymer on the biomagnetic microsphere connects with biotin; the purification element is avidin, which forms a binding effect of an affinity complex with the biotin; more preferably, the avidin is a streptavidin, a modified streptavidin, a streptavidin analog, or a combination thereof; preferably, the polypeptide-type tag is selected from any one of following tags or a variant thereof: a CBP tag, a histidine tag, a C-Myc tag, a FLAG tag, a Spot tag, a C tag, an Avi tag, a Streg tag, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof; preferably, the protein tag is selected from any one of following tags or a variant protein thereof: an affinity protein, a SUMO tag, a GST tag, an MBP tag and a combination thereof; more preferably, the affinity protein is selected from Protein A, Protein G, Protein L, modified Protein A, modified Protein G, modified Protein L and a combination thereof.

4. The biomagnetic microsphere according to claim 2 or 3, wherein the purification element connects to an end of the branched-chain of the polymer through a connection component containing an affinity complex; preferably, the biotin or the biotin analog has the avidin or the avidin analog connected through the affinity complex interaction, the purification element connects to the avidin or the avidin analog directly or indirectly; more preferably, the purification element connects to the biotin or the biotin analog at an end of the branched-chain of the polymer through an avidin-type tag-purification element covalent ligation complex, and through a connection component that is an affinity complex formed between the avidin-type tag and the biotin or the biotin analog; further preferably, the purification element forms a connection component of the affinity complex with the biotin or the biotin analog at the end of the branched-chain of the polymer by a avidin-purification element covalent ligation complex.

5. The biomagnetic microsphere according to any one of claims 2-4, wherein a connection method between the purification element and the end of he branched-chain is: through a covalent bond, through supramolecular interaction, through a connection component, or through a combination thereof; preferably, the covalent bond is a dynamic covalent bond; more preferably, the dynamic covalent bond comprises an imine bond, an acylhydrazone bond, a disulfide bond or a combination thereof; preferably, the supramolecular interaction is selected from: a coordination binding, an affinity complex interaction, an electrostatic adsorption, a hydrogen bonding, a $\pi$-$\pi$ overlapping interaction, a hydrophobic interaction or a combination thereof; preferably, the affinity complex interaction is selected from: a biotin-avidin interaction, a biotin analog-avidin interaction, a biotin-avidin analog interaction, a biotin analog-avidin analog interaction.

6. The biomagnetic microsphere according to claim 1, wherein further comprising an avidin combined with the biotin or the biotin analog; wherein, a binding action of an affinity complex is formed between the biotin or the biotin analog and the avidin; preferably, the avidin is any one of streptavidin, modified streptavidin, and streptavidin analogs or a combination thereof.

7. The biomagnetic microsphere according to claim 6, further comprises an affinity protein connecting to the avidin or the avidin analog.

8. The biomagnetic microsphere according to any one of claims 1-7, wherein a size of the magnetic microsphere body is selected from any one of following particle size scales or a range between any two particle size scales: 0.1 μm, 0.15 μm, 0.2 μm, 0.25 μm, 0.3 μm, 0.35 μm, 0.4 μm, 0.45 μm, 0.5 μm, 0.55 μm, 0.6 μm, 0.65 μm, 0.7 μm, 0.75 μm, 0.8 μm, 0.85 μm, 0.9 μm, 0.95 μm, 1 μm, 1.5 μm, 2 μm, 2.5 μm, 3 μm, 3.5 μm, 4 μm, 4.5 μm, 5 μm, 5.5 μm, 6 μm, 6.5 μm, 7 μm, 7.5 μm, 8 μm, 8.5 μm, 9 μm, 9.5 μm, 10 μm, 15 μm, 20 μm, 25 μm, 30 μm, 35 μm, 40 μm, 45 μm, 50 μm, 55 μm, 60 μm, 65 μm, 70 μm, 75 μm, 80 μm, 85 μm, 90 μm, 95 μm, 100 μm, 150 μm, 200 μm, 250 μm, 300 μm, 350 μm, 400 μm, 450 μm, 500 μm, 550 μm, 600 μm, 650 μm, 700 μm, 750 μm, 800 μm, 850 μm, 900 μm, 950 μm, 1000 μm; the particle size is an average value; preferably, a diameter of the magnetic microsphere body is selected from 0.1-10 μm; preferably, a diameter of the magnetic microsphere body is selected from 0.2-6 μm; preferably, a diameter of the magnetic microsphere body is selected from 0.4-5 μm; preferably, a diameter of the magnetic microsphere body is selected from 0.5-3 μm; preferably, a diameter of the magnetic microsphere body is selected from 0.2-1 μm; preferably, a diameter of the magnetic microsphere body is selected from 0.5-1 μm; preferably, a diameter of the magnetic microsphere body is selected from 1 μm-1mm; preferably, an average diameter of the magnetic microsphere body is 200nm, 250nm, 300nm, 350nm, 400nm, 450nm, 500nm, 550nm, 600nm, 650nm, 700nm, 750nm, 800nm, 850nm, 900nm, 950nm, 1000nm, with a deviation of ±20%, more preferably ±10%.

9. The biomagnetic microsphere according to any one of claims 1-8, wherein the linear backbone of the polymer is a polyolefin backbone or an acrylic polymer backbone; preferably, the linear backbone of the polymer is a polyolefin backbone, and is provided by a backbone of an acrylic polymer; more preferably, a monomer unit of the acrylic polymer is one of acrylic acid, acrylate, acrylic ester, methacrylic acid, methacrylate, methacrylate easter, or a combination thereof.

10. The biomagnetic microsphere according to claim 1, wherein the branched-chain of the polymer covalently binds to the biotin or the biotin analogs through a covalent bond based on a functional group; preferably, the covalent bond based on the functional group refers to a covalent bond having a functional group participating in a covalent coupling, wherein the functional group is a carboxyl group, a hydroxyl group, an amino group, a sulfhydryl group, a salt form of a carboxyl group, a salt form of an amino group, a formate group, or a combination thereof.

11. The biomagnetic microsphere according to any one of claims 1-10, wherein the linear backbone is covalently fixed onto the outer surface of the magnetic microsphere body in a direct manner or an indirectly manner through a connection group.

**12.** The biomagnetic microsphere according to any one of claims 1-11, wherein the magnetic microsphere body is a magnetic material encapsulated with $SiO_2$; preferably, the magnetic material is one or a combination of iron oxides, iron compounds, iron alloys, cobalt compounds, cobalt alloys, nickel compounds, nickel alloys, manganese oxides, and manganese alloys; more preferably, the magnetic material is one of $Fe_3O_4$, $\gamma$-$Fe_2O_3$, Iron Nitride, $Mn_3O_4$, FeCrMo, FeAlC, AlNiCo, FeCrCo, ReCo, ReFe, PtCo, MnAlC, CuNiFe, AlMnAg, MnBi, FeNiMo, FeSi, FeAl, FeSiAl, BaO•6$Fe_2O_3$, SrO•6$Fe_2O_3$, PbO•6$Fe_2O_2$, GdO or a combination thereof.

**13.** A preparation method for the biomagnetic microsphere according to claim 1, wherein comprising following steps: (1) performing a chemical modification to the magnetic microsphere body by an aminated silane coupling agent, introducing an amino group to the outer surface of the magnetic microsphere body to form an amino-modified magnetic microsphere A; the magnetic microsphere body is a magnetic material encapsulated by $SiO_2$; (2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B; (3) under a condition of not adding a cross-linking agent, by a polymerization of a carbon-carbon double bond, a plurality of acrylic monomer molecules are polymerized, and an acrylic polymer obtained has a linear backbone and a branched-chain containing a functional group; the polymer covalently couples to the outer surface of the magnetic microsphere B through one end of the linear backbone to form an acrylic polymer modified magnetic microsphere C; (4) covalently coupling the biotin or the biotin analog to the end of the branched-chain of the polymer through the functional group contained in the branched-chain of the polymer, and obtaining the biomagnetic microsphere.

**14.** The preparation method for the biomagnetic microsphere according to any one of claims 2-5, wherein comprising following steps: (1) performing a chemical modification to the magnetic microsphere body by an aminated silane coupling agent, introducing an amino group to the outer surface of the magnetic microsphere body to form an amino-modified magnetic microsphere A; the magnetic microsphere body is a magnetic material encapsulated by $SiO_2$; (2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B; (3) under a condition of not adding a cross-linking agent, by a polymerization of a carbon-carbon double bond, a plurality of acrylic monomer molecules are polymerized, and an acrylic polymer obtained has a linear backbone and a branched-chain containing a functional group; the polymer covalently couples to the outer surface of the magnetic microsphere B through one end of the linear backbone to form an acrylic polymer modified magnetic microsphere C; (4) covalently coupling the biotin or the biotin analog to the end of the branched-chain of the polymer through the functional group contained in the branched-chain of the polymer, and obtaining a biomagnetic microsphere D modified by the biotin or the biotin analogs; (5) connecting the purification element to the biotin or the biotin analog at the end of the branched-chain of the polymer on the biomagnetic microsphere D, to obtain a biomagnetic microsphere having the purification element bound; preferably, binding a covalent ligation complex of the avidin or the avidin analog and the purification element to an end of the branched-chain of the polymer, forming a binding action of an affinity complex of the biotin or the biotin analog and the avidin or the avidin analog to obtain the biomagnetic microsphere with the purification element; independently and preferably, comprising (6) sedimenting the biomagnetic microspheres by a magnet, removing a liquid phase and cleaning; independently and optionally, comprising a replacement of the covalent ligation complex of the avidin or the avidin analog and the purification element.

**15.** The preparation method for the biomagnetic microsphere according to claim 7, wherein comprising following steps: (1) performing a chemical modification to the magnetic microsphere body by an aminated silane coupling agent, introducing an amino group to the outer surface of the magnetic microsphere body to form an amino-modified magnetic microsphere A; the magnetic microsphere body is a magnetic material encapsulated by $SiO_2$; (2) covalently coupling an acrylic acid molecule to the outer surface of the magnetic microsphere A, by a covalent reaction between the carboxyl group and the amino group, and introducing a carbon-carbon double bond to form a carbon-carbon double bond-containing magnetic microspheres B; (3) under a condition of not adding a cross-linking agent, by a polymerization of a carbon-carbon double bond, a plurality of acrylic monomer molecules are polymerized, and an acrylic polymer obtained has a linear backbone and a branched-chain containing a functional group; the polymer covalently couples to the outer surface of the magnetic microsphere B through one end of the linear backbone to form an acrylic polymer modified magnetic microsphere C; (4) covalently coupling the biotin to the end of the branched-chain of the polymer through the functional group contained in the branched-chain of the polymer, and obtaining a biotin modified biomagnetic microsphere D; (5) binding a covalent ligation complex E of the avidin-affinity protein to the end of the branched-chain of the polymer, forming a binding action of an affinity complex of the biotin and the avidin, and obtaining the biomagnetic microsphere having the affinity protein bound; independently and preferably, comprising (6) sedimenting the biomagnetic microspheres by a magnet, removing a liquid phase and cleaning; independently and optionally, comprising a replacement of the covalent ligation complex of the avidin or the avidin analog and the purification element.

**16.** Use of the biomagnetic microsphere according to any one of claims 1 to 12 in separating and purifying a protein material; preferably, use of the biomagnetic microsphere in separating and purifying an antibody material; when the purification element connects to the end of the branched-chain of the polymer through the connection component containing the affinity complex, the use may comprise a regeneration of the biomagnetic microsphere optionally.

**17.** Use of the biomagnetic microsphere according to any one of claims 2 to 5, and 7 in separating and purifying a protein material, wherein the purification element is an affinity protein; preferably, the affinity protein connects to the branched-chain of the polymer in a way of biotin-avidin-affinity protein; preferably, the antibody material comprises an antibody, an antibody fragment, an antibody fusion protein, and an antibody fragment fusion protein; when the affinity protein connects to the end of the branched-chain of the polymer through the connection component containing the affinity complex, the use further comprises a regeneration and reuse of the biomagnetic microspheres optionally, that is, comprising replacing before reusing the affinity protein.

**18.** A biomagnetic microsphere, comprises a magnetic microsphere body, wherein an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of biotins connected; preferably, the end of the branched-chain of the polymer on the biomagnetic microsphere connects to a plurality of avidins through a binding action of an affinity complex.

**19.** A biomagnetic microsphere, comprises a magnetic microsphere body, wherein an outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of purification elements connected, the purification element is selected from: an avidin-type tag, a polypeptide-type tag, a protein-type tag, an antibody-type tag, an antigen-type tag, or a combination thereof; preferably, the avidin-type tag is avidin, a biotin-binding avidin analog, a biotin analog-binding avidin analog, or a combination thereof; more preferably, the avidin is a streptavidin, a modified streptavidin, a streptavidin analog, or a combination thereof; preferably, the polypeptide-type tag is selected from any one of following tags or a variant thereof: a CBP tag, a histidine tag, a C-Myc tag, a FLAG tag, a Spot tag, a C tag, an Avi tag, a Streg tag, a tag comprising a sequence of WRHPQFGG, a tag comprising a variant sequence of WRHPQFGG, a tag comprising a sequence of RKAAVSHW, a tag comprising a variant sequence of RKAAVSHW, or a combination thereof; preferably, the protein tag is selected from any one of following tags or a variant protein thereof: an affinity protein, a SUMO tag, a GST tag, an MBP tag and a combination thereof; more preferably, the affinity protein is selected from Protein A, Protein G, Protein L, modified Protein A, modified Protein G, modified Protein L and a combination thereof; more preferably, the outer surface of the magnetic microsphere body has at least one polymer with a linear backbone and a branched-chain arranged, an end of the linear backbone is fixed onto the outer surface of the magnetic microsphere body, a plurality of other ends of the polymer are free from the outer surface of the magnetic microsphere body, and an end of the branched-chain of the polymer on the biomagnetic microsphere has a plurality of affinity proteins connected; further preferably, a skeleton of the branched-chain between the affinity protein and the linear backbone of the polymer further has a binding action of the affinity complexes; more preferably, the affinity protein is selected from Protein A, Protein G, Protein L, modified Protein A, modified Protein G, modified Protein L and a combination thereof.

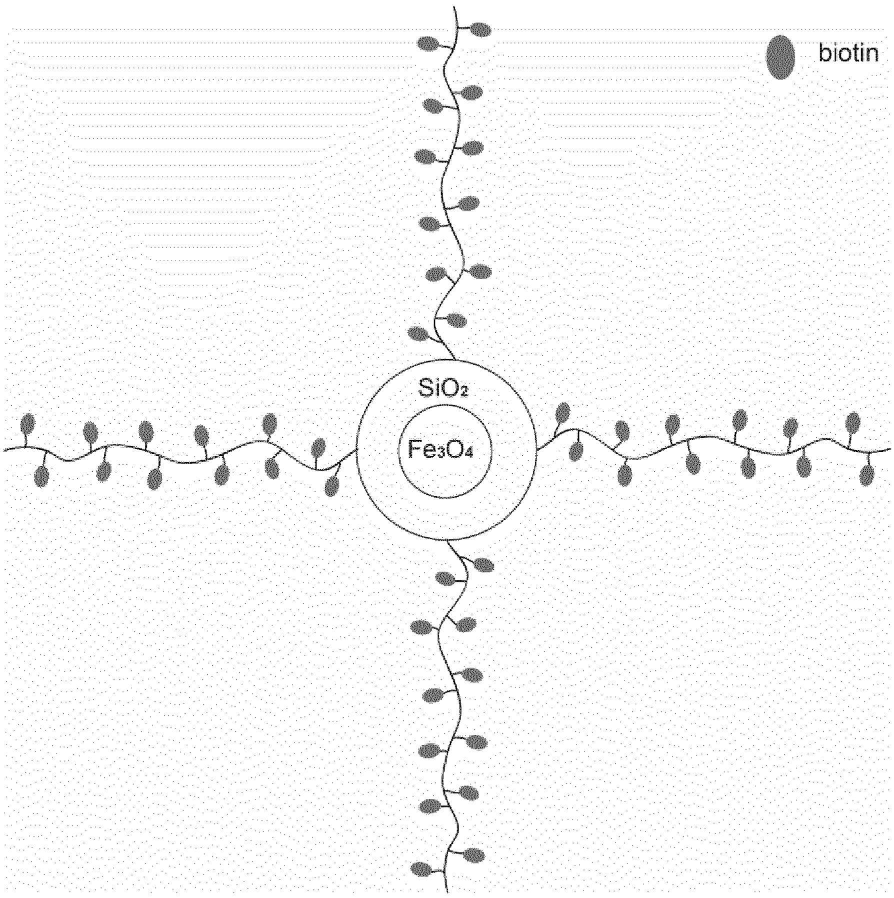


FIG. 1

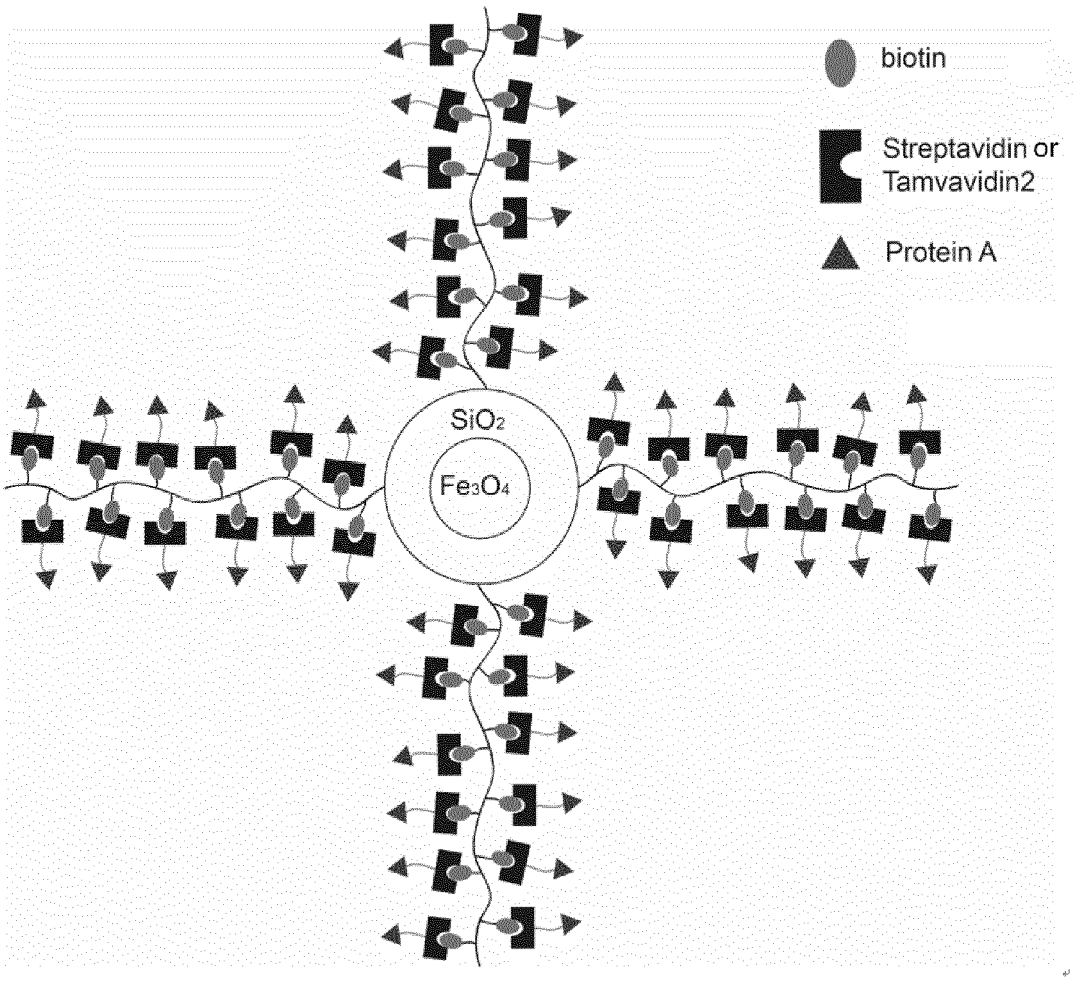


FIG. 2

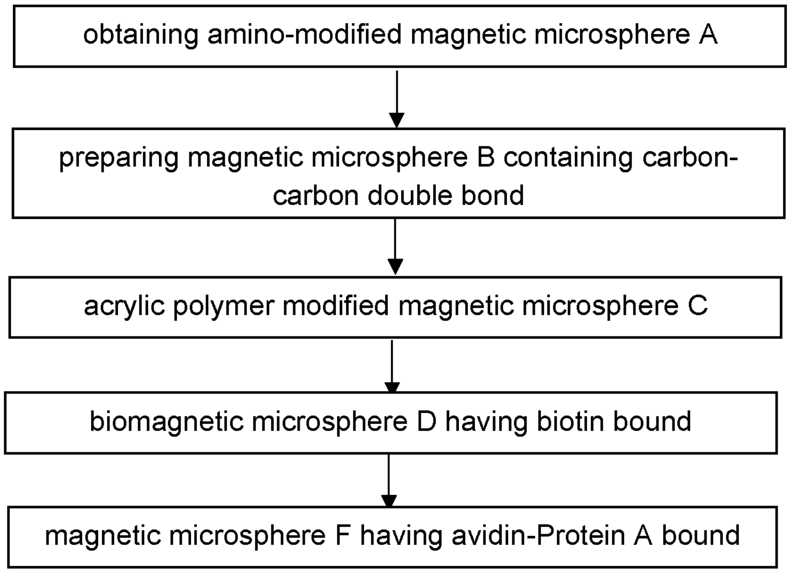
obtaining amino-modified magnetic microsphere A
preparing magnetic microsphere B containing carbon-carbon double bond
acrylic polymer modified magnetic microsphere C
biomagnetic microsphere D having biotin bound
magnetic microsphere F having avidin-Protein A bound

FIG. 3

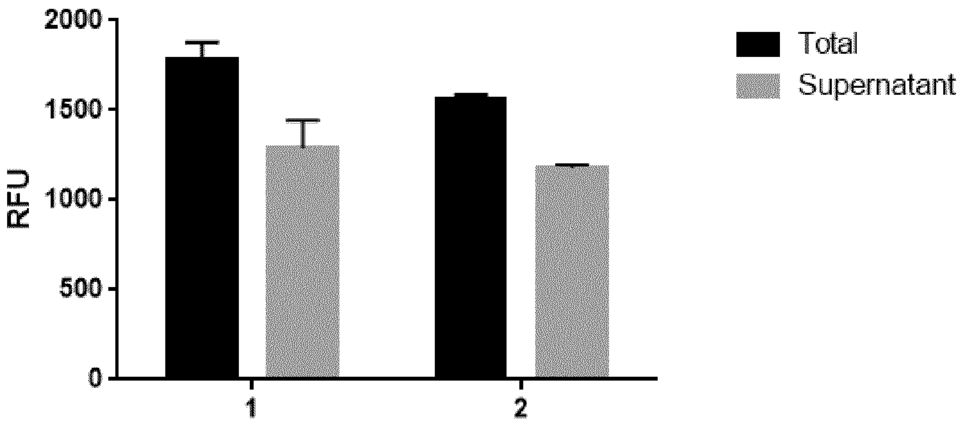
2000
1500
1000
500
0
RFU
1
2
Total
Supernatant

FIG. 4

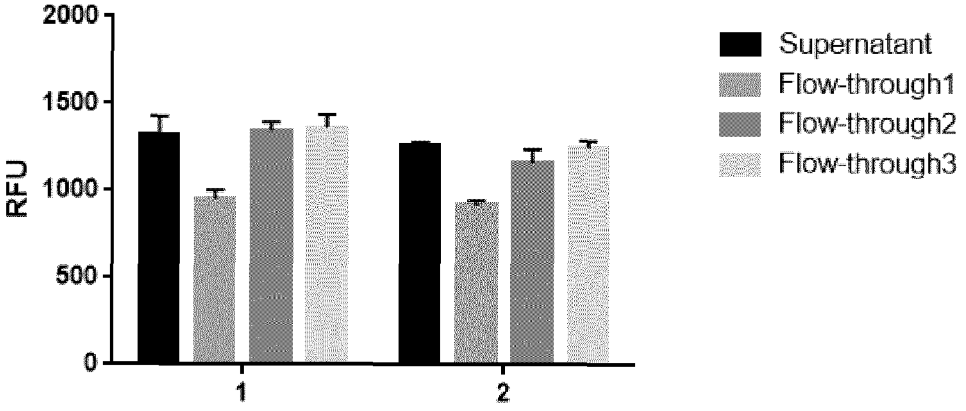
2000
1500
1000
500
0
RFU
1
2
Supernatant
Flow-through1
Flow-through2
Flow-through3

FIG. 5

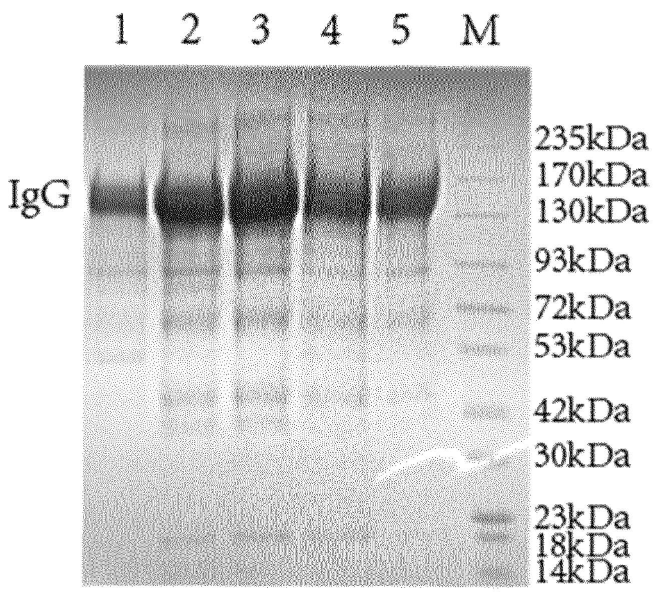
1
2
3
4
5
M
IgG
235kDa
170kDa
130kDa
93kDa
72kDa
53kDa
42kDa
30kDa
23kDa
18kDa
14kDa

FIG. 6

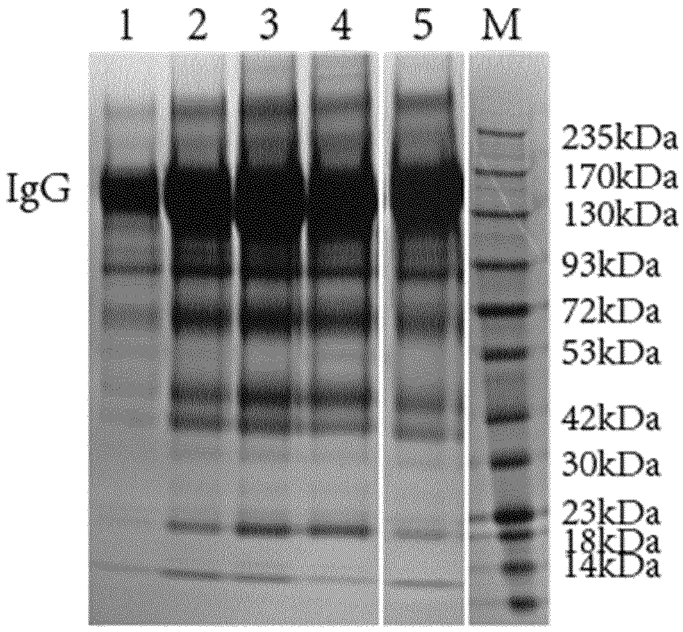
1
2
3
4
5
M
IgG
235kDa
170kDa
130kDa
93kDa
72kDa
53kDa
42kDa
30kDa
23kDa
18kDa
14kDa

FIG. 7

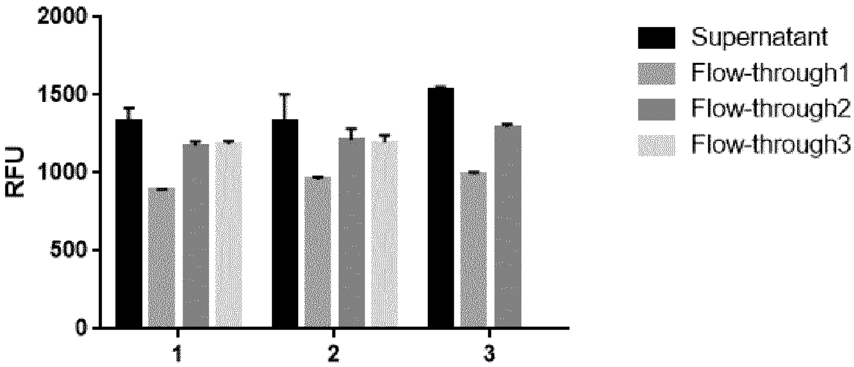
2000
1500
1000
500
0
RFU
1
2
3
Supernatant
Flow-through1
Flow-through2
Flow-through3

FIG. 8

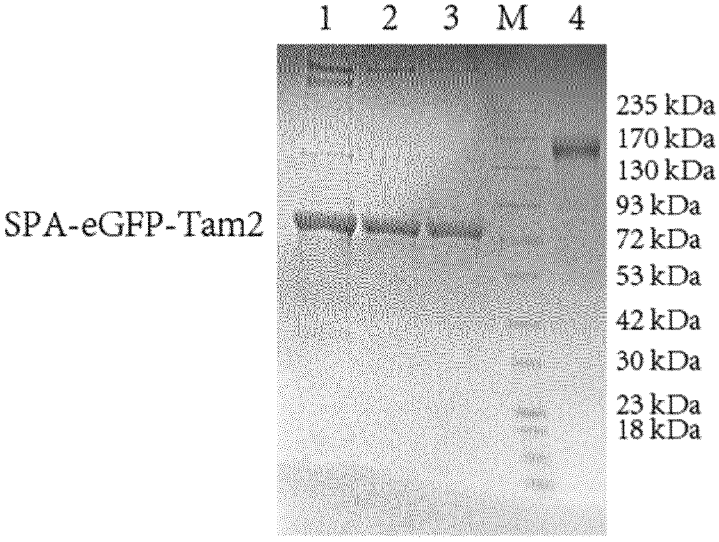
1
2
3
M
4
SPA-eGFP-Tam2
235 kDa
170 kDa
130 kDa
93 kDa
72 kDa
53 kDa
42 kDa
30 kDa
23 kDa
18 kDa

FIG. 9

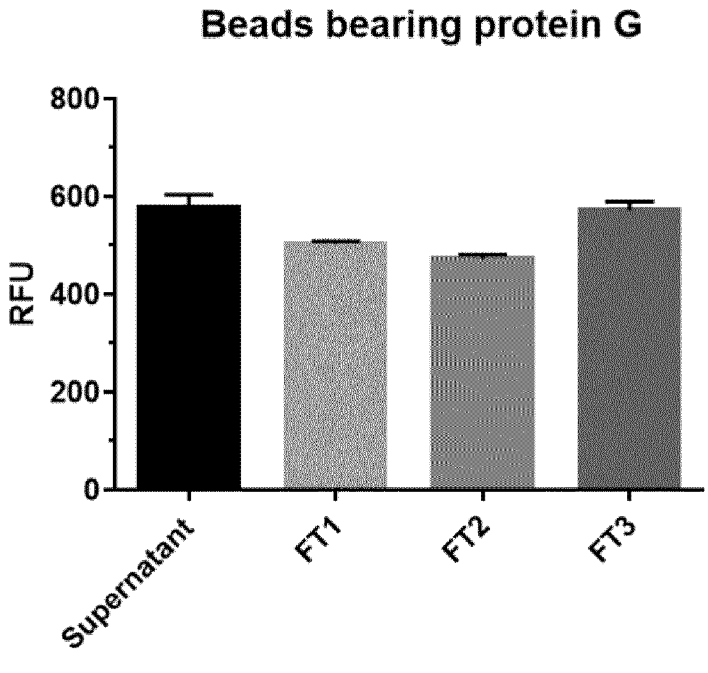
Beads bearing protein G
RFU
800
600
400
200
0
Supernatant
FT1
FT2
FT3

FIG. 10

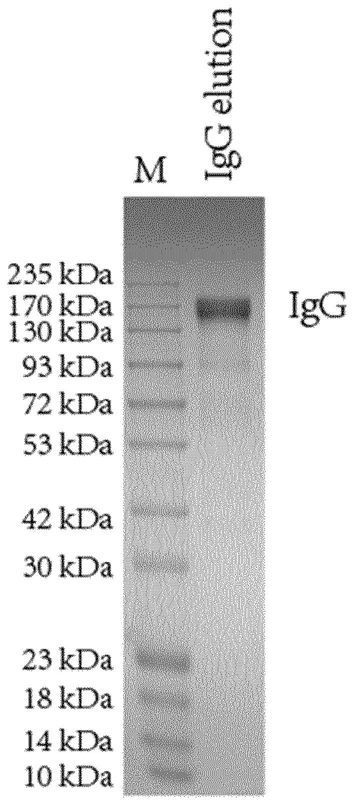
M
IgG elution
235 kDa
170 kDa
130 kDa
93 kDa
72 kDa
53 kDa
42 kDa
30 kDa
23 kDa
18 kDa
14 kDa
10 kDa
IgG

FIG. 11

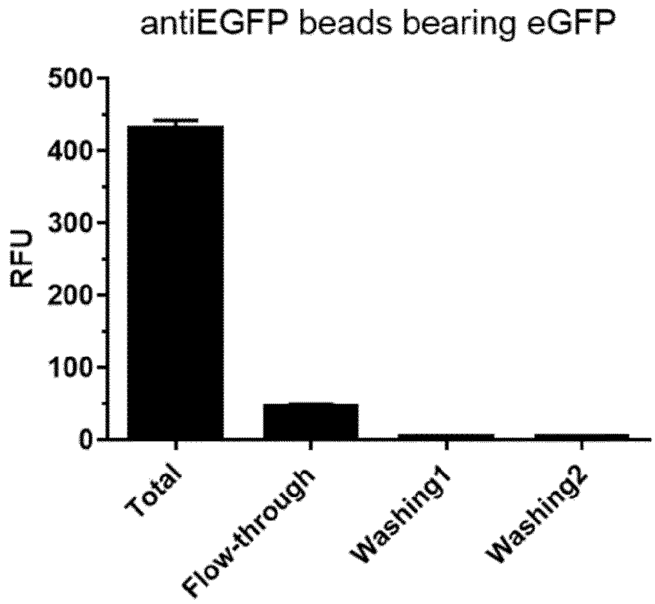
antiEGFP beads bearing eGFP
RFU
500
400
300
200
100
0
Total
Flow-through
Washing1
Washing2

FIG. 12

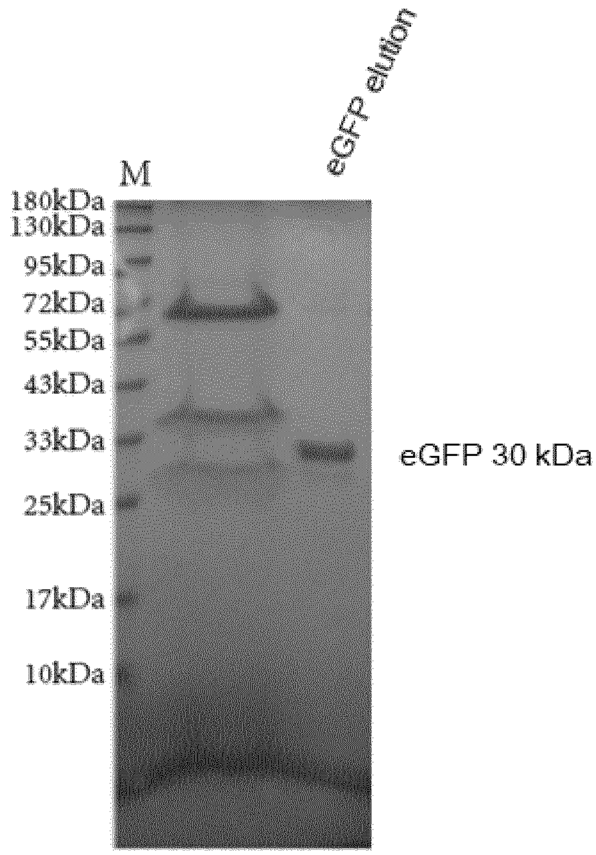
M
eGFP elution
180kDa
130kDa
95kDa
72kDa
55kDa
43kDa
33kDa
25kDa
17kDa
10kDa
eGFP 30 kDa

FIG. 13

## INTERNATIONAL SEARCH REPORT

International application No.
**PCT/CN2020/132171**

**A. CLASSIFICATION OF SUBJECT MATTER**

B01J 13/02(2006.01)i；C07K 1/22(2006.01)i；C12P 21/00(2006.01)i

According to International Patent Classification (IPC) or to both national classification and IPC

**B. FIELDS SEARCHED**

Minimum documentation searched (classification system followed by classification symbols)

B01J; C07K; C12P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, USTXT, EPTXT, CNKI, 万方数据资源系统, PubMed, ISI web of knowledge, GenBank, EBI-EMBL, 中国专利生物序列检索系统: 康码（上海）生物科技有限公司, 磁性, 微球, 聚合物, 生物素, 亲和素, 分离, 纯化, 多肽型标签, 亲和蛋白, SUMO标签, GST标签, MBP标签, WRHPQFGG, WRHPQFGG

**C. DOCUMENTS CONSIDERED TO BE RELEVANT**

| Category* | Citation of document, with indication, where appropriate, of the relevant passages | Relevant to claim No. |
|---|---|---|
| Y | CN 102617810 A (CHONGQING MEDICAL UNIVERSITY) 01 August 2012 (2012-08-01)<br>claims 1-5 | 1-19 |
| Y | 周莅霖 等 (ZHOU, Lilin et al.). "四氧化三铁纳米粒子表面接枝聚合制备磁性聚合物微球 (Preparation of Magnetic Hybrid Nanoparticles via Surface-Initiated Polymerization from Magnetite)"<br>*化学进展 (Progress in Chemistry),* Vol. 21, No. 09, 30 September 2009 (2009-09-30),<br>pp. 1880-1886 | 1-19 |
| Y | CN 1546520 A (SHANGHAI JIAO TONG UNIVERSITY et al.) 17 November 2004 (2004-11-17)<br>claim 1 | 1-19 |
| Y | CN 105126714 A (SHANGHAI JIAO TONG UNIVERSITY) 09 December 2015 (2015-12-09)<br>claims 1-3 | 1-19 |
| A | CN 108084252 A (CHANGZHOU SMART-LIFESCIENCES CO., LTD.) 29 May 2018 (2018-05-29)<br>entire document | 1-19 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 January 2021** | **03 March 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/132171**

**C. DOCUMENTS CONSIDERED TO BE RELEVANT**

| Category* | Citation of document, with indication, where appropriate, of the relevant passages | Relevant to claim No. |
|---|---|---|
| A | CN 1667413 A (INSTITUTE OF PROCESS ENGINEERING, CHINESE ACADEMY OF SCIENCES) 14 September 2005 (2005-09-14) entire document | 1-19 |
| A | US 2019002948 A1 (NUMEN SENSORICS APS) 03 January 2019 (2019-01-03) entire document | 1-19 |
| A | US 2015125896 A1 (AFFINERGY, LLC) 07 May 2015 (2015-05-07) entire document | 1-19 |
| A | US 2017299585 A1 (REGENTS OF THE UNIVERSITY OF MINNESOTA) 19 October 2017 (2017-10-19) entire document | 1-19 |

Form PCT/ISA/210 (second sheet) (January 2015)

**INTERNATIONAL SEARCH REPORT**

International application No.
**PCT/CN2020/132171**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2020/132171**

| Patent document cited in search report | | | Publication date (day/month/year) | Patent family member(s) | | | Publication date (day/month/year) |
|---|---|---|---|---|---|---|---|
| CN | 102617810 | A | 01 August 2012 | CN | 102617810 | B | 24 September 2014 |
| CN | 1546520 | A | 17 November 2004 | CN | 100334105 | C | 29 August 2007 |
| CN | 105126714 | A | 09 December 2015 | CN | 105126714 | B | 14 April 2020 |
| CN | 108084252 | A | 29 May 2018 | None | | | |
| CN | 1667413 | A | 14 September 2005 | None | | | |
| US | 2019002948 | A1 | 03 January 2019 | EP | 3425064 | B1 | 12 August 2020 |
| | | | | EP | 3425064 | A1 | 09 January 2019 |
| US | 2015125896 | A1 | 07 May 2015 | US | 9279810 | B2 | 08 March 2016 |
| US | 2017299585 | A1 | 19 October 2017 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2020132171 W
- CN 201911209156X
- CN 202010346903
- US 6103493 B2
- US 10065996 B2
- US 8735540 B2
- US 20070275416 A1
- CN 109423496 A
- WO 2016005982 A1
- CN 106978349 A
- CN 108535489 A
- CN 108690139 A
- CN 108949801 A
- CN 108642076 A
- CN 109022478 A
- CN 109423497 A
- CN 109423509 A
- CN 109837293 A
- CN 109971783 A
- CN 109988801 A
- CN 109971775 A
- CN 110093284 A
- CN 110408635 A
- CN 110408636 A
- CN 110551745 A
- CN 110551700 A
- CN 110551785 A
- CN 110819647 A
- CN 110845622
- CN 110938649 A
- CN 110964736 A
- CN 111378706 A
- CN 111378707 A
- CN 111378708 A
- CN 111718419 A
- CN 111748569 A
- CN 2019107298813
- CN 2019112066163
- CN 2018112862093
- CN 111118065 A
- CN 2019114181518
- CN 2020100693833
- CN 2020101796894
- CN 202010269333X
- CN 2020102693382
- CN 109593656 A
- CN 201610868691
- WO 2018161374 A1
- KR 20190108180 A
- CN 108535489 B


### Non-patent literature cited in the description


- **SAMBROOK et al.** Molecular Cloning: Laboratory Manual. Cold Spring Harbor Laboratory Press, 1989
- Cell-free Protein Synthesis Experiment Manual
- *Cell-free protein synthesis: methods and protocols [M,* 2008
- *FEBS Journal,* 2009, vol. 276, 1383-1397
- **TAKAKURA Y et al.** Tamavidins: Novel avidin-like biotin-binding proteins from the Tamogitake mushroom [J. *FEBS Journal,* 2009, vol. 276, 1383-1397
- Advances in Cell-Free Biosynthetic Technology. **LU, Y.** Current Developments in Biotechnology and Bioengineering. 2019, 23-45
- *CHEMICAL ABSTRACTS,* 919-30-2
- *CHEMICAL ABSTRACTS,* 110-26-9
- *CHEMICAL ABSTRACTS,* 4432-31-9
- *CHEMICAL ABSTRACTS,* 25952-53-8
- *CHEMICAL ABSTRACTS,* 6066-82-6
- *CHEMICAL ABSTRACTS,* 6066-82 -6